(19) Europäisches Patentamt
European Patent Office
Office européen des brevets

(11) **EP 0 959 896 B1**

(12) **EUROPEAN PATENT SPECIFICATION**

(45) Date of publication and mention
of the grant of the patent:
**25.05.2011 Bulletin 2011/21**

(51) Int Cl.:
*A61K 38/16* [(2006.01)]  *C07K 14/435* [(2006.01)]
*C07K 14/705* [(2006.01)]  *C07K 14/72* [(2006.01)]

(21) Application number: **97929783.5**

(22) Date of filing: **05.06.1997**

(86) International application number:
**PCT/US1997/009624**

(87) International publication number:
**WO 1997/046206 (11.12.1997 Gazette 1997/53)**

(54) **METHOD OF PRODUCING SECRETED TRUNCATED VARIANTS OF HUMAN THYROTROPIN RECEPTOR**

VERFAHREN ZUR HERSTELLUNG VON SEKRETIERTE VERKURZTE VARIANTEN DES HUMANEN THYROTROPINREZEPTORS

METHODE DE PRODUCTION DES VARIANTES TRONQUEES SECRETEES DU RECEPTEUR DE LA THYROTROPINE HUMAINE

(84) Designated Contracting States:
**AT BE CH DE DK ES FI FR GB GR IE IT LI LU MC NL PT SE**

(30) Priority: **05.06.1996  US 19171 P**

(43) Date of publication of application:
**01.12.1999 Bulletin 1999/48**

(73) Proprietors:
• **RAPOPORT, Basil**
**Larkspur, CA 94939 (US)**
• **McLachlan, Sandra**
**Larkspur, CA 94939 (US)**

(72) Inventors:
• **RAPOPORT, Basil**
**Larkspur, CA 94939 (US)**
• **McLachlan, Sandra**
**Larkspur, CA 94939 (US)**

(74) Representative: **Cornish, Kristina Victoria Joy et al**
**Kilburn & Strode LLP**
**20 Red Lion Street**
**London**
**WC1R 4PJ (GB)**

(56) References cited:
**WO-A-92/08726     US-A- 5 614 363**

• **HARFST E ET AL: "Interaction of thyrotropin and thyroid-stimulating antibodies with recombinant extracellular region of human TSH receptor." LANCET. 18 JAN 1992, vol. 339, no. 8786, 18 January 1992 (1992-01-18), pages 193-194, XP001202882 ISSN: 0140-6736**
• **CHAZENBALK GREGORIO D ET AL: "Expression of the Extracellular Domain of the Thyrotropin Receptor in the Baculovirus System Using a Promoter Active Earlier than the Polyhedrin Promoter: Implications for the expression of functional highly glycosylated proteins" JOURNAL OF BIOLOGICAL CHEMISTRY, vol. 270, no. 4, 1995, pages 1543-1549, XP002296093 ISSN: 0021-9258**
• **COSTAGLIOLA S ET AL: "BINDING ASSAY FOR THYROTROPIN RECEPTOR AUTOANTIBODIES USING THE RECOMBINANT RECEPTOR PROTEIN" JOURNAL OF CLINICAL ENDOCRINOLOGY AND METABOLISM, NEW YORK, NY, US, vol. 75, no. 6, 1992, pages 1540-1544, XP001000292 ISSN: 0021-972X**
• **DATABASE EMBL 1 November 1996 (1996-11-01), XP002296098 retrieved from EBI Database accession no. Q16503 & TAKESHITA AKIRA ET AL: "Molecular cloning and sequencing of an alternatively spliced form of the human thyrotropin receptor transcript" BIOCHEMICAL AND BIOPHYSICAL RESEARCH COMMUNICATIONS, vol. 188, no. 3, 1992, pages 1214-1219, XP001194873 ISSN: 0006-291X**

**(Cont. next page)**

- CHAZENBALK GREGORIO D ET AL: "Cleavage of the thyrotropin receptor does not occur at a classical subtilisin-related proprotein convertase endoproteolytic site" JOURNAL OF BIOLOGICAL CHEMISTRY, vol. 269, no. 51, 1994, pages 32209-32213, XP002296094 ISSN: 0021-9258
- RAPOPORT BASIL ET AL: "Critical relationship between autoantibody recognition and thyrotropin receptor maturation as reflected in the acquisition of complex carbohydrate" JOURNAL OF CLINICAL ENDOCRINOLOGY AND METABOLISM, vol. 81, no. 7, July 1996 (1996-07), pages 2525-2533, XP002296095 ISSN: 0021-972X
- CHAZENBALK GREGORIO D ET AL: "Engineering the human thyrotropin receptor ectodomain from a non-secreted form to a secreted, highly immunoreactive glycoprotein that neutralizes autoantibodies in Graves' patients' sera" JOURNAL OF BIOLOGICAL CHEMISTRY, vol. 272, no. 30, 25 July 1997 (1997-07-25), pages 18959-18965, XP002296096 ISSN: 0021-9258
- CHEN CHUN-RONG ET AL: "Evidence that the C terminus of the A subunit suppresses thyrotropin receptor constitutive activity." ENDOCRINOLOGY, vol. 144, no. 9, September 2003 (2003-09), pages 3821-3827, XP002296097 ISSN: 0013-7227
- HUNT N; WILLEY K P; ABEND N; BALVERS M; JAHNER D; NORTHEMANN W; IVELL R: "Novel splicing variants of the human thyrotropin receptor encode truncated polypeptides without a membrane-spanning domain" ENDOCRINE, vol. 3, no. 3, 1995, pages 233-240, XP008035227 UNITED KINGDOM
- J. BIOL. CHEM., 23 December 1994, Vol. 269, No. 51, CHAZENBALK et al., "Cleavage of the Thyrotropin Receptor Does Not Occur at a Classical Subtilisin-Related Proprotein Convertase Endoproteolytic Site", pages 32209-32213.
- RAPOPORT B; CHAZENBALK G D; JAUME J C; MCLACHLAN S M: "The thyrotropin (TSH) receptor: interaction with TSH and autoantibodies." ENDOCRINE REVIEWS, vol. 19, no. 6, December 1998 (1998-12), pages 673-716, US ISSN: 0163-769X
- HUNT N; WILLEY K P; JÄHNER D; IVELL R; NORTHEMANN W; CASTEL M A; LEIDENBERGER F: "Multiple forms of thyroid stimulating hormone receptor associated with Graves' disease" EXPERIMENTAL AND CLINICAL ENDOCRINOLOGY,, vol. 100, no. 1-2, 1 January 1992 (1992-01-01), pages 22-27, XP009114745 LEIPZIG, DE ISSN: 0232-7384

**Description**

**Field of the Invention**

The present invention relates generally to the fields of cell physiology, endocrinology and immunology. More particularly, the present invention relates to a method of producing a secreted, soluble, complex carbohydrate-containing, endoglycosidase H resistant and endoglycosidase F sensitive form of the human thyrotropin receptor (TSHR) ectodomain.

**Background of the Invention**

Hunt et al (Endocrine 3, 233-240, 1995) discusses novel splicing variants of the human thyrotopin receptor that encode truncated polypeptides without a membrane-spanning domain. The authors analysed in detail the in vivo transcription of the human TSH receptor gene (hTSH-R), demonstrating the presence of numerous novel TSH receptor transcripts. Northern blot analysis of mRNA from human thyroid tissue using a radiolabelled cDNA probe specific for the extracellular domain of the hTSH-R revealed the presence of small polyadenylated mRNAs in addition to the full-length hTSH-R mRNA. A PCR strategy devised to clone transcripts with 3' polyadenylation and 5' hTSH-R specific sequences was used to clone five different hTSH-R transcripts from human, thyroid tissue. Sequence analysis demonstrated that the small transcripts arose by alternative splicing of the hTSH-R mRNA.

Hunt et al (Experimental and Clinical Endocrinology 100, 22-27, 1992) relates to multiple forms of thyroid stimulating hormone receptor associated with Graves' disease. In this report, the authors presented data showing that the TSH receptor in Graves' thyroid behaves like a "soluble" receptor. Cloning of the human TSH receptor by homology with published LH receptor sequences enabled the direct investigation of TSH receptor mRNA from Graves' and normal thyroid. Smaller transcripts were found, that were not observed in non-pathological tissue, and which encode a "soluble" receptor. Several full-length and truncated hTSH receptor constructs were transfected into various cell lines and stable, TSH receptor-expressing clones were produced. Further analysis of mRNA from cells transfected with only the extracellular domain of the TSH receptor confirmed the production of a "soluble" receptor.

**Brief Description of the Drawings**

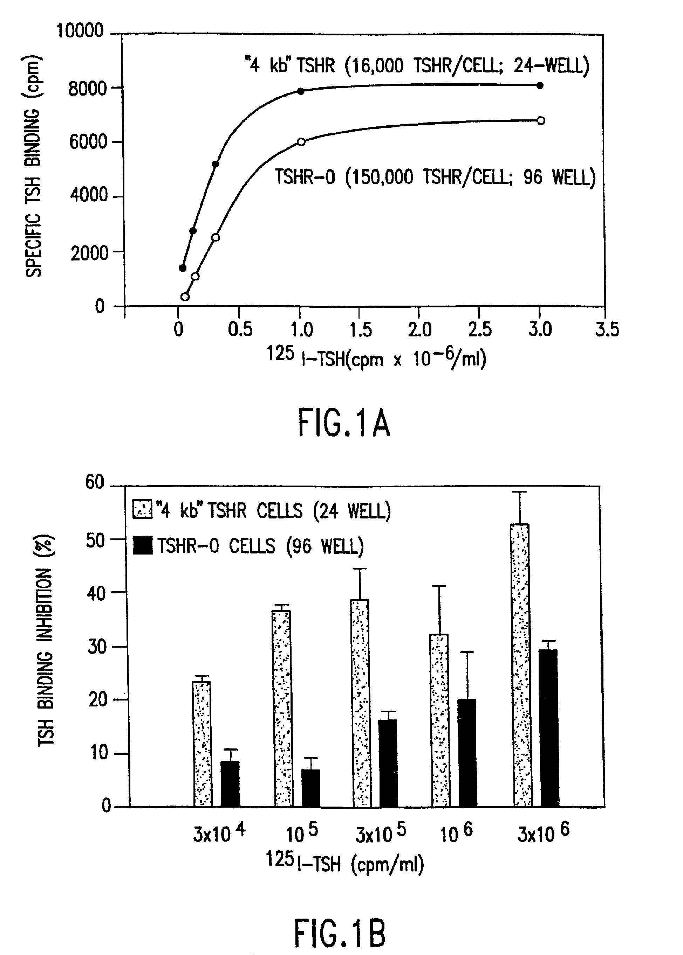

Figure 1: Panel A: Specific [12]I-TSH binding to CHO cell monolayers expressing the human TSHR on their surface. In order to obtain similar numbers of TSHR per well, 4 kb TSHR cells and TSHR-0 cells were cultured in 24 well and 96 well cluster dishes, respectively. Cells were incubated with the indicated concentrations of $^{125}$I-bTSH (250 ml and 50 ml per well in the 24 well and 96 well plates, respectively). Specific; binding was determined by subtraction of tracer bound to untransfected CHO cells in parallel wells. Panel B: TBI assay using 4 kb TSHR and TSHR-0 cells cultured in 24 well and 96 well plates, respectively. The assay was performed with the same Graves' patient serum at a saturating concentration of $^{125}$I-bTSH ($10^6$ cpm/ml), as described in methods. Data shown in both panels are the mean of closely agreeing values from duplicate dishes of cells.

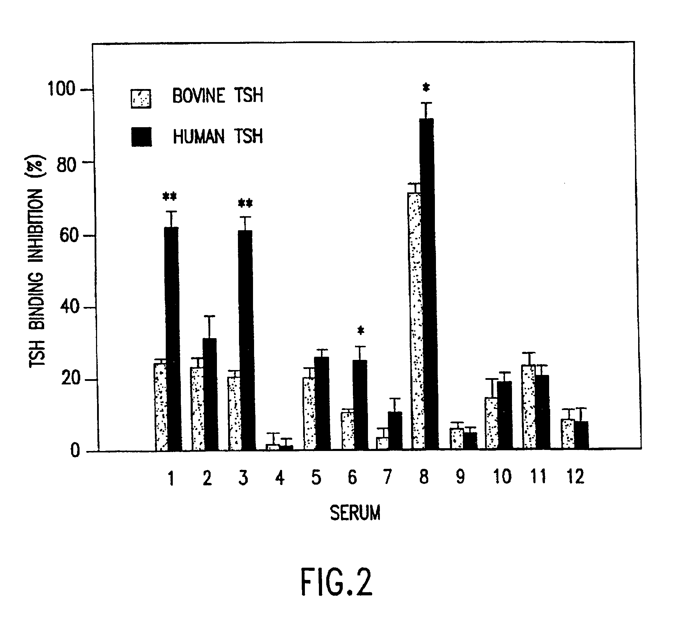

Figure 2: Comparison of radiolabeled human and bovine TSH in a TBI assay. Data are shown for 12 sera assayed using TSHR-0 cells (~150,000 TSHR per cell) cultured in a 96 well microtiter plate. TBI assay was as described in Methods. Fifty ml of either $^{125}$I-bTSH or $^{125}$I-hTSH ($5 \times 10^4$ cpm; $1()°$ cpm/ml) was added to each well. Values shown are the mean of closely agreeing triplicate wells for each tracer. Tracer TSH binding in the presence of normal serum was:- $^{125}$I-bTSH, 12,396 cpm (mean of 12,044, 12,323, 12821 cpm); hTSH, 2442 cpm (mean of 2509. 2362, 2454 cpm).

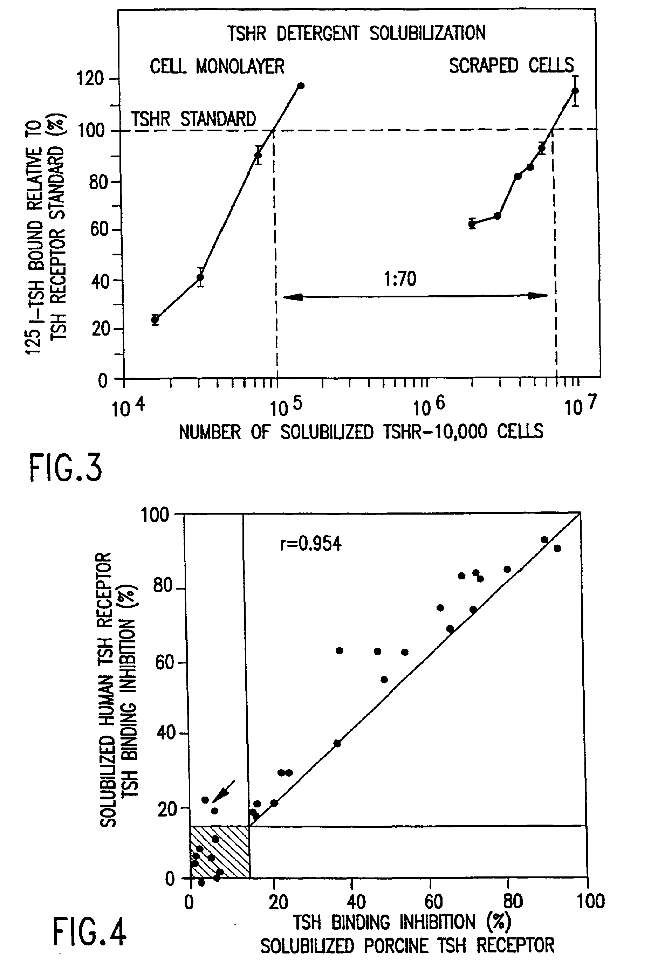

Figure 3: Scraping and homogenizing TSHR-10,000 cells reduces the yield of TSHR extracted with detergent. Cells were scraped from 50 confluent 10 cm dishes ($5 \times 10^8$ cells) and pelleted. After extraction with buffer containing 1% Triton X-100 (Methods), aliquots (50 ml) derived from the indicated number of cells was substituted for the same volume of porcine TSHR normally used in the kit. In a separate protocol, cells were "directly" extracted with buffer containing 1% Triton X-100 without removing the cells from the culture dishes (Methods). Three ml of detergent-containing buffer was added to a 10 cm diameter culture dish ($10^7$ cells). During dilution of the cell extracts, the detergent concentration was kept constant. The final concentration of 0.25% Triton X-100 in the assay was found not to affect $^{125}$I-TSH binding. The dashed vertical lines indicate the number of CHO cells needed to attain TSH binding equivalent to 50 ml of solubilized porcine TSHR, defined as 100% (horizontal, dashed line).

Figure 4: Comparison of solubilized porcine and human TSHR in a TSH binding inhibition assay. TBI activity was determined in 30 sera sent to a clinical laboratory for known or suspected Graves' disease. Sera were tested with a commercial kit using porcine TSHR. In addition, the same sera were assayed with the same reagents with the exception that solubilized human TSHR was substituted for porcine TSHR (Methods). The cut-off point for positivity, as defined in the kit (TBI >15%), is indicated. The arrow indicated two sera that have detectable TBI activity with the human TSHR. but not with the porcine TSHR.

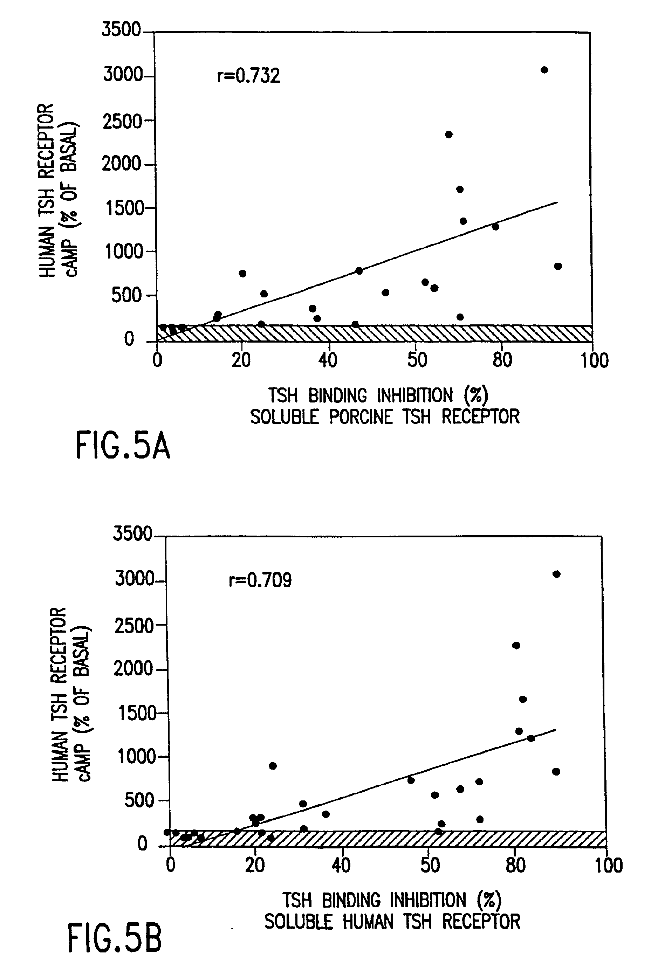

Figure 5: Correlation between thyroid stimulating immunoglobulin (TSI) activity and TSH binding inhibitory (TBI) activity determined with solubilized human (panel A) or porcine (panel B) TSHR. TSI activity was determined on 28 of the sera depicted in Fig. 4 using a bioassay involving Chinese hamster ovary (CHO) cells stably transfected with the human TSHR (Methods). The hatched area indicates the mean $\pm$ 2 S.D. of cAMP levels (130% of basal) determined on sera from normal individuals (n= 20).

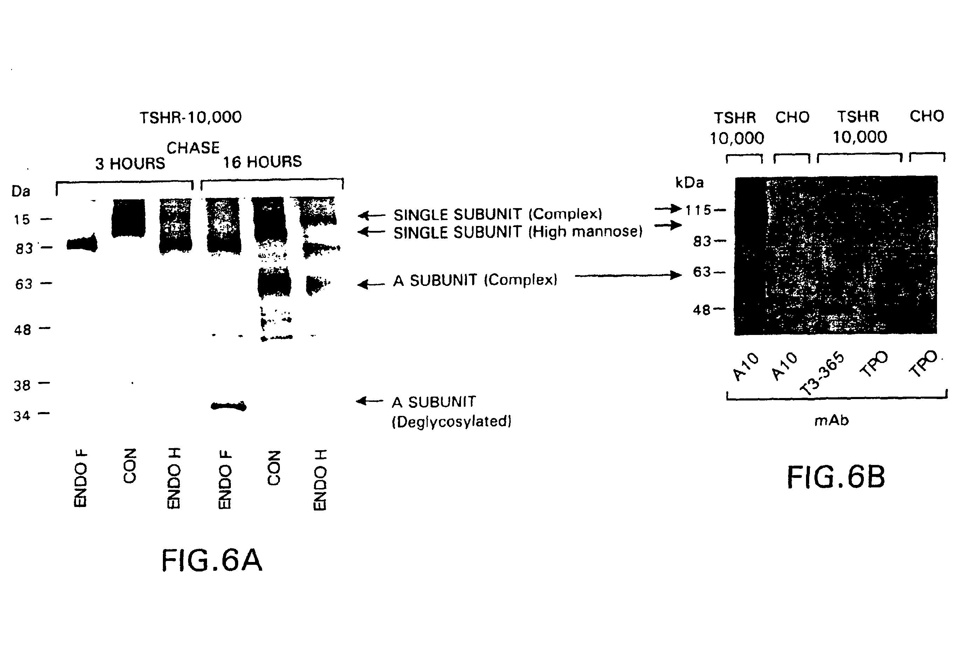

Figure 6: Panel A: Immunoprecipitation of precursor-labeled TSH receptors in TSHR-10,000 cells. Cell proteins were labeled with $^{35}$S-methionine and $^{35}$S-cyseine (one hr pulse; 3 and 16 hr chases) followed by immunoprecipitation using mouse mAb A10 whose epitope is TSHR amino acid residues 22-35 (17)(Methods). Portions of the immunoprecipitated material were left untreated (Con) or were treated with endoglycosidase H (Endo H) or with N-glycosidase F (Endo F). The products were subjected to polyacrylamide gel electrophoresis (10%) under reducing conditions. Autoradiography was for 16 h. The sizes of the prestained molecular weight markers used in this and subsequent experiments were determined by prior calibration against unstained markers (Methods). Panel B: Specificity of mAb for the TSHR. Immunoprecipitations were performed after an overnight chase using both TSHR-10,000 cells and untransfected CHO cells. In addition, precursor-labeled TSHR-10,000 cells were incubated with a mAb to TPO. Immunoprecipitated proteins (not enzymatically deglycosylated) were applied to a 7.5% polyacrylamide gel. Note the non-specificity of the 48 kDa band. Also shown in this figure, immunoprecipitation of the TSR in TSHR-10,000 cells with mAb T3-365 to the B subunit was much less efficient than with mAb A10, possibly because the former recognizes primarily denatured TSHR (21). The apparent doublet at 48 kDa with mAb T3-365 is a non-reproducible artifact.

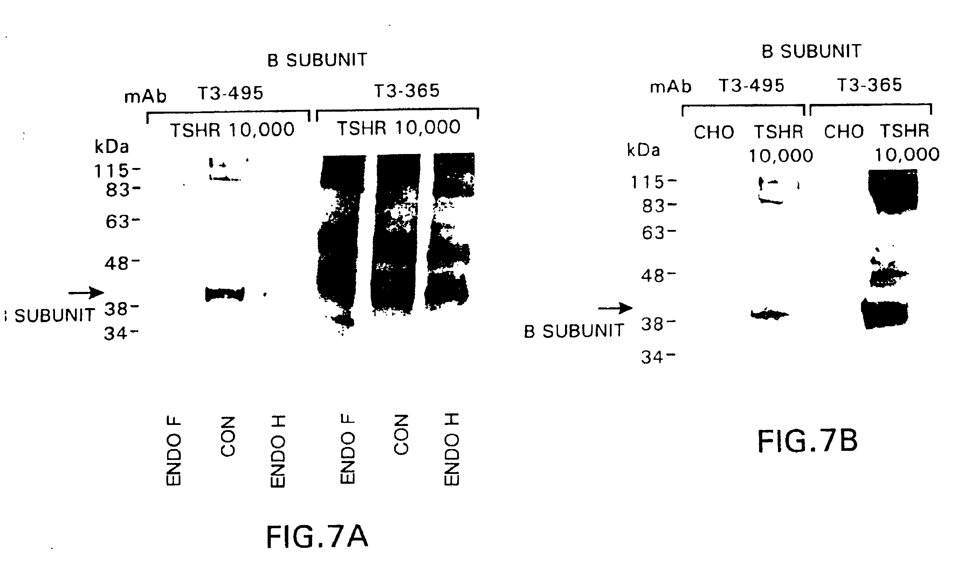

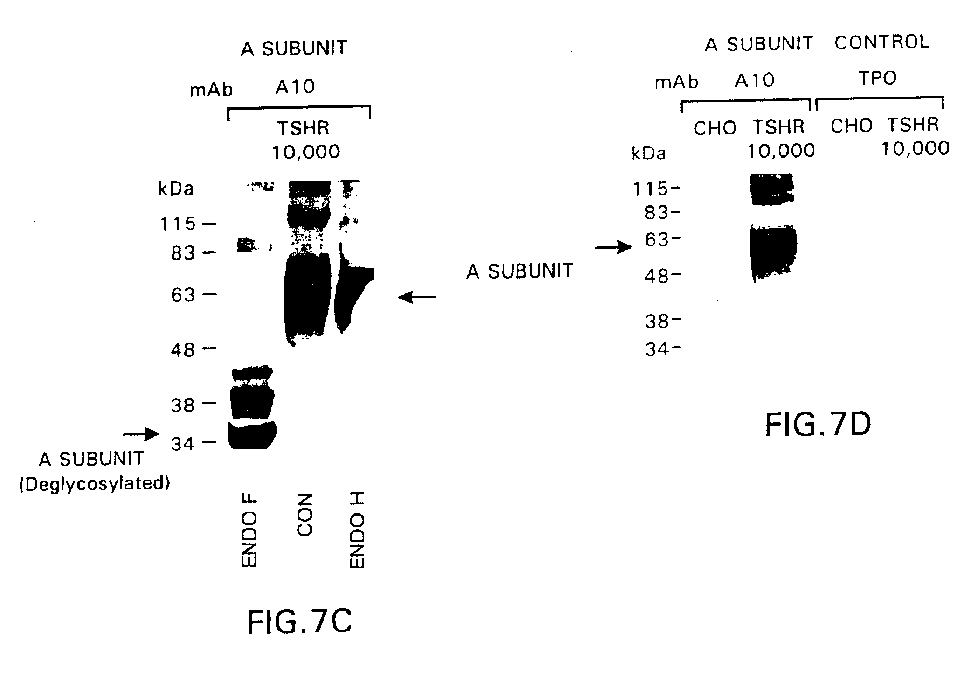

Figure 7: Immunoblots of the TSHR overexpressed in TSHR,-10,000 cells. Where indicated, crude membrane preparations of these cells, or of untransfected CHO cells ("CHO"), were left untreated (Con) or were treated with N-glycosidase F (Endo F) or with endoglycosidase H (Endo H)(Methods). The material was then subjected to polyacrylamide gel electrophoresis (10%) under reducing conditions, transferred to PVDF membranes and probed with the indicated mAb. Panel A:. Immunoblots using mAb T3-495 and T3-365, both to the TSHR B subunit (epitope within residues 604-764)(Methods). Panel B: Specificity of the T3-495 and T3-365 mAb, as determined by their lack of interaction with untransfected cells. Panel C: Confirmation of the size of the deglycosylated A subunit on immunohlotting with mAb A10 (epitope including TSHR amino acid residues 22-35). Similar data were obtained with another mAb to the TSHR A subunit, mAb A11 (data not shown). Panel D: Specificity of mAb A10 for the TSHR as determined by its lack of recognition of untransfected CHO cells, as well as by the lack of detection of the TSHR by a mAb to a non-relevant antigen (TPO).

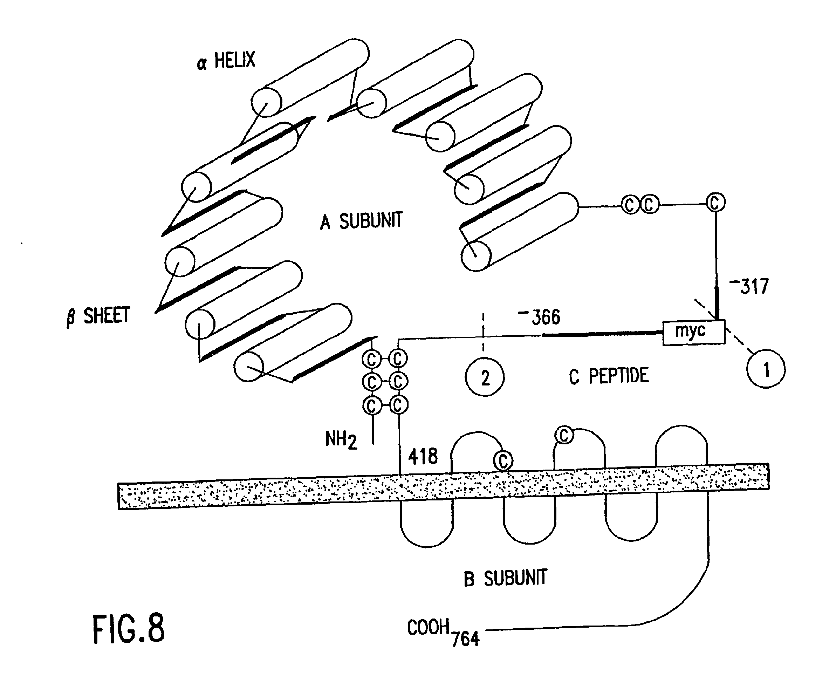

Figure 8: Schematic representation of the TSH receptor subunits. The diagram is not drawn to scale and is a modification of our previous version (12) based on the new information in the present study. The amino terminal two-thirds of the TSHR ectodomain contains 9 leucine rich repeats, each with an $\alpha$-helix and $\beta$-sheet, and is based on the three-dimensional structure of ribonuclease A inhibitor (28). A more detailed model of this region has been performed by Kajava et al. (29). The mass of the A subunit polypeptide backbone is 35 kDa, hence the estimated cleavage site #1 at approximately amino acid residue 330 (3). The primary non-glycosylated B subunit is ~42 kDa, thereby placing the second cleavage site at about residue 380. Cleavage at these two sites would release a putative C peptide of -50 amino acid residues. The thick line between residues 317-366 represents a 50 amino acid "insertion" that is unique to the TSHR relative to the other glycoprotein hormone receptors (3,25). Only 6 of the 8 cysteines in the A subunit are shown. The cysteines shown to he involved in disulfide bonding are hypothetical, but convey the fact that they maintain A and B subunit linkage after TSHR cleavage. TSHRmyc contains a c-myc epitope substituted for residues 338-349.

Detection by a mAb to this epitope of only the single subunit forms of the TSHR would indicate loss of this epitope consequent to cleavage into two subunits. In this case, cleavage could be upstream of, or within, the c-myc epitope.

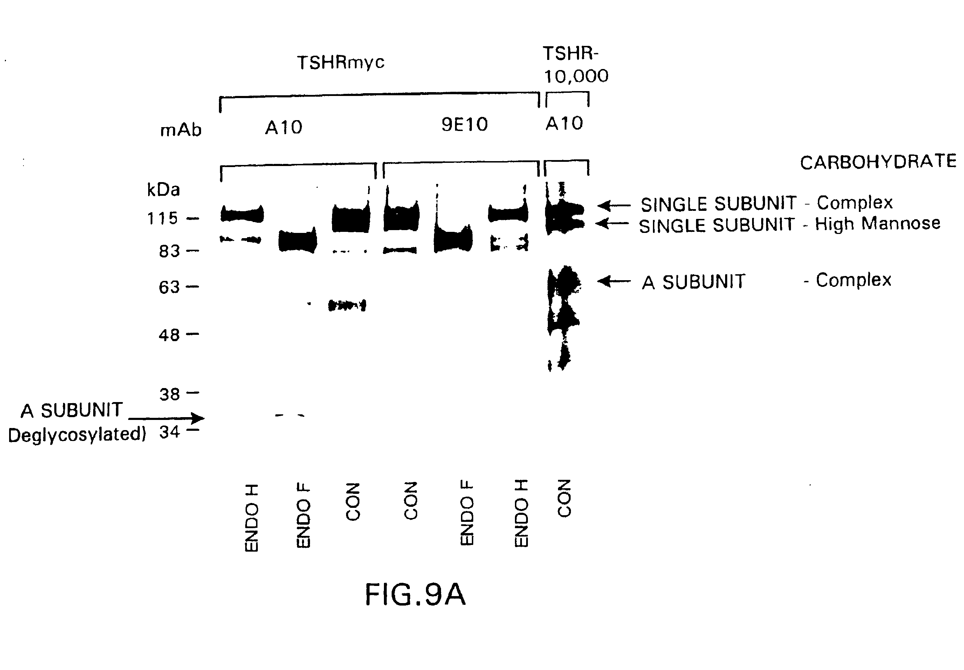

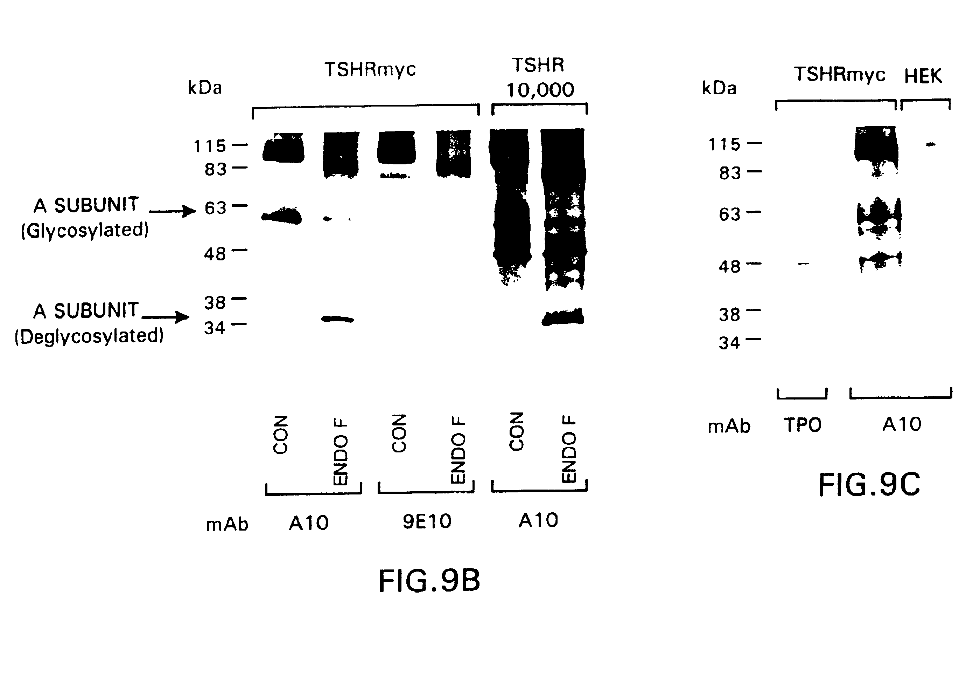

Figure 9: Immunoprecipitation of c-myc epitope-tagged TSHR (TSHRmyc) stably expressed in HEK cells. Panel A: Cell proteins were labeled with $^{35}$S-methionine and $^{35}$S-cysteine (one hr pulse: overnight chase) followed by immunoprecipitation with either mAb A10 (A subunit) or mAb 9E10 (c-myc epitope). Precipitated samples were left untreated (Con) or were treated with endoglycosidase H (Endo H) or N-glycosidase F (Endo F)(Methods). The products were subjected to polyacrylamide gel electrophoresis (10%) under reducing conditions. As a control to emphasize the position of the glycosylated A subunit, immunoprecipitation by mAb A10 of TSHR-10,000 cells (approximately 1/20 the amount of the TSHRmyc material) is shown in the extreme right hand lane. Note that the glycosylated A subunit is a diffuse band overlapping with a sharper, non-glycosylated band art ~ 62 kDa. Autoradiography of the dried gel was for 17 days. Panel B: An experiment similar to that in Panel A demonstrating a number of additional or subtle features. First, and most important, the deglycosylated A subunit in TSHR-10,000 cells is included in the same gel to indicate identity in size with the deglycosylated A subunit in the TSHR myc cells. Second, proponionality of the different receptor forms varies from experiment to experiment. Also note that in TSHR-10.000 cells, the relative proportion of cleaved to uncleaved receptor, as well as of the different forms of single chain receptor, varies in the two experiments shown, a feature noted by us in earlier TSH cross-linking experiments (7). Autoradiography was for 17 days. Panel C: Specificity of the deglycosylated, 35 kDa A subunit band recognized by mAb A10, as determined by immunoprecipitation of untransfected HEK cells, as well as of TSHRmyc cells with a non-relevant mAb to TPO. Note the non-specificity of the 48 kDa band. The diffuse glycosylated A subunit is superimposed on sharper, TSHR components that are not glycosylated (see Panel A) and may represent precursor or degradation products of the TSHR within the cell. For data on the specificity of mAb 9E10 of the c-myc epitope in the TSHRmyc cells, see Ref. 15.

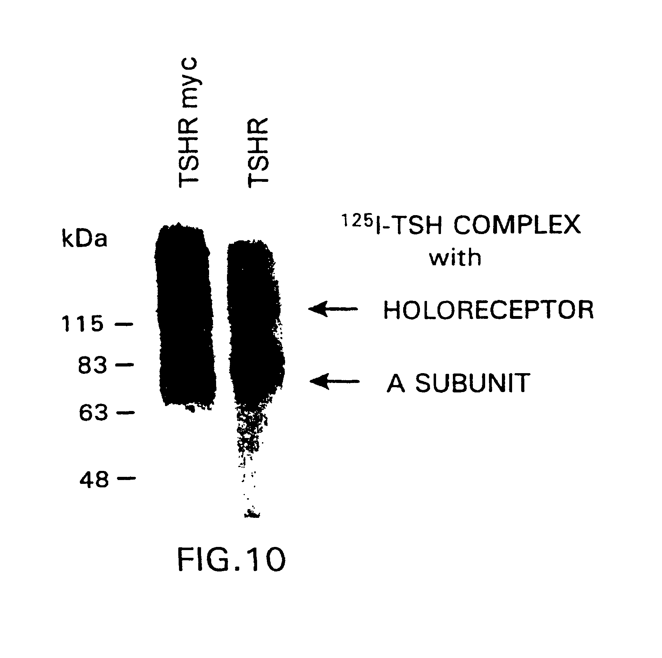

Figure 10: Comparison of $^{125}$I-TSH cross-linking to TSHR on the surface of intact TSHRmyc and TSHR-0 cells. Both cell types express similar number of TSHR ( $\sim 10^5$ and $- 1.5 \times 10^5$ per cell, respectively). Radiolabeled TSH crosslinking, PAGE (10%) under reducing conditions and autoradiography (18 hr) were as described in Methods. Note that the ligand, $^{125}$I-TSH, itself contains two subunits linked by disulfide bonds. Under reducing conditions, only one of these subunits (- 14 kDa) remains covalently linked to the TSHR. Therefore, the size of the TSHR can be deduced by subtracting this mass from the complex.

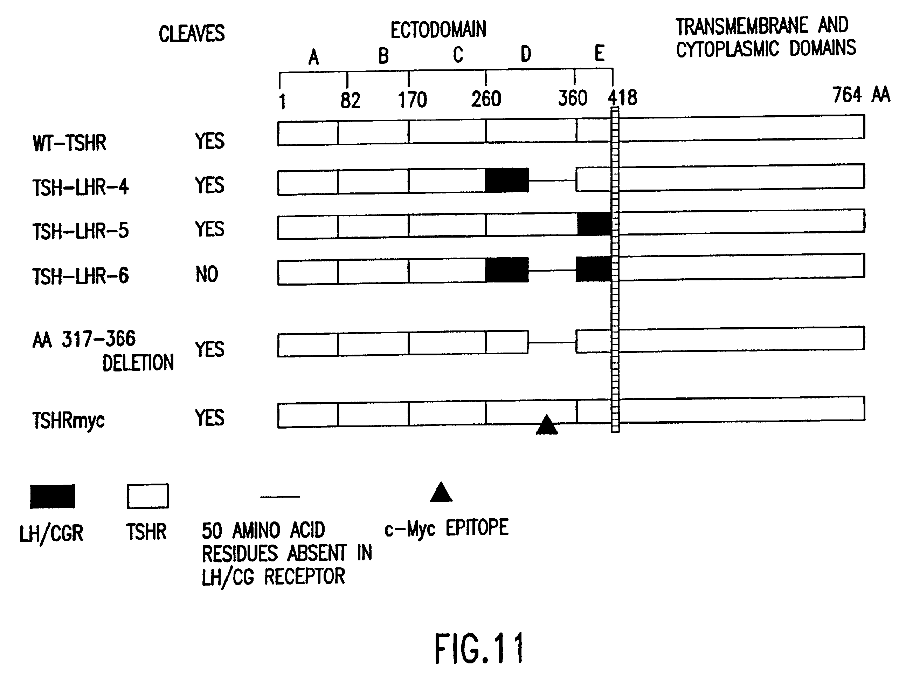

Figure 11: Additional evidence for the existence of two cleavage sites in the TSHR. The ectodomain of the TSHR is shown divided into 5 arbitrary domains (A through E) that were used in creating chimeric TSH-LH/CG receptor molecules (24). Three chimeric receptors that are relevant to the present study are indicated, as well as a TSHR mutant in which amino acid residues 317-366 are deleted (25). These 50 amino acids are not present in the LH/CG receptor. Therefore, when domain D of the TSHR is replaced with the corresponding segment of the LH/CG receptor, residues 317-360 are missing. The site of the c-myc epitope in TSHRmyc is shown relative to the other segment.

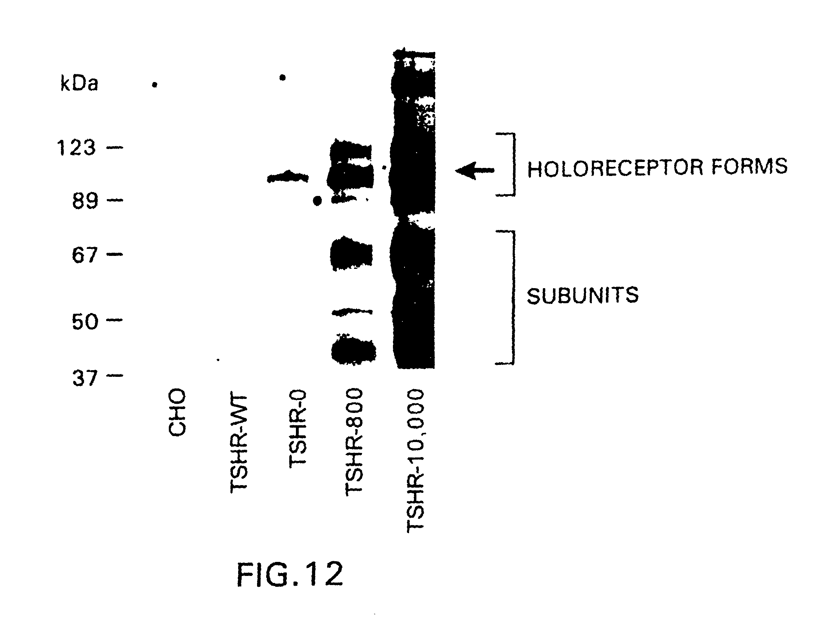

Figure 12: Immunoprecipitation of TSHR protein in whole CHO cells. Cell proteins were precursor-labeled with $^{35}$S-methionine and $^{35}$S-cysteine (one hour pulse and 8 hour chase in the experiment shown) followed by immunoprecipitation under native conditions using mouse monoclonal antibodies to the TSHR (A9 + A10; Dr. Paul Banga, London, U.K.). The following clonal CHO cell lines were used:- CHO - untransfected cells; TSHR-WT - cell transfected with the 4 kb TSHR cDNA containing both 5'- and 3'-untranslated regions (26); TSHR-0 - cells transfected with the 2.3 kb TSHR cDNA with deleted untranslated regions (25); TSHR-800 and TSHR-10,000 - cells transfected with the 2.3 kb TSHR cDNA and subsequently adapted to growth in 800 nM or 10,000 nM methotrexate, respectively. Immunoprecipitated proteins were subjected to polyacrylamide gel electrophoresis (7.5%) under reducing conditions. Autoradiography was for 10 days. Essentially identical data were obtained in a second experiment. The arrow indicates the 100 kDa TSHR holoreceptor form previously observed by Misrahi et al. (12) that was quantitated by densitometry.

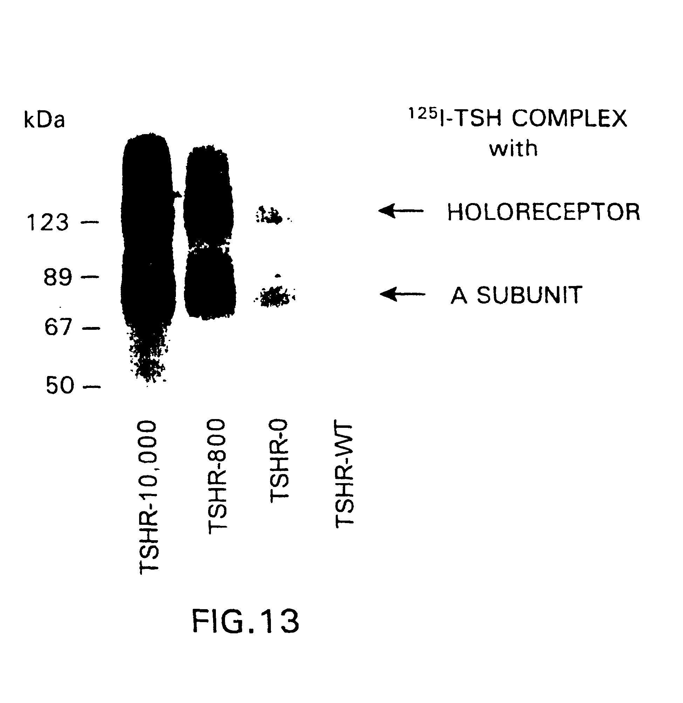

Figure 13: Crosslinking of $^{125}$I-TSH to TSHR on the surface of intact cells. The cells used are described in the legend to Fig. 12. Radiolabeled TSH crosslinking, PAGE (7.5%) under reducing conditions and autoradiography (20 hr) were as described in Methods. Equal amounts of cell membrane protein was applied to each lane. Much longer periods of autoradiography (at least 10 days) were required for visualization of the TSHR in the TSHR-WT cells stably transfected with the 4 kb TSHR cDNA (26). Similar results were obtained in a second experiment.

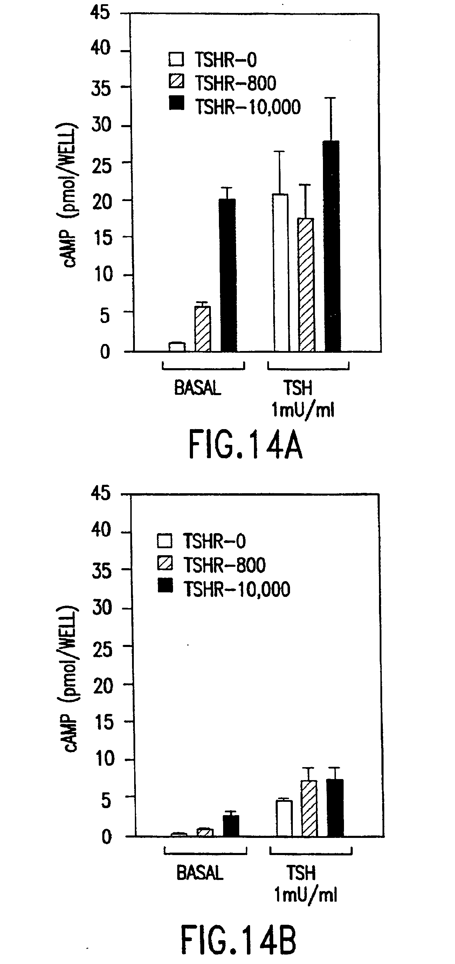

Figure 14: Cyclic cAMP levels in stably-transfected CHO cell lines expressing different numbers of TSHR. Cells were incubated in hypotonic medium (Fig. 14A) or in isotonic medium (Fig. 14B), as described in Methods. These media were either deficient in or were supplemented with, 1 mU/ml TSH ($10^{-9}$ M). This concentration is supramaximal in cells exposed to hypotonic medium (34,35) and slightly below the EC50 in cells incubated in isotonic medium (49.50). The data shown are the mean $\pm$ S.E. of values obtained in 3-4 experiments.

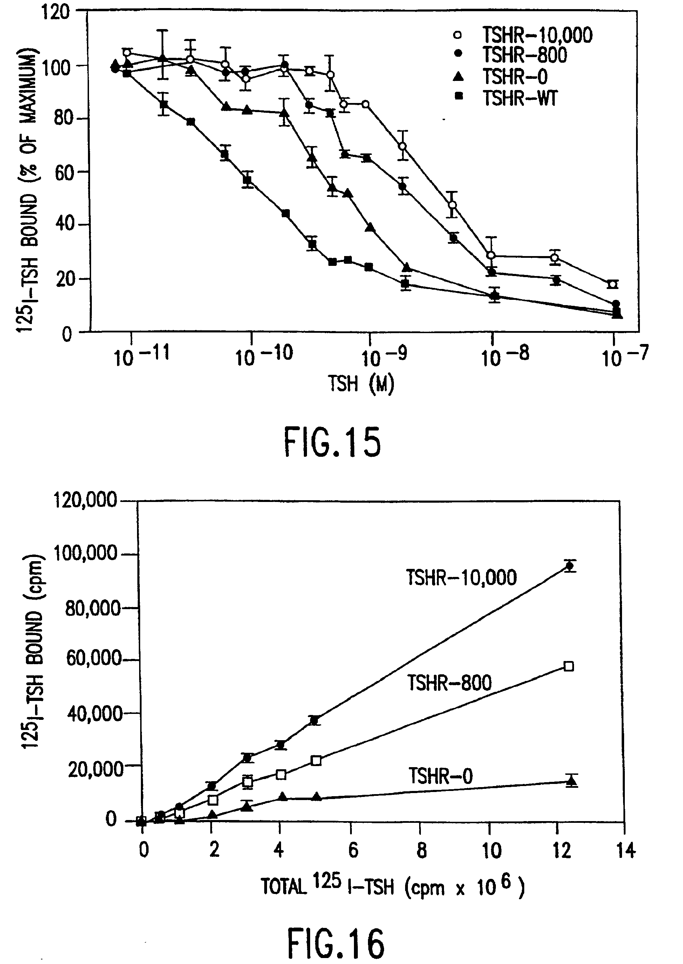

Figure 15: Binding of $^{125}$I-TSH to CHO cells expressing the TSHR in the presence of increasing concentrations of unlabeled TSH. The stably-transfected cells lines are describe in the legend to Fig. 12. Intact cells in monolayer were incubated in medium containing TSH, as described in Methods. The brackets indicate the mean $\pm$ S.E. of values obtained in triplicate dishes of cells. In order to attain partial receptor saturation with ligand, the TSHR-800 and TSHR-10,000 cells were diluted 1:50 with untransfected CHO cells. Because the maximum binding differed for each cell line, the values are expressed as a % of maximum TSH binding in the abence of unlabeled TSH. Similar results were observed when the TSHR-800 and TSHR-10,000 were diluted 1:200 in CHO cells, except that the maximum binding was lower.

Figure 16: TSH binding to cells expressing the TSHR by $^{125}$I-TSH in the absence of unlabeled TSH. Cells in 96-well plates were incubated in $^{125}$I-TSH concentrations upto $1.25 \times 10^6$ cpm/well ($2.5 \times 10^7$ cpm/well), as described in Methods. The data shown are the means $\pm$ S.E. of values obtained in triplicate dishes of cells. The data are representative of three separate experiments.

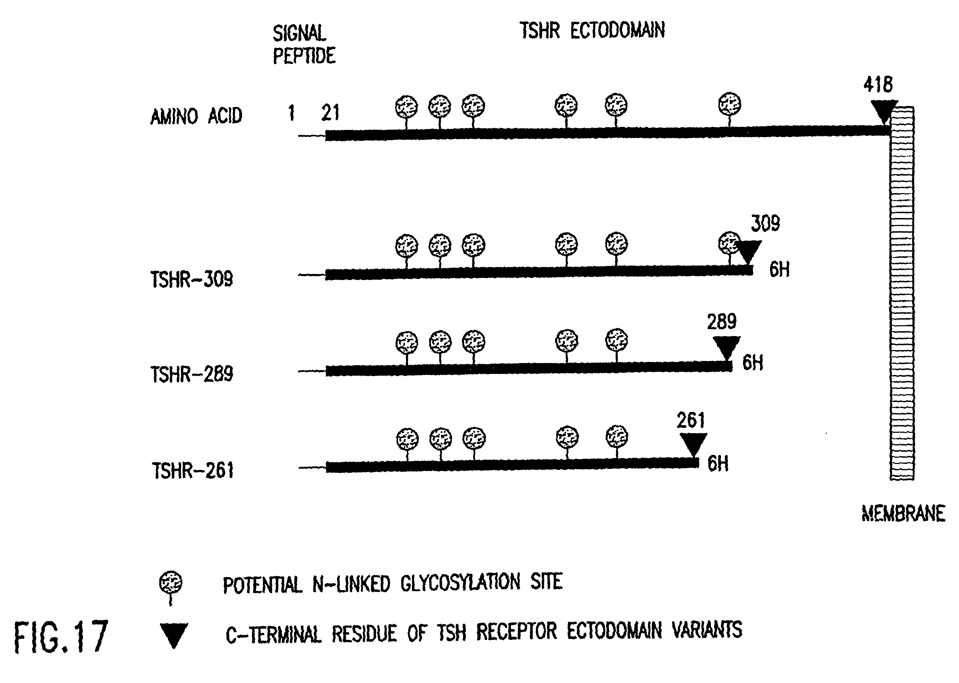

Figure 17: Schematic representation of three TSHR ectodomain variants truncated at their carboxyl-termini. The serpentine transmembrane and cytoplasmic portions of the holoreceptor (764 amino acid residues including signal peptide) are not shown. Six histidine residues (6H) followed by a stop codon are inserted after the indicated TSHR residues. Insertion of a stop codon at residue 418 has previously been shown to generate an ectodomain containing predominantly high mannose carbohydrate that is largely retained within the cell and is not recognized by TSHR-autoantibodies in Graves' patients sera (34). As shown in the present study, progressive carboxyl-terminal truncations of the TSHR ectodomain lead to a high level of secretion of material with mature, complex carbohydrate that can completely neutralize TSHR autoantibody activity.

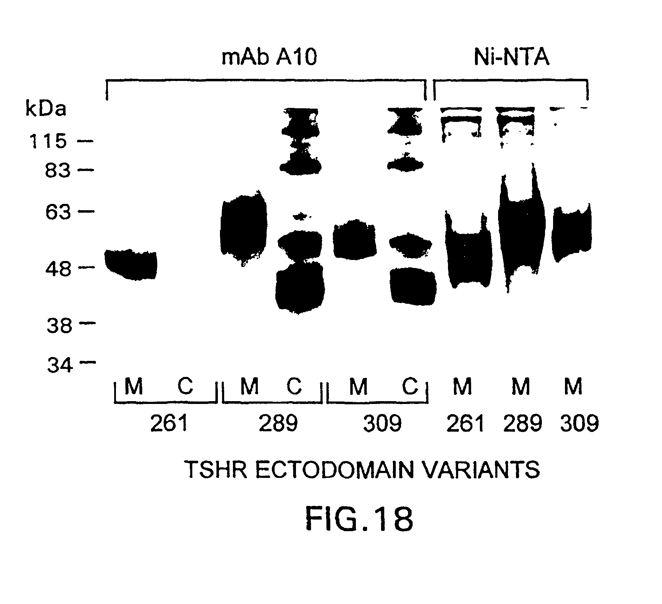

Figure 18: Relative secretion into the culture medium of TSHR ectodomain variants. CHO cells stably expressing TSHR ectodomain variants truncated at amino acid residues 261, 289 and 309 were precursor labeled for 1 hr followed by a chase of 16 hr (Methods). TSHR in medium (M) and cells (C) was then immunoprecipitated with a murine mAb (A10) to amino acid residues 22-35 (37). TSHR in medium was also recovered using Ni-NTA resin that binds to the 6 histidine residues inserted at the C-termini of the ectodomain variants. NI-NTA is not an effective method for identifying precursor-labeled TSHR in cells because of its interaction with a large number of CHO cell proteins. Autoradiography in the experiment shown was for 12 hr.

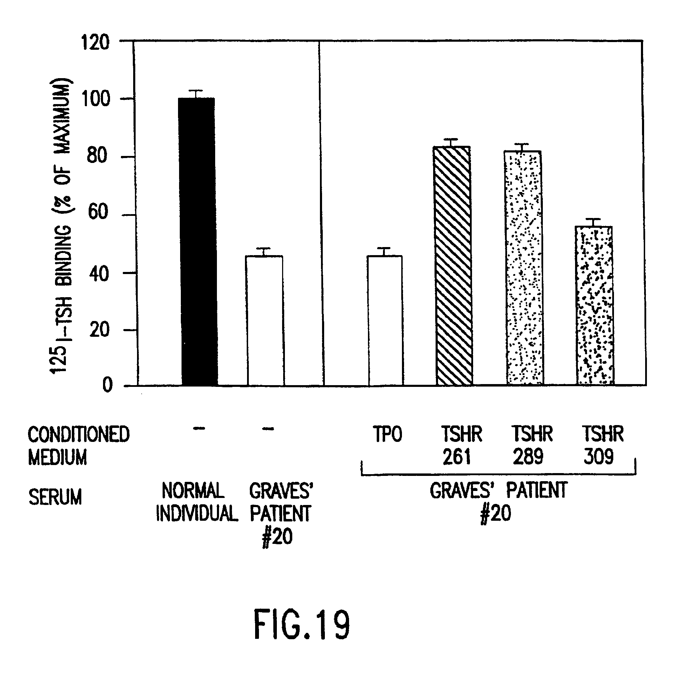

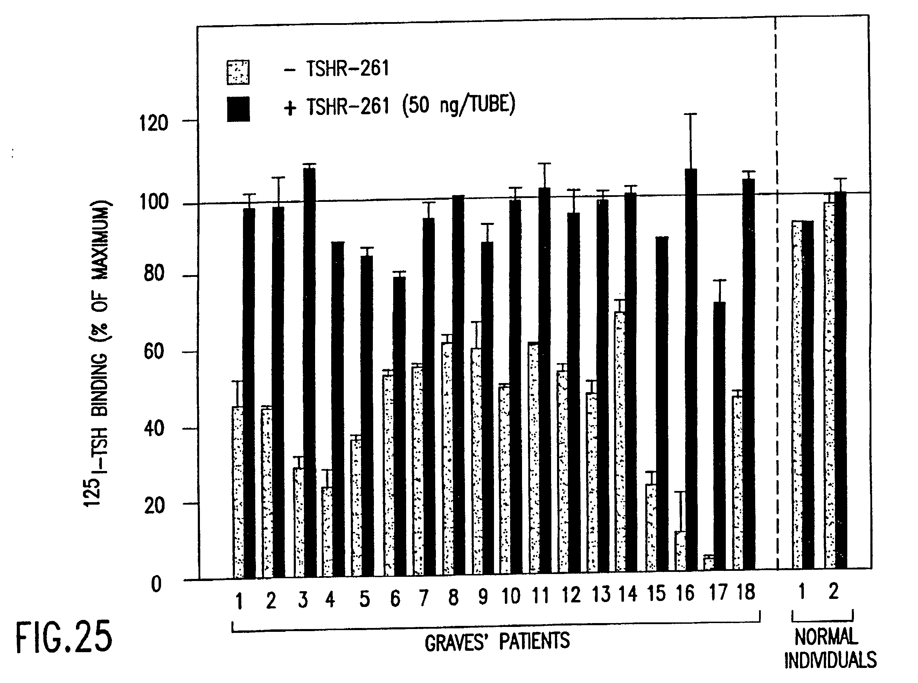

Figure 19: Recognition of TSHR ectodomain variants by TSHR autoantibodies in Graves' disease serum. The assay involves the ability of autoantibodies to compete for $^{125}$I.TSH binding to TSHR in solubilized porcine thyroid membranes (25)(Methods). Left panel: In the absence of conditioned medium from CHO cells, serum from a Graves' patient, unlike serum from a normal individual, reduces $^{125}$I-TSH binding by ~ 60%. Right panel: Conditioned medium from a non-relevant cell culture secreting thyroid peroxidase (TPO) has no effect on TSH binding inhibitory (TBI) activity. In contrast, conditioned medium from TSHR-261 and TSHR-289 cell cultures (unlike TSHR-309) nearly completely reverses the TBI activity. Bars indicate the mean $\pm$ range of duplicate determinations. See Fig. 25 for data on 18 Graves' sera using TSHR-261 after partial purification from conditioned medium.

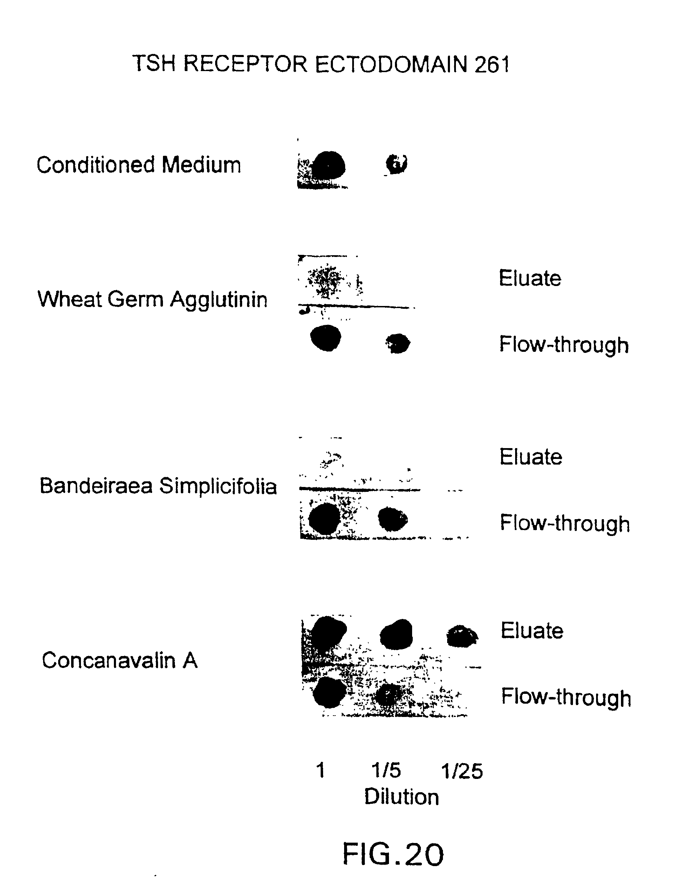

Figure 20: Lectin specificity for TSHR-261 ectodomain variant. Conditioned medium from CHO cell cultures expressing TSHR-261 was adsorbed with Sepharose linked to the indicated lectins (Methods). Both unadsorbed ("Flow-through") and material recovered from the beads ("Eluate") was spotted (neat or after dilution) on nitrocellulose filters and probed with mAb A 10 to the amino terminus of the TSHR.

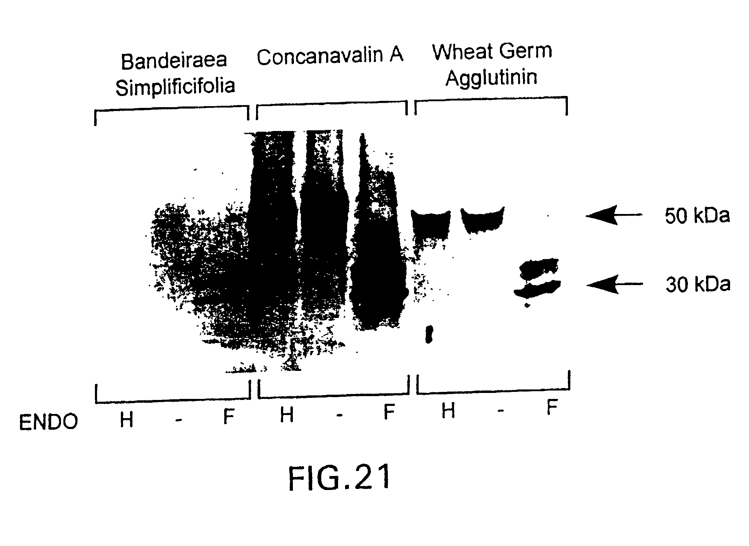

Figure 21: Immunoblots of TSHR-261enriched from conditioned medium using lectins.

Material obtained from equivalent volumes of the same medium using Bandeiraea simplificifolia, Concanavalin A or Wheat germ agglutinin was either left untreated (-) or was subjected to endoglycosidase H (Endo H) or endoglycosidase F (Endo F) digestion (Methods).

The samples were electrophoresed on a 10% polyacrylamide gel. Proteins were transferred to PVDF membrane and probed with murine mAb A10 (Methods).

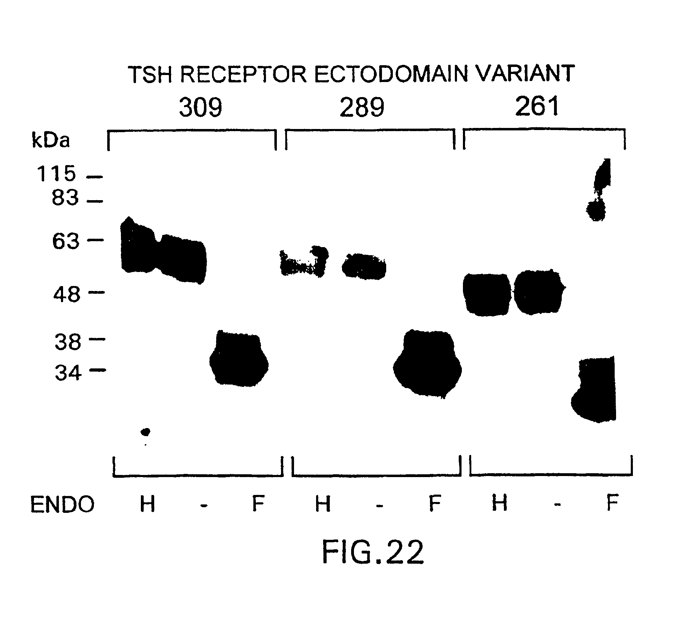

Figure 22: Immunoblot of TSHR ectodomain variants. TSHR-261, TSHR-289 and TSHR-309 were affinity-enriched from conditioned medium using Concanavalin A (Methods). Material was either left untreated (-) or was subjected to endoglycosidase H (Endo H) or endoglycosidase F (Endo F) digestion (Methods). The samples were electrophoresed on a 10% polyacrylamide gel. Proteins were transferred to PVDF membrane and probed with murine mAb A10 to amino acid residues 22-35 (37) using the ECL system (Methods).

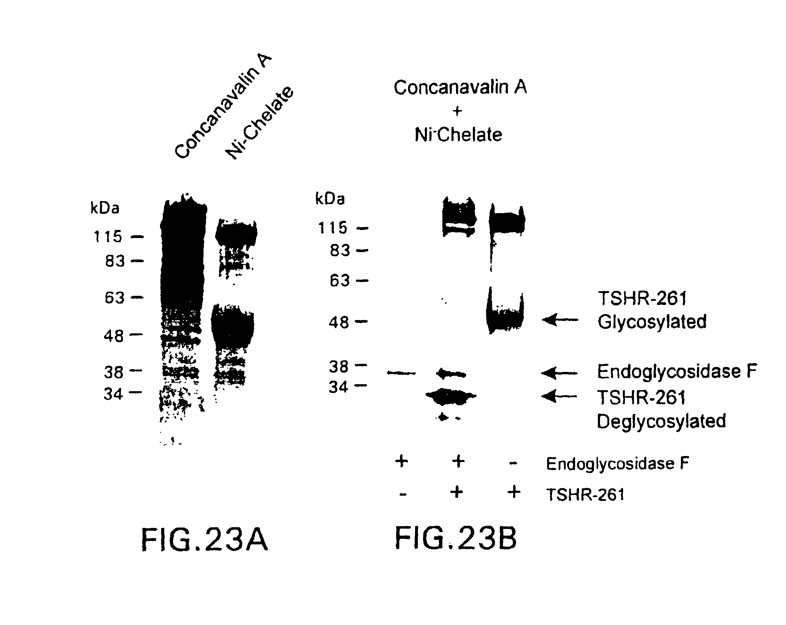

Figure 23: Direct visualization and quantitation of TSHR-261. left panel: Polyacrylamide gel electrophoresis and Coomassie blue staining of material recovered after capture from the conditioned medium with Concanavalin A (first lane) and after the subsequent Ni-chelate chromatography step (second lane). Right panel: Estimation of the TSHR-261 concentration by polyacrylamide gel electrophoresis of enzymatically deglycosylated, Coomassie blue-stained material. Deglycosylation yields a sharper, more intense band than the glycosylated protein. The recombinant endoglycosidase F present in the reaction provides an internal standard of 0.7 mg protein. The TSHR-261 polypeptide represents 60% of the mass of the glycosylated protein (see also Figs. 5,6).

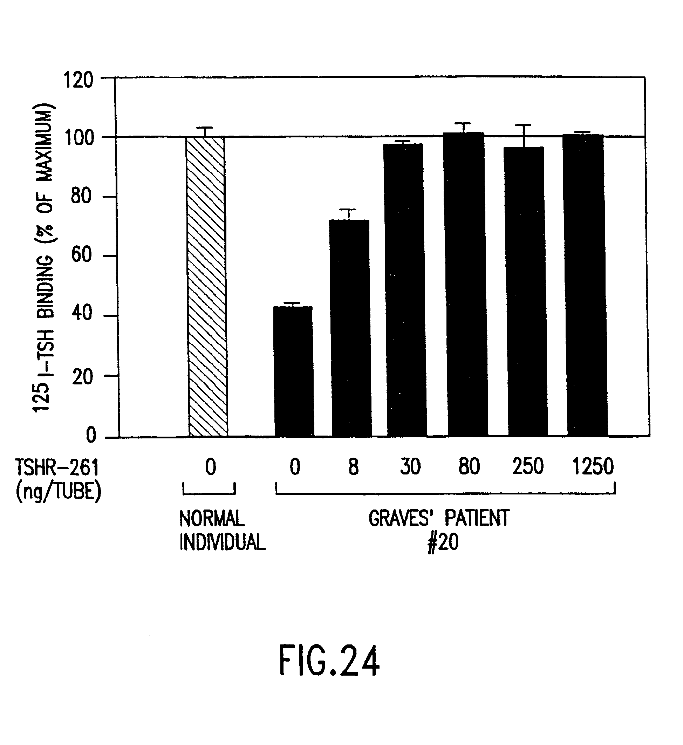

Figure 24: Titration of TSHR autoantibody neutralization by TSHR-261. Serum from a Graves' patient with moderate TSH binding inhibitory (TBI) activity was assayed using the commercial autoantibody kit (Methods) in the presence of increasing concentrations of partially purified TSHR-261. Serum from a normal individual does not inhibit $^{125}$I-TSH binding to solubilized porcine thyroid TSHR (hatched bar). In the absence of TSHR-261, TSH binding is reduced to - 40% of maximum. Incubation volume in the assay is 0.2 ml. Bars indicate the mean $\pm$ range of duplicate determinations.

Figure 25: Neutralization of TSHR autoantibodies by TSHR-261 partially purified from conditioned medium. TSH binding inhibition (TBI) activity of autoantibodies in the sera of 18 Graves' patients was measured using a commercial kit (Methods). These sera, unlike two sera from normal individuals, compete for $^{125}$I-TSH binding to solubilized membranes from porcine thyroids (hatched bars). Inclusion of TSHR-261 (50 ng per tube) neutralizes all or most of the autoantibody activity in the 18 sera. Bars indicate the mean $\pm$ range of duplicate determinations.

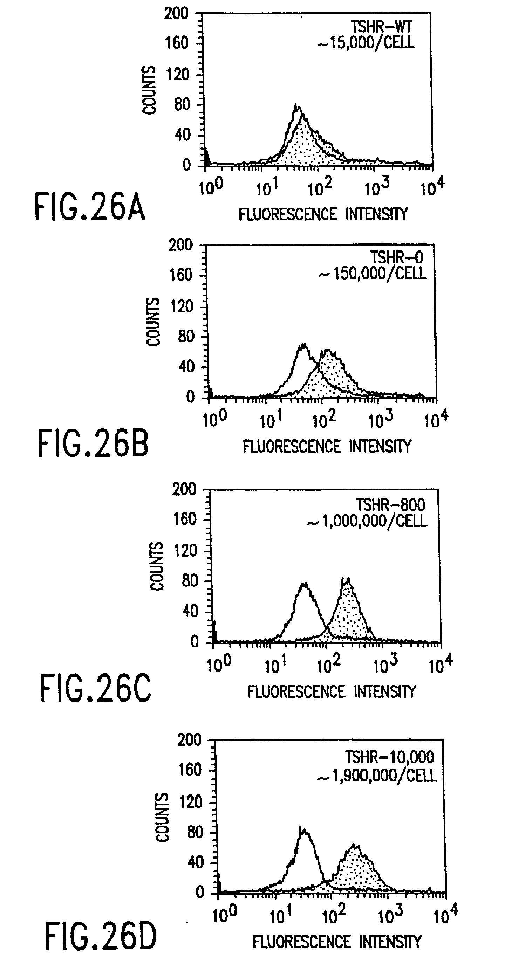

Figure 26: Flow cytometric analysis of IgG-class TSHR autoantibody binding to CHO cells expressing different numbers of TSHR on their surface. TSHR-WT cells are stably transfected with the 4 kb TSH cDNA (24). TSHR-0 cells contain the 2.3 kb translated region of the TSHR cDNA (25). In the TSHR-800 and TSHR-10,000 cells, the transgenome has been amplified and TSHR expression has been increased to ~ $10^6$ and - 1.9 x $10^6$ per cell, respectively (18). Cells were incubated with serum (1:10) from a normal individual (open histogram) and from a patient with Graves' disease (BB1) containing high levels of TSHR autoantibodies as measured by the TSH binding inhibition assay (shaded histogram). Fluorescence was developed as described in Methods.

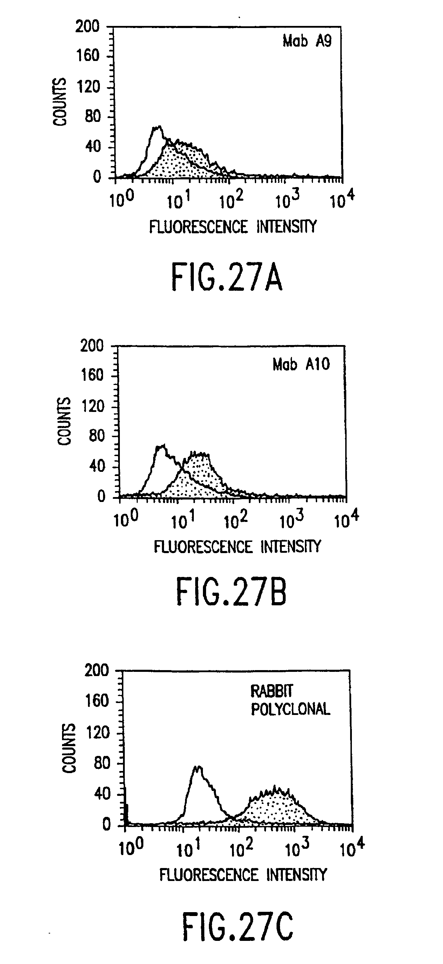

Figure 27: Flow cytometric analysis of TSHR-10,000 cells using mouse monoclonal and rabbit polyclonal antisera to the TSHR. TSHR-10,000 cells were incubated with mouse monoclonal antibodies A9 (1:100)(panel A) and A10 (1:100)(panel B), as well as with rabbit serum R8 (1:60)(panel C)(shaded histograms). Controls sera (open histograms) were a mouse monoclonal antibody to thyroid peroxidase and normal rabbit serum, all at the same the dilutions used for the specific sera. Fluorescence was developed as described in Methods.

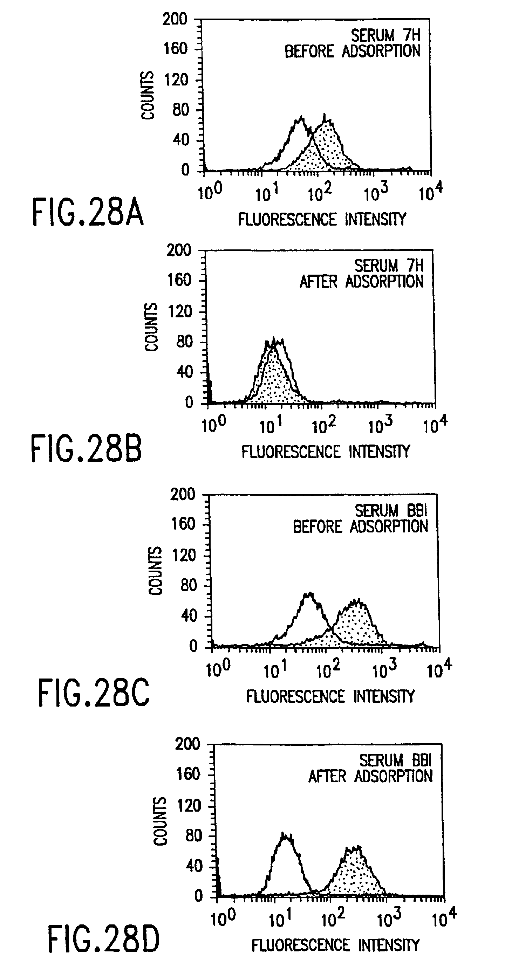

Figure 28: Effect of adsorption of sera with untransfected CHO cells on the specificity of the autoantibody fluorescence signal on flow cytometry with TSHR-expressing cells.

Representative examples are shown of sera from two individuals. 7H and BBI (shaded histograms), each with very high TBI values (81.7% and 100% inhibition, respectively). Sera (1:10 dilution) were either not preadsorbed (upper panels) or preadsorbed on untransfected CHO cells (lower panels) prior to incubation with TSHR-10,000 cells (Methods). Included as a negative control is serum from a normal individual without TSHR autoantibodies detectable by the TBI assay (open histogram).

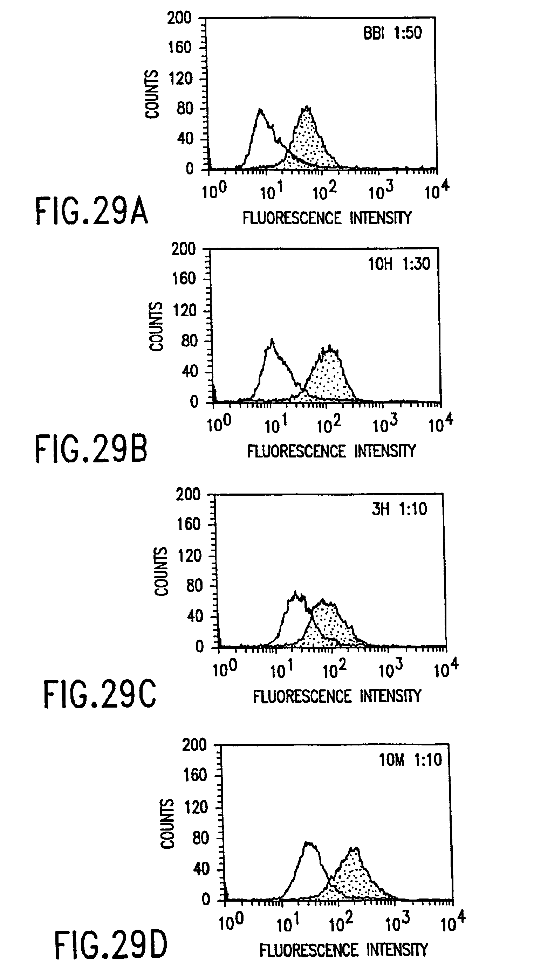

Figure 29: Adsorption of TSHR autoantibodies using cells expressing the recombinant TSHR on their surface. Sera BBI, 10H. 3H and 10M that generated a fluorescent signal on FACS analysis with TSHR-10,000 cells (Table 1) were preadsorbed (0.5 hours at room temperature, three times) on TSHR-10,000 cells prior to analysis using the same cells (open histograms).

Fluorescence generated by the same sera after preadsorption on untransfected CHO cells is shown by the shaded histograms. To optimize adsorption of TSHR autoantibodies, sera were diluted according to their respective titers in the TSH binding inhibition (TBI) assay (Table 2).

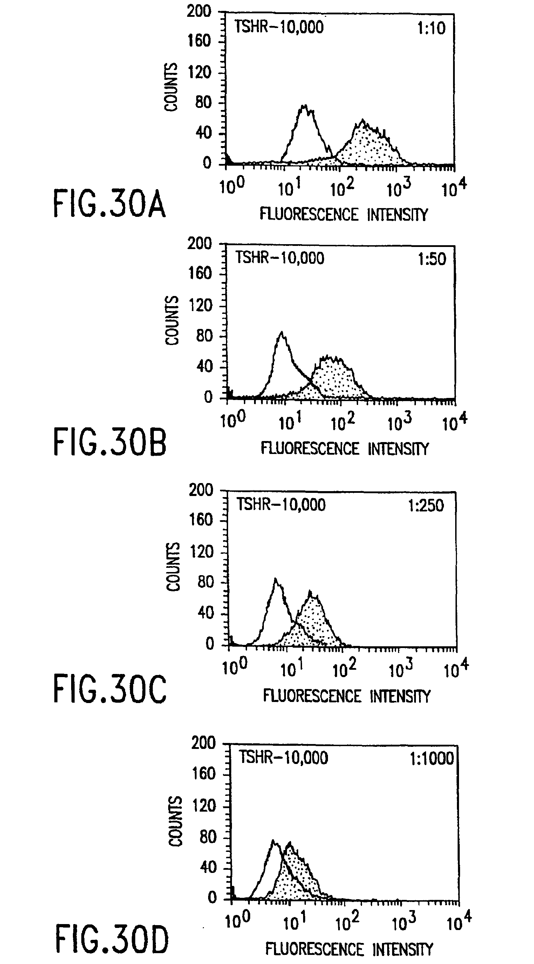

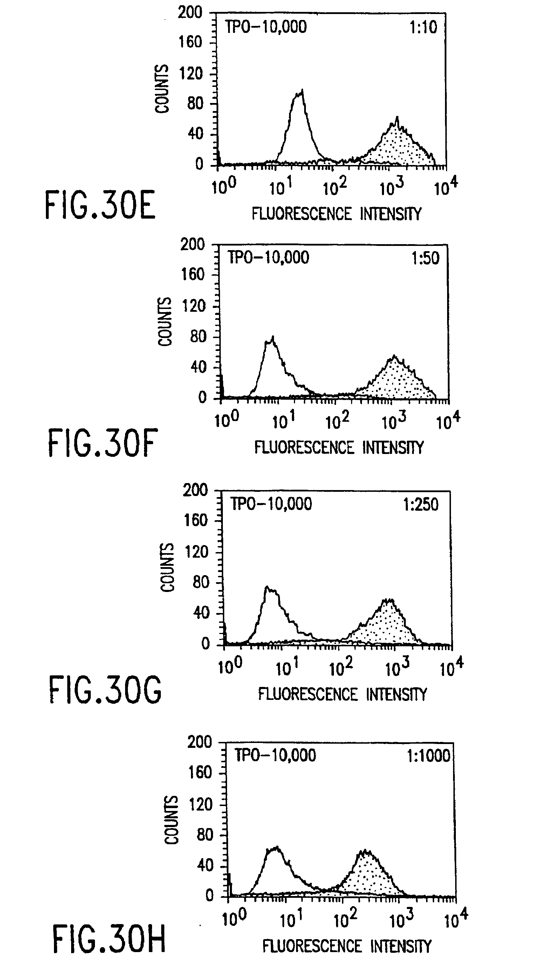

Figure 30: Relative titers of TPO and TSHR autoantibodies in the BBI serum, as determined by flow cytometry on CHO cells expressing either the TSHR or TPO on their surface. Both TSHR-10,000 cells (18) and CHO-TPO cells, previously described as C4C cells (20), contain genes amplified using the dihydrofolate reductase gene with cells resistant to 10,000 nM methotrexate. These two cell lines express similar numbers ($\sim 2 \times 10^6$) of TSHR or TPO molecules on their surface. Serum BB1 (shaded histograms) and scrum from a normal individual without TSHR or TPO autoantibodies detectable by clinical assay (open histograms) were tested at dilutions between 1:10 and 1:1000.

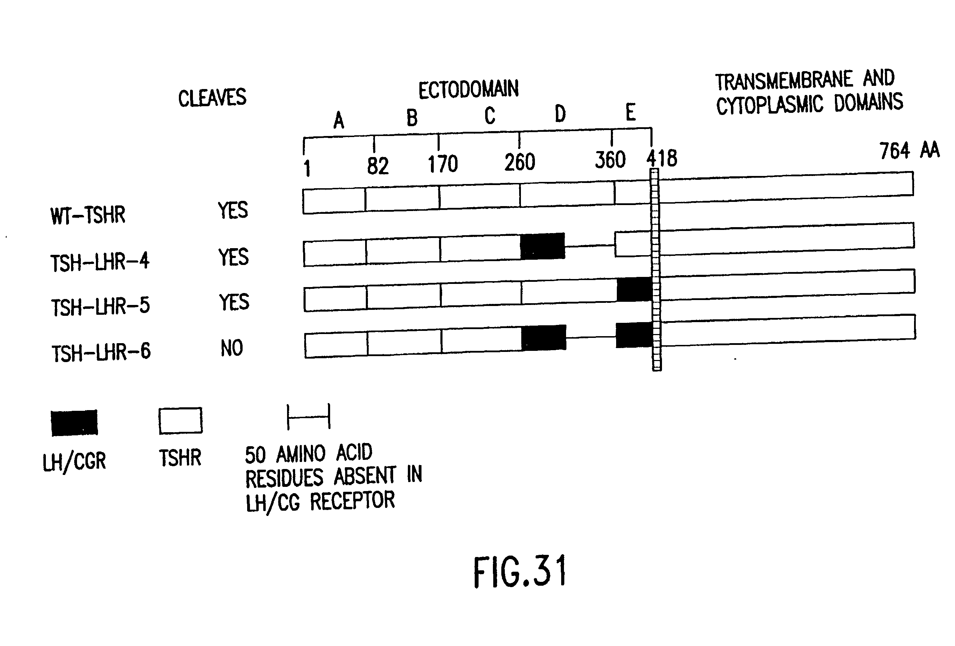

Figure 31: The ectodomain of the TSHR is shown divided into 5 arbitrary domains (A through E) that were used in creating chimeric TSH-LH/CG receptor molecules (19). Three chimeric receptors that are relevant to the present study are indicated. Note that amino acid residues 317-366 are not present in the LH/CG receptor. Therefore, when domain D of the TSHR is replaced with the corresponding segment of the LH/CG receptor, residues 317-360 are missing.

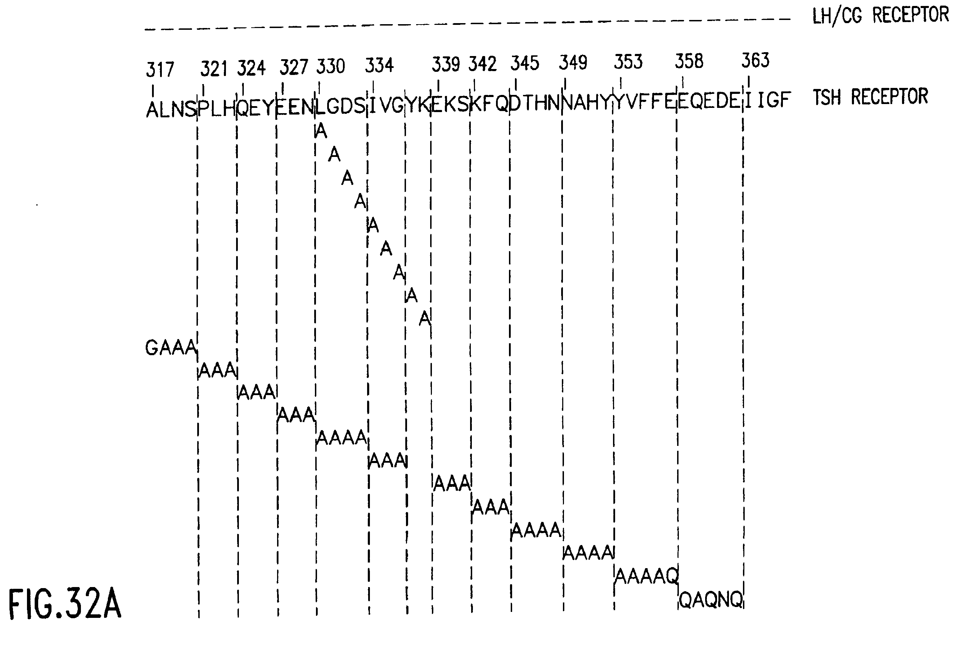

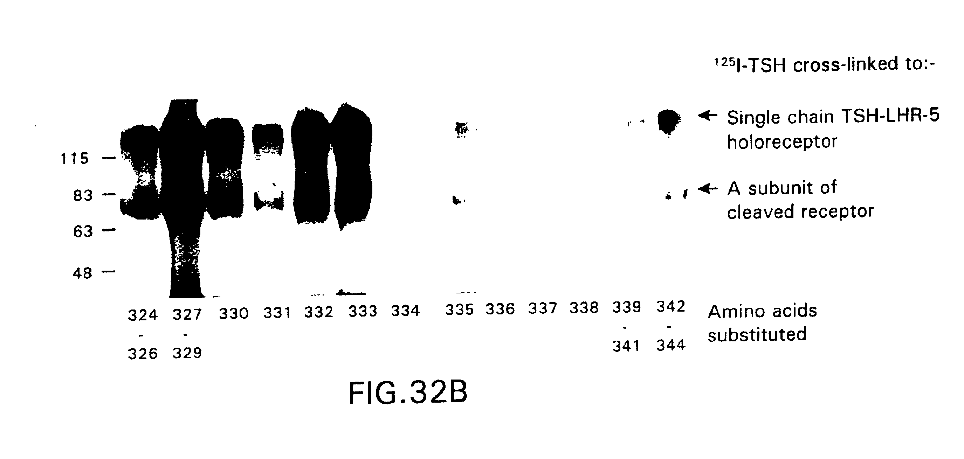

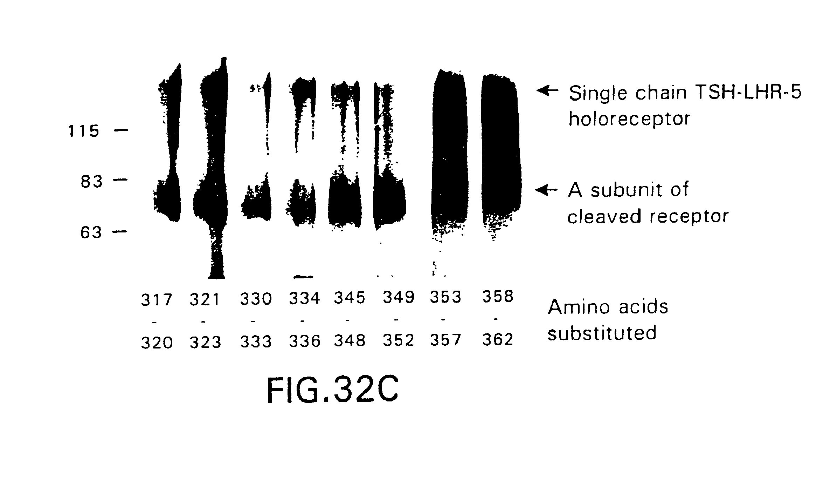

Figure 32: A. Amino acid substitutions introduced in the region of putative cleavage site I in the TSHR. Mutations were made in the D domain of chimeric receptor TSH-LHR-5 (Fig. 31). The dashed line for the LH/CG receptor indicates that this region is unique to the TSHR and is absent in the LH/CG receptor.

B. Radiolabeled TSH crosslinking to CHO cells stably expressing the receptors described in A. Cross-linked products were subjected to PAGE (7.5%) under reducing conditions followed by autoradiography. Note that the ligand, $^{125}$I-TSH, binds to the uncleaved TSH holoreceptor or to the ligand-binding A subunit in the cleaved TSHR, indicating the presence of both cleaved and uncleaved TSHR on the cell surface. The mass of the hormone ligand complex includes one subunit of the ligand, which itself contains two subunits linked by disulfide bonds. Under reducing conditions, only one ligand subunit (- 14 kDa) remains covalently linked to the TSHR.

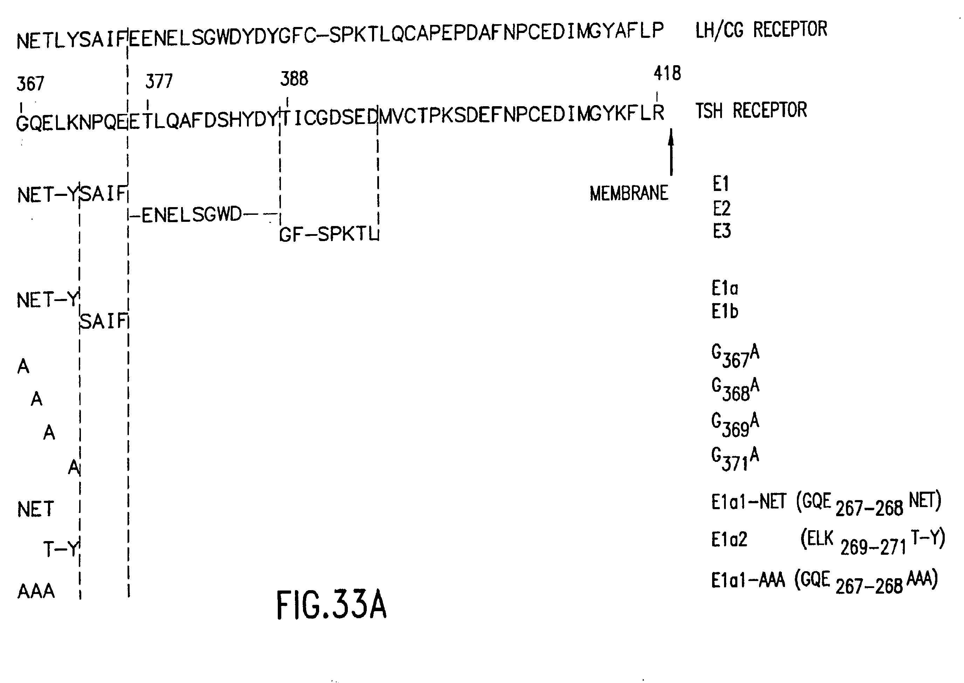

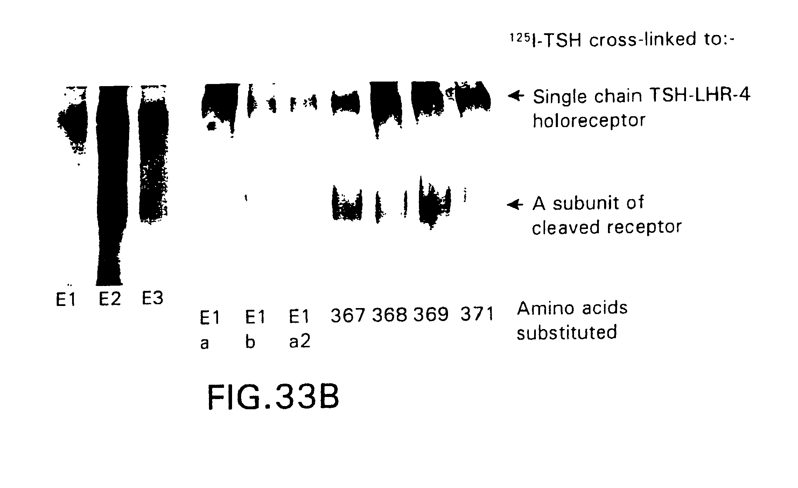

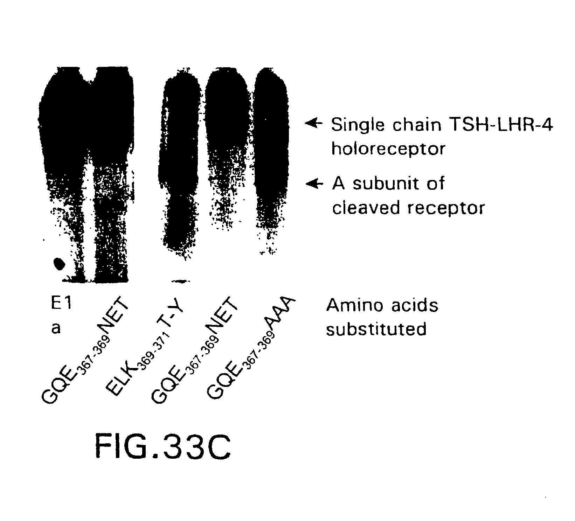

Figure 33: A. Amino acid substitutions introduced in the region of putative cleavage site 2 in the TSHR. Mutations were made in the E domain of chimeric receptor TSH-LHR-4 (Fig. 31). The mutations shown in bold prevent cleavage, as determined by TSH cross-linking. In the E1. E2 and E3 mutations "-" represents no substitution because the amino acid residues are the same in both receptors. In E3, "." represents the absence of an amino acid which is not present in the LH/CG receptor.

B. Radiolabeled TSH crosslinking to intact CHO cells stably expressing the receptors described in A. Cross-linked products were reduced in order to dissociate the subunits in cleaved receptors. The upper two panels depict autoradiograms of 7.5% gels, the lower two panels show material electrophoresed in 10% gels.

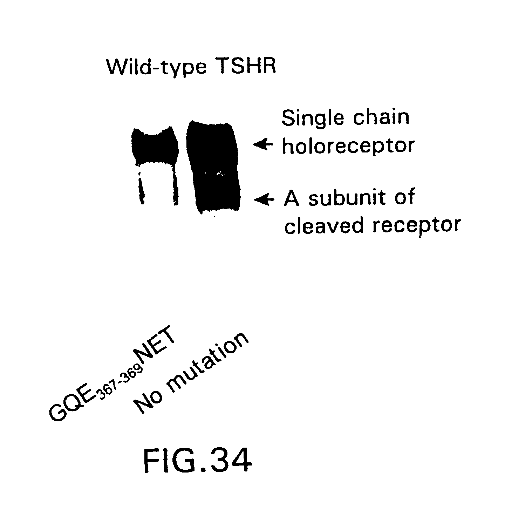

Figure 34: The presence of the GQE$_{267-269}$NET mutation does not prevent cleavage of the wild-type TSHR. Cross-linked $^{125}$I-TSH-TSHR products were reduced and subjected to PAGE (10%) and autoradiography.

## Description of the Preferred Embodiments

The present invention provides a method of producing a form of the human thyrotropin receptor (TSHR) ectodomain, comprising

a) culturing a host cell comprising a vector comprising a recombinant nucleic acid sequence encoding a secreted, soluble, complex carbohydrate-containing, endoglycosidase H resistant and endoglycosidase F sensitive form of the TSHR ectodomain which is C-terminal truncated at an amino acid residue selected from the group consisting of amino acid residues 261, 289 and 309 of the TSHR, wherein said culturing is under conditions allowing expression of said form of the TSHR ectodomain, and

b) recovering said form of the TSHR ectodomain.

In one embodiment, the vector is a plasmid. In one embodiment, the host cell is a mammalian cell. In one embodiment, the host cell is a Chinese Hamster Ovary cell. In one embodiment, the host cell is stably transfected.

Also described herein are human thyrotropin compositions which are useful in diagnostic and therapeutic methods for the diagnosis and treatment of autoimmune diseases, particularly Graves' disease.

Technical and scientific terms used herein have the meanings commonly understood by one of ordinary skill in the art to which the present invention pertains, unless otherwise defined. Reference is made herein to various methodologies known to those of skill in the art. Standard reference works setting forth the general principles of recombinant DNA technology include Sambrook, J.; et al., Molecular Cloning: A Laboratory Manual, 2d Ed., Cold Spring Harbor Laboratory Press, Plainview, New York (1989); Kaufman, P.B., et al., Eds., Handbook of Molecular and Cellular Methods in Biology and Medicine, CRC Press, Boca Raton (1995); McPherson, M.J., Ed., Directed Mutagenesis: A Practical Approach, IRL Press, Oxford (1991); Jones, J., Amino Acid and Peptide Synthesis, Oxford Science Publications, Oxford (1992); Austen, B. M. and Westwood, O. M. R., Protein Targeting and Secretion, IRL Press, Oxford (1991). Any suitable materials and/or methods known to those of skill can be utilized in carrying out the present invention; however, preferred materials and/or methods are described. Materials, reagents and the like to which reference is made in the following description and examples are obtainable from commercial sources, unless otherwise noted.

It has now been found that in an intact cell TBI assay, (i) the use of cells with more TSHR reduces, rather than improves the sensitivity of the assay and, (ii) the species of radiolabeled TSH (bovine vs. human) can influence the TBI value obtained. On the other hand, cells expressing very large numbers of receptors are an excellent source of detergent-solubilized TSHR, but only when the extraction procedure is modified according to the present invention. Described herein is the previously unknown ability to use recombinant human TSHR, rather than porcine TSHR, in practical and cost-efficient TBI assays.

Described herein are methods for the therapeutic treatment of autoimmune disease, and particularly, Graves' disease, involving administration of the therapeutic compositions described herein and such variants thereof as will be appreciated by those of skill. Accordingly, in one aspect described herein is a method for the treatment of Graves' disease comprising administering to a patient suffering from Graves' disease an effective amount of a composition comprising one or more of the substantially isolated human thyrotropin compositions described herein, or a variant thereof, alone or in conjunction with one or more other active ingredients. Such compositions described herein are preferably selected for use as described herein by means of one or more assay methods disclosed herein or known to those of skill.

Compositions useful as described herein are preferably substantially isolated human thyrotropin receptor, which may be recombinantly produced as described herein, or variants or mutants thereof, capable of modulating Graves' disease in a patient, and may be selected, as described herein, by means of appropriate assays. TSH compositions so selected may be administered by methods known to those of skill in order to achieve the desired therapeutic result.

Other variations in the compositions described herein may be desirable, for example, to increase or decrease the half life of the resulting peptide in the bloodstream or tissue. Thus, it is within the contemplated scope described herein to produce variants of compositions useful according to the methods described herein, by introducing therein alterations, such as are known in the art, which may include the use of synthetic or non-traditional amino acid residues, side chains, non-amide bonds as in peptides, and the like, which may act as blocking groups to protect the peptide against degradation. These and other methods of modifying compositions are well known in the art.

Compositions useful as described herein may be isolated from natural sources and purified. However, it is preferred to synthesize the compositions by means well known in the art. Preferred is solid phase synthesis, although any suitable method of synthesis may be employed.

Those of skill will appreciate that when treating populations of cells, such as thyroidal tissue cells, a therapeutic effect may be observed by any number of known clinical endpoints. The compounds described herein also have utility in combatting drug resistance, which may be a problem with current treatments. In this embodiment, the compounds described herein may be used in conjunction with other agents. Mechanisms of drug resistance are described, for example, in Remington's Pharmaceutical Sciences, 18th Edition, supra.

The quantities of active ingredient necessary for effective therapy will depend on many different factors, including means of administration, target site, physiological state of the patient, and other medicaments administered. Thus, treatment dosages should be titrated to optimize safety and efficacy. Typically, dosages used in vitro may provide useful guidance in the amounts useful for in situ administration of the active ingredients. Animal testing of effective doses for treatment of particular disorders will provide further predictive indication of human dosage. Various considerations are described, for example, Goodman and Gilman's The Pharmacological Basis of Therapeutics, 7th Ed., MacMillan Publishing Co., New York (1985), and Remington's Pharmaceutical Sciences 18th Ed., Mack Publishing Co., Easton, Penn (1990). Methods for administration are discussed therein, including oral, intravenous, intraperitoneal, intramuscular, transdermal, nasal, iontophoretic administration, and the like.

In selecting a dose for use in treating a patient as described herein, a treatment regimen will be selected which will achieve a sufficient concentration of the composition(s) to achieve the desired therapeutic effect in the target cells. Those of skill will be able to determine an effective dose, which will vary depending upon the manner and mode of administration, in order to achieve effective concentrations.

One method for administering the compositions described herein is inhalation of dry-powder formulations comprising the composition(s). In order to allow for absorption of the active components through the alveoli into the bloodstream, the powder must be very fine; on the order of 1-5 micron particles. The highly disbursable powder is delivered via an inhaler which generates an aerosol cloud containing the bolus of drug at the top of the inhalation chamber.

The compositions described herein will lend themselves to injection into the bloodstream of a patient. The half life of the active compositions so administered may be manipulated for best therapeutic effect by employing known drug technologies. One example of such technologies is known as DEPOFOAM phospholipid spheres (Depo Tech Corp., San Diego, CA), which gradually release the active component(s) over a period of days to weeks. This allows for a constant level of systemic concentration with lower initial drug levels and injection frequency.

Known methods of entrapping and stabilizing the compositions described herein may be employed to accommodate several different routes of drug delivery. One example of such technologies is the TECHNOSPHERE powder (Pharmaceutical Discovery Corp., Elmsford, No, which reliably forms two micron diameter spheres under conditions preserving the structural and functional integrity of the active peptide component. The pH-sensitive spheres, when injected into the blood, dissolve and release the active component, which is rapidly absorbed. Fine powders such as these are suitable for pulmonary, oral, intravenous and intraperitoneal administration.

The site of administration and cells will be selected by one of ordinary skill in the art based upon an understanding of the particular disorder being treated. In addition, the dosage, dosage frequency, and length of course of treatment, can be determined and optimized by one of ordinary skill in the art depending upon the particular disorder being treated. The particular mode of administration can also be readily selected by one of ordinary skill in the art and can include, for example, oral, intravenous, subcutaneous, intramuscular, etc. Principles of pharmaceutical dosage and drug delivery are known and are described, for example, in Ansel, H. C. and Popovich, N. G., Pharmaceutical Dosage Forms and Drug Delivery Systems, 5th Ed, Lea & Febiger, Pub., Philadelphia, PA (1990). It is possible, for example, to utilize liposomes to specifically deliver the agents described herein. Such liposomes can be produced so that they contain additional bioactive compounds and the like such as drugs, radioisotopes, lectins and toxins, which would act at the target site.

Nucleic acid compositions encoding the compositions and variants thereof useful as described herein will generally be in RNA or DNA forms, mixed polymeric forms, or any synthetic nucleotide structure capable of binding in a base-specific manner to a complementary strand of nucleic acid. Such a nucleic acid embodiment is typically derived from genomic DNA or cDNA, prepared by synthesis, or derived from combinations thereof. The DNA compositions generally include the complete coding region encoding the compositions, or fragments thereof.

By "functional equivalent" is meant a peptide possessing a biological activity or immunological characteristic substantially similar to that of a composition described herein, and is intended to include "fragments," "variants," "analogs," "homologs," or "chemical derivatives" possessing such activity or characteristic. Functional equivalents of a peptide as described herein, then, may not share an identical amino acid sequence, and conservative or non-conservative amino acid substitutions of conventional or unconventional amino acids are possible.

Reference herein to "conservative" amino acid substitution is intended to mean the interchangeability of amino acid residues having similar side chains. For example, glycine, alanine, valine, leucine and isoleucine make up a group of amino acids having aliphatic side chains; serine and threonine are amino acids having aliphatic-hydroxyl side chains; asparagine and glutamine are Amino acids having amide-containing side chains; phenylalanine, tyrosine and tryptophan are amino acids having aromatic side chains; lysine, arginine and histidine are amino acids having basic side chains; and cysteine and methionine are amino acids having sulfur-containing side chains. Interchanging one amino acid from a given group with another amino acid from that same group would be considered a conservative substitution. Preferred conservative substitution groups include asparagine-glutamine, alanine-valine, lysine-arginine, phenylalanine-tyrosine and valine-leucine-isoleucine.

A "mutant" as used herein refers to a peptide having an amino acid sequence which differs from that of a known peptide or protein by at least one amino acid. Mutants may have the same biological and immunological activity as the known protein. However, the biological or immunological activity of mutants may differ or be lacking. For example, a mutant may lack the biological activity which characterizes a TSHR as described herein, but may be useful as an epitopic determinant for raising antibodies or for the detection or purification of antibodies thereagainst, or as an agonist (competitive or non-competitive), antagonist, or partial agonist of the function thereof.

Suitable labels for use in assays as described herein include a detectable label such as an enzyme, radioactive isotope, fluorescent compound, chemiluminescent compound, or bioluminescent compound. Those of ordinary skill in the art will know of other suitable labels or will be able to ascertain such using routine experimentation. Furthermore, the binding of these labels to the peptides is accomplished using standard techniques known in the art.

The further isolation, purification and sequencing of TSHR as described herein may be accomplished by standard biochemical methods such as, for example, those described in Cantor, C., ed., Protein purification: Principles and Practice, Springer Verlag, Heidelberg, Publisher (1982); Hancock, W., ed., New Methods in Peptide Mapping for the Characterization of Proteins, CRC Press, Boca Raton, Florida, Publisher (1996), following the teachings herein.

Peptidomimetic agents are of use in the therapeutic treatment of disease. Such peptidomimetics are also described herein, and can act as drugs for the modulation of autoimmune disease, such as Graves' disease. Peptidomimetics are commonly understood in the pharmaceutical industry to include non-peptide drugs having properties analogous to those of the mimicked peptide. The principles and practices of peptidomimetic design are known in the art and are described, for example, in Fauchere J., Adv. Drug Res. 15: 29 (1986); and Evans, et al., J. Med. Chem. 30:1229 (1987). Peptidomimetics which bear structural similarity to therapeutically useful peptides may be used to produce an equivalent therapeutic or prophylactic effect. Typically, such peptidomimetics have one or more peptide linkages optionally replaced by a linkage which may convert desirable properties such as resistance to chemical breakdown *in vivo*. Such linkages may include -CH$_2$NH-, -CH$_2$S-, -CH$_2$-CH$_2$-, -CH=CH-, -COCH$_2$-, -CH(OH)CH$_2$-, and -CH$_2$SO-. Peptidomimetics may exhibit enhanced pharmacological properties (biological half life, absorption rates, etc.), different specificity, increased stability, production economies, lessened antigenicity and the like which makes their use as therapeutics particularly desirable.

It will be appreciated by those of skill that the precise chemical structure of compositions as described herein may vary depending upon a number of factors. For example, a given protein may be obtained as an acidic or basic salt, or in neutral form, since ionizable carboxyl and amino groups are found in the molecule. For the purposes of the disclosure, then, any form of peptide comprising human TSHR which retains the therapeutic or diagnostic activity of the compositions described herein is intended to be within the scope of the disclosure.

The compositions described herein may be produced by recombinant DNA techniques known in the art. For example, nucleotide sequences encoding human TSHR described herein may be inserted into a suitable DNA vector, such as a plasmid, and the vector used to transform a suitable host. The recombinant human TSHR is produced in the host by expression. The transformed host may be a prokaryotic or eukaryotic cell. Preferred nucleotide sequences for this purpose encoding a human TSHR are disclosed herein.

Synthetic polynucleotide sequences may be constructed by known chemical synthetic methods for the synthesis of oligonucleotides. Such synthetic methods are described, for example, in Blackburn, G.M. and Gait, M.J., Eds., Nucleic Acids in Chemistry and Biology, IRL Press, Oxford, England (1990), and it will be evident that commercially available oligonucleotide synthesizers also may be used according to the manufacturer's instructions. One such manufacturer is Applied Bio Systems.

Polymerase chain reaction (PCR) using primers based on the nucleotide sequence data disclosed herein may be used to amplify DNA fragments from mRNA pools, cDNA clone libraries or genomic DNA. PCR nucleotide amplification methods are known in the art and are described, for example, in Erlich, H.A., Ed., PCR Technology: Principles and Applications for DNA Amplification, Stockton Press, New York, New York (1989); U.S. Patent No. 4,683,202; U.S. Patent No. 4,800,159; and U.S. Patent No. 4,683,195. Various nucleotide deletions, additions and substitutions may be incorporated into the polynucleotides disclosed herein as will be recognized by those of skill, who will also recognize that variation in the nucleotide sequence encoding human TSHR may occur as a result of, for example, allelic polymorphisms, minor sequencing errors, and the like. The polynucleotides encoding the peptides described herein may include short oligonucleotides which are useful, for example, as hybridization probes and PCR primers. The polynucleotide sequences described herein also may comprise a portion of a larger polynucleotide and, through polynucleotide linkage, they may be fused, in frame, with one or more polynucleotide sequences encoding different proteins. In this event, the expressed protein may comprise a fusion protein. Of course, the polynucleotide sequences described herein may be used in the PCR method to detect the presence of mRNA encoding autoantibodies in the diagnosis of disease or in forensic analysis.

The sequence of amino acid residues in a protein or peptide is designated herein either through the use of their commonly employed three-letter designations or by their single-letter designations. A listing of these three-letter and one-letter designations may be found in textbooks such as Lehninger, A., Biochemistry, 2d Ed, Worth Publishers, New York, New York (1975). When the amino acid sequence is listed horizontally, the amino terminus is intended to be on the left end whereas the carboxy terminus is intended to be at the right end. The residues of amino acids in a peptide may be separated by hyphens. Such hyphens are intended solely to facilitate the presentation of a sequence.

Suitable agents for use as described herein include human TSHR and mimetics, fragments, functional equivalents and/or hybrids or mutants thereof, as well as mutants, and vectors containing cDNA encoding any of the foregoing. Agents can be administered alone or in combination with and/or concurrently with other suitable drugs and/or courses of therapy.

The agents described herein are suitable for the treatment of autoimmune diseases which are characterized by inappropriate cell death. Autoimmune diseases are disorders caused by an immune response directed against self antigens. Such diseases are characterized by the presence of circulating autoantibodies or cell-mediated immunity against autoantigens in conjunction with inflammatory lesions caused by immunologically competent cells or immune complexes in tissues containing the autoantigens. Such diseases include systemic lupus erythematosus (SLE), rheumatoid arthritis, and Graves' disease.

Standard reference works setting forth the general principles of immunology include Sell, S., Immunology, Immunopathology & Immunity, 5th Ed., Appleton & Lange, Publ., Stamford, CT (1996); Male, D., et al., Advanced Immunology, 3d Ed., Times Mirror Int'1 Publishers Ltd., Publ., London (1996); Stites, D. P., and Terr, A. I., Basic and Clinical Immunology, 7th Ed., Appleton & Lange, Publ., Norwalk, CT (1991); and Abbas, A. K., et al., Cellular and Molecular Immunology, W. B. Saunders Co., Publ., Philadelphia, PA (1991).

The human TSHR peptides, mimetics, agents and the like disclosed herein, as well as vectors comprising nucleotide sequences encoding them or their corresponding antisense sequences, and hosts comprising such vectors, may be used in the manufacture of medicaments for the treatment of diseases including autoimmune diseases.

The invention may be appreciated in certain aspects with reference to the following examples, offered by way of illustration, not by way of limitation.

## EXAMPLES

### EXAMPLE 1: Human Thyrotropin Receptor in a TSH Binding Inhibition (TBI) Assay for Thyrotropin Receptor Autoantibodies

Autoantibodies to the TSH receptor (TSHR) are the hallmark of the autoimmune response to the thyroid gland in Graves' disease (reviewed in 1). In most cases, these TSHR autoantibodies activate the receptor and lead to hyperthyroidism. More rarely, receptor occupancy by non-stimulatory TSHR autoantibodies can prevent TSH action and cause hypothyroidism (2-4). In contrast to autoantibodies to other thyroid autoantigens (thyroid peroxidase and thyroglobulin) there is, at present, no direct clinical assay for TSHR autoantibodies. Instead, these autoantibodies are detected either by their ability to inhibit radiolabeled TSH binding (TSH binding inhibition; TBI) or in a bioassay of TSHR activation (TSHR slimulatory immunoglobulin assay; TSI)(reviewed in 5). The most widely used assay is a TBI involving <u>porcine</u> TSHR solubilized with detergent from thyroid glands (6).

The molecular cloning of the TSHR cDNA led to the expectation that TBI assays using recombinant <u>human</u> TSHR would soon arise. However, although mammalian cell lines stably transfected with the human TSHR cDNA have been reported (7-12) and although large amounts of receptor protein have been generated in bacteria (13-18) and in insect cells (19-21) or as cell-free translates (22), recombinant human TSHR has failed to supplant the use of porcine TSHR in the TBI assay.

TSHR expressed in mammalian cells are well-recognized by autoantibodies in TBI assays involving intact cells (9,23), cell particulate fractions (10,24,25) and in detergent-solubilized membranes (25). However, assays using cultured cells are impractical for general use. Further, the small amount of recombinant TSHR recovered from mammalian cell paniculate fractions (10,25) makes use of this material prohibitively expensive.

There exists a need, therefore, for improved and practical TSH binding inhibition assays employing recombinant human TSHR compositions.

## METHODS

<u>Cell cultures</u>: Three different, stably-transfected Chinese hamster ovary (CHO) cell lines were used, each expressing different numbers of human TSHR on their cell surface (Table 1). The transgenome in the "4 kb" TSHR cells contains the full-length cDNA with both the 5' and 3' untranslated ends (7). TSHR-0 cells, expressing only the coding region of the TSHR cDNA, were renamed from the previously cumbersome pTSHR-5'3TR-NEO-ECE (26). The zero implies that the TSHR cDNA transgenome in these cells has not been amplified. The Transgenome in the TSHR-10,000 cells was amplified by adaptation of these cells to 10,000 nM methotrexate (27). For this study, all cell lines (cloned by limiting dilution in selection medium) were grown in Ham's F-12 medium supplemented with 10% fetal calf serum and standard antibiotics. Cells were cultured to confluence in either 10 cm diameter, 24 well cluster or 96 well microtiter culture dishes, as described in the text.

Table 1: Characteristics of cell lines expressing the human TSH receptor

| CHO cell lines | Receptors on cell surface | cDNA | Transgenome amplification | Reference |
|---|---|---|---|---|
| "4kb" TSHR | 16,000 | 4.0 kb | No | 7,26 |
| TSHR-0 | 150,000 | 2.3 kb | No | 26,27 |
| TSHR-10,000 | 1,900,000 | 2.3 kb | Yes | 27 |

Sera: 42 sera were used. All were kindly provided by Mr. Juan Tercero of Corning Nichols Institute, a reference laboratory to which the sera were sent for known or suspected Graves' disease. Sera were selected to provide a balanced spectrum TBI values (high, medium and low or negative) as determined by Corning Nichols. Sera were reassayed in our laboratory as described below.

TSH bindine inhibition (TBI) assay using intact cells: Highly purified bovine TSH (N.I.H.) or recombinant human TSH (Sigma, St. Louis, MO)(5 $\mu$g) was radiolabeled with $^{125}$I to a specific activity of - 80 $\mu$Ci/$\mu$g protein using the Bolton-Hunter reagent (4400 Ci/mmol; DuPont-New England Nuclear, Boston, MA) according to the protocol of the manufacturer, followed by Sephadex G-100 chromatography (28). "4kb" TSHR and TSHR-0 cells were grown to confluence in 24 well and 96 cells culture dishes, respectively. TBI activity was determined in a "two-step" assay as described previously (23) using polyethylene glycol (PEG)-precipitated IgG (23), with the following modifications. Cells were preincubated for 1.5 hr at 37°C with 0.25 ml (24 well plate) or 0.1 ml (96 well plate) of the IgG preparation in phosphate-buffered saline (PBS). In some assays using 96 well plates, when indicated, cells were preincubated in whole serum (50 ml) prior to rinsing and the addition of $^{125}$I-TSH. With the two-step assay, we found no difference between whole serum and PEG-precipitated IgG. After 2 rinses with "binding buffer" (Hank's buffer containing 280 mM sucrose instead of NaCl and supplemented with 0.25% bovine serum albumin)(29), $^{125}$I-TSH in binding buffer was added to the cells (2 hr at 37°C) in the amounts described in the text (250 ml and 50 ml per well in the 24 well and 96 well plates respectively). Bound $^{125}$I-TSH was measured as previously described (23). Non-specific binding to untransfected CHO cells was subtracted to obtain values for specific binding.

Solubilized TSH receptor preparation: Receptors were prepared from TSHR-10,000 cells in two procedures:-

(i) Cells removed from the culture dishes: Fifty confluent 10 cm diameter dishes of cells ($10^7$ cells per dish) were rinsed once with PBS and cells were resuspended by scraping into buffer A (10 mM Tris, pH 7.5, 0.1 mg/ml phenylmethylsulfonyl fluoride, 1 $\mu$g/ml leupeptin, 1 $\mu$g/ml aprotinin and 2 $\mu$g/ml pepstatin A; Sigma)(3 ml/dish). After brief homogenization with a Polytron (10 seconds x 3 at 4°C), the 500 - 20,000 x g particulate fraction was processed according to the protocol of Rees Smith et al. (6,30). The final extraction was with 5 ml of 10 mM Tris, pH 7.5, 50 mM NaCl and 1% Triton X-100. This material was used for TSH binding either directly or after dilution in the same buffer, as described in the text.

(ii) Direct extraction of cells in monolayer: Culture medium in one confluent 10 cm diameter dish of cells was removed and replaced with 3 ml of the 1% Triton X-100 buffer described above, supplemented with 5 mM EDTA, 5 mM EGTA, 5 mM N-ethylmaleimide, 10% glycerol, 0.5% BSA and 0.5% gelatin. After rocking for 2 hr at 4°C, the buffer was recovered, centrifuged (1 hr, 100,000 x g) and the supernatant used in the TBI assay as described above. The supplements to the buffer were found not to alter TSH binding in the assay.

TSH binding inhibition (TBI) assay using solubilized TSHR: Sera were assayed using TSHR antibody (TRAb) kits purchased from Kronus, San Clemente, CA. Reagents from this kit (from RSR Ltd., Cardiff, U.K.) were also used in conjuction with solubilized human TSHR, obtained as described above. TBI values were calculated as:-

$$1 - \frac{(\text{cpm serum sample} - \text{cpm in the absence of TSHR})}{(\text{cpm negative reference serum} - \text{cpm in the absence of TSHR})} \times 100$$

Thyroid stimulating immunoglobulin (TSI) assay: TSHR-0 cells, grown to confluence in 96 well culture plates, were assayed as previously described for human thyroid cells (31,32). This procedure employs the modification of using hypotonic medium (33). For this study, the following additional modifications were introduced:- IgG was precipitated with PEG (see above) and resuspended in the hypotonic medium supplemented with 10 mM Hepes, pH 7.4, 1 mM 3-isobutyl 1-methylxanthine and 0.3% BSA. Cells were incubated in this medium (0.1 ml) for 2 hr at 37°C. Cyclic AMP in the medium, diluted in 50 mM Na acetate, pH 6.2 and acetylated (32), was measured by radioimmunoassay using cAMP, 2'-O-succinyl $^{125}$I-iodotyrosine methyl ester (Dupont, Boston MA) and a rabbit anti-cAMP antibody (Calbiochem, San Diego CA). TSI activity was expressed as a percentage of the cyclic AMP value in the test serum relative to cAMP measured after concurrent incubation with serum from normal individuals.

## RESULTS

The TBI assays performed were of two main types (Table 2) involving either intact CHO cells or solubilized TSHR.

Table 2: Types of TSH binding inhibition (TBI) assays performed.

| Assay type | TSHR species | TSHR number per cell | Ligand (TSH) species | Receptor preparation |
|---|---|---|---|---|
| Intact cells | Human | 16,000 vs. 150,000 | Bovine | Monolayer |
| | Human | 150,000 | Bovine vs. Human | Monolayer |
| Solubilized TSHR | Human | 1,900,000 | Bovine | Scrape cells vs. Direct extraction of monolayer |
| | Human vs. Porcine | 1,900,000 vs Intact thyroid tissue | Bovine Bovine | Extraction of cell monolayer Commercial kit |

TSH binding inhibition (TBI) assay usine cells in monolayer: Previously, we reported data on a TBI assay using intact CHO cells stably transfected with the full-length 4 kb human TSHR- cDNA (23). The cells used in this assay (7), which expressed about 16,000 receptors per cell (26), were equal, if not better, in sensitivity to the commercially available, solubilized porcine TSHR assay (6). However, the availability of CHO cells expressing larger numbers of TSHR (26,27) prompted us to examine whether these cells would provide an even more effective TBI assay. To the contrary, in preliminary experiments, we unexpectedly found that the pig TSHR TBI assay was far superior to an assay using intact CHO cells overexpressing the human TSHR. For example, TBI values (% inhibition of $^{125}$I-TSH binding) with three potent sera were 79%, 91% and 83% with the commercial assay versus 10%, 13% and 57% with the CHO cells, respectively.

The deleterious effect on TBI sensitivity of a large number of TSHR per cell could be overcome, in part, by culturing fewer cells with more receptors in smaller wells. Thus, TSHR-0 cells (150,000 TSHR per cell)(26) cultured in microtiter (0.36 cm$^2$) wells provide only - 2-fold more receptors per well than the "4kb" TSHR cell line (16,000 TSHR per cell)(7,27) cultured in 24 well cluster plates (1.77 cm$^2$ wells). Two major advantages of microtiter plates were the use of less serum and a smaller volume of $^{125}$I-TSH with less background binding. By this means, the tracer concentration could be increased up to 100-fold, with a progressive increase in TSHR saturation (Fig. 1A). Nevertheless. TBI values with the TSHR-0 cells were lower than with the 4 kb TSHR cell line, reflecting the greater number of TSHR per well with the former cells (Fig. 1B).

Finally, in studies on intact human TSHR-expressing CHO cells, we compared the use of $^{125}$I-bovine (b) with $^{125}$I-human (h) TSH in a TBI assay using TSHR,-0 cells plated in microtiter wells. With 12 patients' sera covering a wide range of TBI values, the data obtained were generally similar regardless of the species of radiolabeled TSH used (Fig. 2). Nevertheless, of interest and potential importance, a few sera (for example, #1,3 and 6) produced greater inhibition of radioligand binding with human than with bovine TSH. It must be emphasized, however, that the use of hTSH in a TBI assay is presently limited because this species of TSH is a less effective ligand than bTSH (34). Indeed, in the experiment shown, maximal $^{125}$I-TSH binding was 5-fold lower with hTSH than with bTSH (4.9% vs. 24.8%).

TSH binding inhibition assay using detergent-extracted recombinant human TSHR: As mentioned above, intact CHO cells expressing large numbers of receptors on their surface cannot be used for TBI assays. However, we wished to determine whether such cells would be a good source of recombinant TSHR in a soluble receptor assay. For this purpose, we used cells (TSHR-10,000), that express very large numbers (- 1.9 x 10$^6$) of TSHR on their surface (27). Cells were suspended by scraping, homogenized in buffer containing 1% Triton X-100 and compared with the solubilized porcine TSHR in the universally used commercial kit as a standard. Recombinant receptor extracted from 7 x 10$^6$ cells was required to obtain $^{125}$I-bTSH binding comparable to that of the porcine TSHR standard (Fig. 3). Surprisingly and unexpectedly, in contrast to this low yield, far more TSHR capable of TSH binding was recovered when monolayers of the same cells were incubated with detergent-containing buffer without detaching the cells from the culture dishes. In this case, TSHR from 70-fold fewer ($\sim 10^5$) cells produced binding similar to the porcine TSHR standard. Indeed, a single 10 cm diameter dish of cells provided sufficient TSHR for 100-200 replicate determinations when substituted for the porcine TSHR in the kit.

The efficacy of the solubilized human and porcine TSHR was compared in a TBI assay using sera from 30 individuals with known or suspected Graves' disease. Ten of these sera had undetectable TSHR autoantibodies using the porcine TSHR in the kit (TBI < 15%). The remaining 20 scera contained a wide range of TSHR autoantibody activity (Fig. 4). TBI values obtained when human TSHR was substituted in the kit correlated very well with values determined with poreine TSHR (r = 0.954; p < 0.001). However, two sera that were negative with porcine TSHR antigen were now positive with the human TSHR (Fig. 4). Radiolabeled human TSH could not be used in the soluble TSHR assay because, unlike $^{125}$I-bTSH. polyethylene glycol precipitation of the tracer was strongly and variably influenced by the individual sera.

TBI values obtained with the solubilized porcine TSHR are known to correlate only weakly with thyroid stimulating activity determined in a bioassay (TSI) involving activation of the human TSHR (11,23,35). We, therefore wished to determine whether TBI values obtained with solubilized <u>human</u> TSHR correlated better with the bioactivity of IgG in the same sera. Sufficient serum was available from 28 of the 30 samples depicted in Fig. 4 to permit determination of TSI activity using CHO cells stably transfected with the human TSHR. The correlation between TBI and TSI activities was no better when human, rather than bovine, TSHR was used in the TBI assay (r = 0.732 and 0.709, respectively) (Fig. 5A and B).

## DISCUSSION

Disclosed herein are improved assays for the detection of TSHR autoantibodies. Surprisingly and unexpectedly, it has been found that mammalian cells expressing <u>more</u> TSHR on their surface provide a <u>less</u> sensitive TBI assay when this assay is performed with intact cells in monolayer culture. Although not intending to be bound by any particular theory, one likely reason for this phenomenon is the very low absolute concentration in serum of TSHR autoantibodies. Although previously suspected, only very recently has it been shown by flow cytometric analysis that TSHR autoantibodies are generally present at much lower concentrations than thyroid peroxidase (TPO) autoantibodies in the same serum (36). The efficacy of an occupancy assay such as the TBI assay depends on an excess of antibody over antigen. Thus, the combination of a large amount of antigen (TSHR) and a low concentration of antibody leads to low occupancy of the antigen. This, in turn, will reduce the sensitivity of the TBI assay, because many TSHR unoccupied by antibody are available for $^{125}$I-TSH binding. An optimum TBI assay as described herein, therefore, should utilize a small amount of receptor and a very effective ligand, as is the case with the affinity-purified bovine $^{125}$I-TSH in the procedure developed by Rees-Smith (6,30).

A TBI assay using cell monolayers is at a serious practical disadvantage relative to assays utilizing solubilized TSHR. However, although solubilized porcine TSHR preparations are clearly effective, human TSHR have been suggested to be the most appropriate for study of TSHR autoantibodies (30). To date, however, seven years after its molecular cloning (7,37,38), it is surprising that recombinant human TSHR has not supplanted the use of porcine material in a practical TBI assay. The most important contribution of the present invention is the demonstration for the first time that soluble, recombinant human TSHR can readily be obtained from mammalian cells in large amounts and in a form suitable for an effective TBI assay. Soon after a stably-transfected CHO cell line was generated (7), we found that TSH binding to cells scraped from culture dishes was greatly reduced when compared to binding to cells in monolayer culture; hence our initial use of intact cells in a TBI assay (23). Similarly, examination of the data of Costagliola *et al.,* reveals that the yield of solubilized TSHR from scraped JP09 cells is much lower than expected (25). The yield of solubilized TSHR from stably-transfected mouse myeloma cells grown to high density in a fermentor (10) has not been reported. The present invention demonstrates this low recovery of effective TSHR from resuspended cells and indicates that the direct extraction of TSHR from cell monolayers can overcome the evident fragility of this very difficult receptor. However, only a cell line such as TSHR-10,000, that expresses very high levels of TSHR, can provide TSHR suitable for direct use in a TBI assay as described herein without further purification or concentration, and is preferred on that basis.

There is evidence that TSHR species may be important in <u>bioassays</u> for stimulatory autoantibodies (11,35,39). Whether or not the use of human, rather than porcine, TSHR would be advantageous in a TBI assay has not heretofore been established. This factor was considered during the original development of the TBI assay using solubilized porcine TSHR (30). However, because TBI values in 18 sera did not differ greatly when TSHR of either species was used, and because of the easier access to porcine than to human thyroid tissue, porcine TSHR became the standard in TBI assays. It is worth noting that most sera in this study had relatively high T33I values, making discrimination at the very important low end of the assay difficult to discern.

The availability of solubilized recombinant human TSHR according to the present invention allowed reassessment of this question. Consistent with previous data on non-recombinant material (30), TRI values of 30 sera were generally comparable with solubilized porcine and human TSHR. However, our data indicate that a few sera that were negative in the porcine TSHR assay are positive when human TSHR is used.

Finally, the question arises whether, in addition to TSHR species, the species of the ligand (TSH) used in TBI assays is important. Human TSH is a very difficult ligand to use in a TRI assay because of its lower specific activity rclative to bovine TSH, even when interacting with the human TSHR. Nevertheless, in a TRI assay using intact cells, human TSH and bovine TSH ligands did not produce identical results with all sera. The reason for the variable background that, together with low absolute binding, precluded us from using human TSH in a solubilized TSHR TBI assay is uncertain.

## REFERENCES CITED IN EXAMPLE 1

1. Reels Smith, B, McLachlan, SM, Furmaniak, J. Autoantibodies to the thyrotropin receptor. Endocr Rev. 1988; 9: 106-121..

2. Orgiazzi, J, Williams. DE, Chopra, IJ, Solomon, DH. Human thyroid adenyl cyclase-stimulating activity in immunoglobulin G of patients with Graves' disease. J Clin Endocrinol Metab. 1976; 42:341-354.

3. Endo, K, Kasagi, K, Konishi, J, Ikekubo. K. Tatsuyb, O. Takeda. Y, Mori, T, Torizuka, K. Detection and properties of TSH-binding inhibitor immunoglobulins in patients with Graves' disease and Hashimoto's thyroiditis. J Clin Endocrinol Metab. 1978; 46:734-739.

4. Matsuura, N, Yamada, Y, Nohara, Y, Konishi, J. Kasagi, K, Endo, K, Kojima, K, Wataya. K. Familial neonatal transient hypothyroidism due to maternal TSH-binding inhibitor immunoglobulins. N Engl J Med. 1980; 303:738-741.

5. McKenzie, JM, Zakarija, M. Clinical Review 3: The clinical use of thyrotropin receptor antibody measurements. J Clin Endocrinol Metab. 1989; 69:1093-1096.

6. Shewring, GA, Rees Smith, B. An improved radioreceptor assay for TSH receptor antibodies. Clin.Endocrinol. 1982; 17:409-417.

7. Nagayama, Y, Kaufman, KD, Seto, P, Rapoport. B. Molecular cloning, sequence and functional expression of the cDNA for the human thyrotropin receptor. Biochem.Biophys.Res.Comm. 1989; 165:1184-1190.

8. Perret, J, Ludgate, M, Libert, F, Gerard, C, Dumont. JE, Vassan, G, Parmentier, M. Stable expression of the human TSH receptors in CHO cells and characterization of differentially expressing clones. Biochem.Biophys.Res.Comm. 1990; 171:1044-loss.

9. Harfst. E, Johnstone, AP. Gout, I, Taylor, AH, Walerlield, MD. Nussey. SS. The use of the amplifiable high-expression vector pEE14 to study the interactions of autoantibodies with recombinant human thyrotropin receptor. Molec.Cell.l.Endocrinol. 1992.83:117.123.

10. Matsuba, T, Yamada. M. Suzuki, H, Kanai, A, Isozaki, O, Yoshida, T, Tsushima, T, Yasukawa, K. Expression of recombinant human thyrotropin receptor in myeloma cells. J.Biochem. 1995; 118:265-270.

11. Murakami, M, Miyashita, K, Kakizaki, S, Saito. S. Yarnada, M. Iriuchijima, T, Takeuchi. T, Mori, M. Clinical usefulness of thyroid-stimulating antiobody measurement using Chinese hamster ovary cells expressing human thyrotropin receptors. Eur J Endocrinol. 1995; 133:80-86.

12. Kim, WB, Cho, BY, Park, HY, Lee, HK, Kohn, LD, Tahara, K, Koh, C-S. Epitopes for thyroid-stimulating antibodies in Graves' sera: A possible link of heterogeneity to differences in response to antithyroid drug treatment. J Clin Endocrinol Metab. 1996; 81:1758-1767.

13. Takai, O, Desai. RK, Seetharatnaiah, GS, Jones, CA, Allaway, GP, Akamizu, T, Kohn, LD, Prabhakar, BS. Prokaryotic expression of the thyrotropin receptor and identification of an immunogenic region of the protein using synthetic peptides. Biochem.Biophys.Ites.Comm. 1991; 179:319-326.

14. Loosfelt, H. Pichon, C, Jolivet A, Misrahi, M, Caillou, B. Jamous. M, Vannier. B, Milgrom, E. Two-subunit structure of the human thyrotropin receptor. Proc Natl Acad Sci USA. 1992; 895:3765-3769.

15. Harfst. E, Johnstone. AP, Nussey, SS. Characterization of the extracellular region of the human thyrotropin receptor expressed as a recombinant protein. J Mol Endocrinol. 1992; 9:227-236.

16. Huang, GC, Collison, KS, McGregor. AM, Banga, JP. Expression of a human thyrotropin receptor fragment in Escherichia coli and its interaction with the hormone and autoantibodies from patients with Graves' disease. J Mol Endocrinol. 1992; 8:137-144.

17. Costagliola, S, Alcalde, L, Ruf, J, Vassart, G, Ludgate, M. Overexpression of the extracellular domain of the thyrotrophin receptor in bacteria, production of thyrotrophin-binding inhibiting immunoglobulins. J Mol Endocrinol. 1994; 13:11-21.

18. Graves, PN, Vlase, H, Davies, TF. Folding of the recombinant human thyrotropin (TSH) receptor extracellular domain: identification of folded monomeric and tetrameric complexes that bind TSH receptor autoantibodies. Endocrinology. 1995; 136:521-527.

19. Huang, GC. Page, M, Nicholson, LB, Collison, KS, McGregor, AM. Banga. JP. The thyrotropin hormone receptor of Graves' disease: overexpression of the extracellular domain in insect cells using recombinant baculovirus, immunoaffinity purification and analysis of autoantibody binding. J Mol Endocrinol. 1993: 10:127-142.

20. Seetharamaiah, GS. Desai, RK, Dallas. JS, Tahara, K, Kohn, LD. Prabhakar, BS. Induction of TSH binding inhibitory immunoglobulins with the extracellular domain of human thyrotropin receptor produced using baculovims expression system. Autoimmunity. 1993; 14:315-320.

21. Vlase, H, Graves, P. Magnusson, RP, Davies. TF. Human autoantibodies to the thyrotropin receptor: recognition of linear, folded, and glycosylated recombinant extracellular domain. J Clin Endocrinol Metab. 1995; 80:46-53.

22. Morgenthaler, NG, Tremble, J, Huang, G, Scherbaum. WA, McGregor, AM, Banga, JP. Binding of antithyrotropin receptor autoantibodies in Graves' disease serum to nascent, in vitro translated thyrotropin receptor: Ability to map epitopes recognized by antibodies. J Clin Endocrinol Metab. 1996; 81:700-706.

23. Filetti, S, Foti, D, Costante. G, Rapoport, B. Recombinant human TSH receptor in a radioreceptor assay for the measurement of TSH receptor autoantibodies. J Clin Endocrinol Metab. 1991; 72:1096-1101.

24. Libert, F. Parmentier, M. Maenhaut, C, Lefort. A, Gerard, C, Ferret, J, Van Sande. J, Dumont, JE, Vassart, G. Molecular cloning of a dog thyrotropin (TSH) receptor variant. Molec.Cell.Endocrinol. 1990; 68: R15-RI7.

25. Costagliola, S, Swillens, S, Niccoli, P, Dumont, JE, Vassart, G, Ludgate, M. Binding assay for thyrotropin receptor autoantibodies using the recombinant receptor protein. J Clin Endocrinol Metab. 1992; 75:1540-1544.

26. Kakinuma, A, Chazenbalk. G, Filetti, S, McLachlan, SM, Rapoport, B. Both the 5' and 3' non-coding regions of the thyrotropin receptor messenger RNA influence the level of receptor protein expression in transfected mammalian cells. Endocrinology. 19996; 137:2664-2669.

27. Chazenbalk. GD, Kakinuma, A, Jaume, JC, McLachlan. SM, Rapoport, B. Evidence for negative cooperativity among human thyrotropin receptors overexpressed in mammalian cells. Endocrinology. 1996: 137:4586-4591.

28. Goldfine. ID, Amir, SM, Petersen, AW, Ingbar, SH. Preparation of biologically active 125I-TSH. Endocrinology. 1974; 95:1228-1233.

29. Tramontano, D, Ingbar, SH. Properties and regulation of the thyrotropin receptor in the FRTL5 rat thyroid cell line. Endocrinology. 1986; 118:1945-1951.

30. Rees Smith, B, Hall, R. Measurement of thyrotropin receptor antibodies. Methods in Enzymology. 1981, 74: 405-420.

31. Hinds. WE. Takai, N, Rapoport, B. Filetti, S, Clark, OH. Thyroid-stimulating immunoglobulin bioassay using cultured human thyroid cells. J Clin Endocrinol Metab. 1981; 52:1204-1210.

32. Rapoport. B. Greenspan. FS, Filetti, S, Pepitone, M. Clinical experience with a human thyroid cell bioassay for thyroid-stimulating immunoglobulin. J Clin Endocrinol Metab. 1984; 58:332-338.

33. Kasagi, K, Konishi, J, Iida, Y, Ikekubo, K, Mori, T, Kuma, K, Torizuka, K. A new in vitro assay for human thyroid stimulator using cultured thyroid cells: Effect of sodium chloride on adenosine 3'.5'-monophosphate increase. J Clin Endocrinol Metab. 1982; 54:108-114.

34. Pierce, JG, Parsons, If. Glycoprotein hormones: structure and function. Ann Rev Biochem. 1981; 50:465-495.

35. Vitti, P, Elisei, R, Tonacchera, M, Chiovato, L, Mancusi, F, Rago, T, Mammoli, C, Ludgate, M, Vassart, G, Pinchera, A. Detection of thyroid-stimulating antibody using Chinese hamster ovary cells transected with cloned human thyrotropin receptor. J.Clin.Endo.Metab. 1993; 76:499-503.

36. Jaume, JC, Kakinuma, A, Chazenbalk, GD, Rapoport, B, McLachlan. SM. TSH receptor autoantibodies in serum are present at much lower concentrations than thyroid peroxidase autoantibodies: Analysis by flow cytometry. J Clin Endocrinol Metab. 1997 (In Press)

37. Libert, F, Lefort, A, Gerard, C, Parmentier, M, Ferret. J, Ludgate, M, Dumont JE, Vassart, G. Cloning, sequencing and expression of the human thyrotropin (TSH) receptor: Evidence for binding of autoantibodies. Biochem.Biophys.Res.Comm. 1989; 165:1250-1255.

38. Misrahi, M, Loosfelt. H, Atger, M, Sar, S, Guiochon-Mantel, A, Milgrom, E. Cloning, sequencing and expression of human TSH receptor. Biochem.Biophys.Res.Comm. 1990; 166:394-403.

39. Endo, T, Ohmori, M. Ikeda, M. Anzai. E, Onaya, T. Heterogeneous responses of recombinant human thyrotropin receptor to immunoglobulins from patients with Graves' disease. BBRC. 1992; 186:1391-1396.

40. Beall, GN, Kruger, SR. Binding of 125I-human TSH by gamma globulins of sera containing thyroid-stimulating immunoglobulin (TSI). Life Sciences. 1983: 32:77-83.

41. Szkudlinski, MW, Teh, NG, Grossmann, M. Tropea, JE. Weintraub, BD. Engineering human glycoprotein hormone superactive analogues. Nature Biotech. 1996; 14:1257-1263.

## EXAMPLE 2: THE THYROTROPIN DOMAIN CONTAINS TWO CLEAVAGE SITES

The present Example presents the surprising and novel finding for G protein-coupled receptors that contrary to the prevailing concept of one cleavage site in the TSHR, there are, in fact, two such sites. The TSHR, like insulin, may release a C peptide during intramolecular cleavage into two subunits.

### INTRODUCTION

A two subunit form of the thyrotropin receptor (TSHR) has been recognized for many years. Thus, covalent crosslinking of radiolabeled TSH to thyrocyte membranes revealed a ligand-binding glycoprotein A subunit linked by disulfide bonds to a membrane-spanning B subunit (1). Because the TSHR is encoded by a single mRNA species (2-5), the A and B subunits must be formed by intramolecular cleavage. In addition to the two subunit form of the receptor, an uncleaved single chain TSHR is also present on the surface of cultured thyroid cells (6) and transfected mammalian cells (7).

The functional importance of TSHR cleavage is presently an enigma. Light trypsinization of cells expressing the TSHR leads to the loss of an epitope at amino acid residues 354-359 concomitant with receptor activation (8). On the other hand, TSH can activate chimeric TSH-LH/CG receptors that do not cleave into two subunits (9). Identification of the cleavage site in the TSHR ectodomain would be important for elucidating the structure-function relationship of the TSHR subunits. Seductions from studies of TSH crosslinking to TSHR mutants and chimeric TSH-LH/CG receptors (10) suggested a cleavage site closely upstream to amino acid residue 317 in the 418 residue ectodomain (numbering includes a putative 21 residue signal peptide). Other data obtained using a rabbit antiserum to the TSHR favored a cleavage site further downstream, in the vicinity of residue 366 (11). Mutagenesis of the three striking arginine- and lysine-rich clusters closely upstream to amino acid residue 317 did not prevent TSHR cleavage into two subunits, suggesting the absence of a role for classical subtilisin-related proprotein convertases (12). More recently, matrix metalloproteinases have been implicated in TSHR cleavage (13).

In the present study, we have used two new mammalian cell lines expressing the recombinant TSHR to refine further the site of cleavage in the ectodomain. One line (TSHR-10,000)(14) overexpresses the TSHR, thereby overcoming the handicap of a low signal to noise ratio experienced on direct immunodetection (without prior affinity purification) using conventional TSHR-expressing mammalian cell lines. A second cell line, TSHRmyc (15), contains a c-myc epitope tag in the region of the cleavage site. Data obtained with these cell lines provide a surprising and novel finding for G protein-coupled receptors; namely there appear to he two, not one, cleavage site in the human TSHR ectodomain. A corollary of this finding is that the TSHR, like insulin, may released a C peptide during intramolecular cleavage.

## METHODS

Cells expressing the TSHR: (i) TSHR-10,000 (14) is a Chinese hamster ovary (CHO) cell line overexpressing the human TSHR ($\sim 2 \times 10^6$ receptors per cell). Overexpression was attained using a dihydrofolate reductase minigene to amplify the stably-transfected TSHR cDNA transgenome. (ii) TSHR-() are CHO cells expressing the same TSHR cDNA,-but without transgenome amplitication (-1.5 x $10^5$ receptors per ccll)(14.16). (iii) TSHRmyc are 293 human embryonal kidney (HEK) cells stably expressing the unamplified gene for an epitope-tagged human TSHR (15). Epitope-tagging was achieved by replacing TSHR amino acids 338 to 349 with the human c-myc peptide EEQKLISEEDLL. Cells were propagated in Ham's F-12 medium (CHO cells) or Dulbecco's modified Eagle's medium (DMEM)(293 HEK cells), supplemented with 10% fetal calf serum (FCS), penicillin (100 U/ml), gentamicin (50 $\mu$g/ml) and amphotericin B (2.5 $\mu$g/ml).

Immunoprecipitation of precursor-labeled TSHR: Cells near confluence in 100 mm diameter culture dishes were rinsed with phosphate-buffered saline (PBS) and pre-incubated (0.5 h, twice) in DME-H21 methionine- and cysteine-free medium containing 5% heat-inactivated FCS. The cells were then pulsed (1 h at 37 C) in 5 ml fresh medium supplemented with ~ 0.5 mCi of $^{35}$S-methionine/cysteine (> 1000 Ci/mmole, DuPont NEN, Wilmington DE). After aspiration of the medium and rinsing the cells once with PBS, chase was performed for the indicated times in standard, non-selective medium with 10% FCS. Cells were washed twice with PBS and scraped into 1 ml ice-cold 20 mM Hepes. pH 7.2, 150 mM NaCl (buffer A) containing the protease inhibitors phenylmethylsulfonyl fluoride (PMSF) (100 $\mu$g/ml), leupeptin (1$\mu$g/ml) aprotinin (1$\mu$g/ml), and pepstatin A (2 $\mu$g/ml) (all from Sigma, St. Louis, MO). The cells were pelleted (5 min, 100 x g), washed twice with PBS and resuspended in buffer A containing 1% Triton X-100. After 90 min at 4°C with occasional vortexing, the mixture was centrifuged for 45 min at 100.000 x g and the supernatant was diluted 1:4 in immunoprecipitation buffer (20 mM Hepes pH 7.2. 300 mM NaCl, 0.1% sodium dodecyl sulfate. 0.5% Nonidet-P40, 2 mM EDTA). The solubilized cell proteins were precleared for 1 h at 4°C with - 150 $\mu$g normal mouse serum IgG prebound to 25 $\mu$l packed and washed protein A-agarosc (Sigma). The protein A was removed by centrifugation (3 min at 10,000 X g) in a microcentrifuge. Mouse monoclonal antibodies (mAb) were then added, as described in the text. A10 and A11 (kind gifts of Dr Paul Banga, London. U.K.) both recognize TSHR amino acid residues 22-35 (17) and were used at a final dilution of 1:1000. The anti-myc mAb 9E10 (obtained from ATCC) was used at a final dilution of 1:500. T3-365, a mAb to the TSHR B subunit (kindly provided by Drs. E. Milgrom and H. Loosfelt, Le Kremlin-Bicetre, France) and a mAb to human thyroid peroxidase (kindly provided by Dr. Scott Hutchison, Nichols Institute, San Juan Capistrano, CA, USA) were used at a dilution of 1:1000. After 3 h at 4°C, 25 $\mu$l packed and washed protein A-agarose was added and the tubes were tumbled for 1 h at 4°C. The protein A was recovered by centrifugation for 3 min at 10,000 X g (4°C), washed 5 times with I ml immunoprecipitation buffer, and then once with 10 mM Tris, pH 7.4, 2 mM EDTA and 0.5% sodium dodecyl sulfate (SDS). Finally the pellet was resuspended in Laemmli sample buffer (18) with 0.7 M -mercaptoethanol (30 min at 50°C) and electrophoresed on 7.5 % or 10% SDS-polyacrylamide gels (BioRad, Hercules, CA). Prestained molecular weight markers (BioRad) were included in parallel lanes. We precalibrated these markers against more accurate unstained markers to obtain the molecular weights indicated in the text. Radiolabeled proteins were visualized by autoradiography on Kodak XAR-5 X-ray film (Eastman Kodak, Rochester, NY).

Immunoblots of TSHR. proteins: Stably transfected TSHR-10,000 cells (in two 100 mm diameter dishes) were resuspended by incubation in Ca++- and Mg++-free PBS with 0.5 mM EDTA. The cells were pelleted (5 min., 100 x g, 4°C), resuspended in 1.5 ml of 10 mM Tris-HCl, pH 7.4, containing the protease inhibitors described above and homogenized with a Polytron homogenizer (Brinkman Instruments, Westbury, CT). After centrifugation for 10 min at 500 x g (4°C), the supernatant was recentrifuged for 20 min at 10,000 x g (4°C). The pellet was resuspended in 0.1 ml of the same buffer, after which Laemmli buffer with 0.7 M β-mercaptoethanol was added (30 min at 50°C) and the sample electrophoresed on 10% SDS-polyact-ylamide gels. Prestained molecular weight markers are described above. Proteins were transferred to ProBlott membranes (Applied Biosystems, Foster City, CA), which were processed as described previously (19). Membranes were incubated overnight (4°C) with mAb A10 or A11 to the A subunit, or with mAb to the B subunit; T3-495 (TSH-R1; Transbio, Boulogne. France) or T3-365 (final dilutions of 1:1000). After rinsing, the membranes were incubated for 1 hr at room temperature with alkaline phosphatase conjugated goat anti-mouse immunoglobulin G (1:400 dilution)(Cappel. Durham, NC). The signal was developed with nitroblue tetrazolium and 5-bromo. 4-chloro, 3-indolyl phosphate in 100mM Tris-HCl buffer, pH 9.5, containing 100 mM NaCl and 5 mM MgCl$_2$. Enzymatic deglycosylation of TSHR protein: The Protein A/IgG/TSHR complex or the 10,000 x g crude membrane fraction (see above) were incubated (10 min., 100°C) in denaturing buffer containing 0.5% SDS, 1% β-mercaptoethanol. Enzymatic deglycosylation was performed according to the protocol of the manufacturer (N.E. Biolabs, Beverly MA). N-glycosidase F digestion (100 U for 2 h at 37°C) was in 50 mM Na phosphate. pH 7.5, 1% NP-40. Endoglycosidase H digestion (50 U for 2 h at 37°C) was in 50 mM Na citrate, pH 5.5. Samples were then subjected to SDS-PAGE, as described above.

Covalent cross-linking of radiolabeled TSH: Highly purified bovine TSH (5 μg, 30 U/mg protein) was radiolabeled with $^{125}$I to a specific activity of ~ 80 μCi/μg protein using the Bolton-Hunter reagent (4400 Ci/mmol; DuPont-NEN) according to the protocol of the manufacturer, followed by Sephadex G-100 chromatography (20). Confluent 100 mm diameter dishes of TSHR-expressing cells were incubated for 2h at 37 C with 5 μCi $^{125}$I-TSH in 5 ml modified Hank's buffer (without NaCl), supplemented with 280 mM sucrose and 0.25% BSA (binding buffer). Unbound $^{125}$I-TSH was removed by rinsing the cells three times with ice-cold binding buffer. Disuccinimidyl suberate (DSS; 1 mM; Sigma) in 10 mM Na phosphate buffer, pH 7.4, containing the protease inhibitors described above was then added for 20 min at room temperature. The cross-linking reaction was terminated by the addition of 20 mM ammonium acetate (final concentration).

After cross-linking, the cells were rinsed twice with PBS and scraped into 10 mM Tris, pH 7.5, containing the same protease inhibitors. Cells were homogenized using a Polytron homogenizer and centrifuged for 5 min at 4°C (.500 x g). The supernatant was centrifuged (15 min, 10,000 x g, 4°C) and the pellet was resuspended in 50 μl 10 mM Tris, pH 7.5. After the addition of Laemmli buffer containing 0.7 M β-mercaptocthanol (30 min at 42°C). the samples were subjected to 10% SDS-PAGE and autoradiography as described above.

## RESULTS

Immunodetection of the TSHR in TSHR-10,000 cells: Immunoprecipitation studies of precursor-labeled TSHR.-10,000 cells were performed with mAb A10 (17) to TSHR amino acid residues 22-35 at the amino terminus of the A subunit. Multiple forms of the TSHR were observed under reducing conditions after chase periods of 3 hr and 16 (Fig. 61A). Two forms of single subunit (uncleaved) TSHR were:- (i) ~ 15 kDa in size (complex carbohydrate resistant to endoglycosidase H) and, (ii) ~ 100 kDa in size (immature, high mannose carbohydrate sensitive to endoglycosidase H) (Fig. 6). N-glycosidase F digestion removed both forms of carbohydrate, exposing a -84 kDa polypeptide backbone. Cleaved (two subunit) TSHR. was also present. The extracellular A subunit was visible as a broad -62 kDa band with complex carbohydrate, which upon deglycosylation became a more focused 35 kDa band. The largely transmembrane B subunit could not be visualized in these immunoprecipitation experiments (Fig. 6A), presumably because of its detachment from the antibody-bound A subunit and consequent loss during the very stringent washing procedure. The specificity of mAb for the TSHR was evident in control experiments using untransfected CHO cells and with a mAb to thyroid peroxidase (TPO)(Fig. 6B).

In contrast to the immunoprecipitation experiments, the B subunit was clearly visualized in immunohlots of TSHR-10,000 cells using B subunit-specific mAb T3-495 and T3-365 (21)(Figs. 7A, 7B). Thus, in addition to single subunit forms of the TSHR, a primary B subunit band of - 42 kDa was evident, as well as some minor bands of slightly greater size. Incubation with endoglycosidases F and H did not affect the mobility of the B subunit bands, consistent with their lack of N-linked glycosylation sites (Fig, 7A). Specificity of mAb T3-495 and T3-365 was confirmed on immunoblotting with untransfected CHO cells (Fig. 7B). As in the immunoprecipitation experiments, the size of the A subunit could clearly be determined by immunoblotting with mAb A10 to the TSHR amino terminus. The ~62 kDa mature A subunit contained complex carbohydrate (endoglycosidase H resistant)(Fig. 7C). Most important, deglycosylation with N-glycosidase F confirmed a ~35 kDa A subunit backbone. Lesser fragments of -39 kDa and -42 kDa were also evident. Again, the specificity of mAb A10 to the A subunit was confirmed on immunoblots with untransfected CHO cells and on immunohlotting of TSHR-10,000 cells with a mAb to TPO (Fig. 7D).

Conundrum of a missing piece, of the TSHR: The human TSHR, without its 21 amino acid residue signal peptide, has a predicted polypeptide backbone of 84.5 kDa (743 amino acid residues). However, from the immunoprecipitation and immunoblot studies shown above, the sum of the enzymatically deglycosylated A subunit (35 kDa) and the primary B subunit fragment (-42 kDa) was only -77 kDa. A 35 kDa polypeptide backbone for the TSHR A subunit would place the cleavage site in the region of amino acid residue 330, taking into account the absence of the signal peptide. Further, a -42 kDa size for the non-glycosylated B subunit would be consistent with a holoreceptor cleavage site at about residue 380. It, therefore, appeared that a "C peptide" fragment in the vicinity of residues 330 - 380 could be missing from the cleaved TSHR ectodomain (Fig. 8). This deduction is inconsistent with the prevailing concept of a single cleavage site in the TSHR ectodomain.

TSHR subunits in TSHRmyc cells: In order to explore the possibility of two cleavage sites in the TSHR, we used TSHRmyc cells that express a receptor with a 12 amino acid human c-myc epitope in place of residues 338-349. This epitope lies within the segment of the TSHR predicted to be missing if the two cleavage site hypothesis is correct, namely residues - 330-380 (Fig. 8). Detection by a mAb to c-myc of only the single subunit forms of the TSHR. and not the cleaved A subunit, would support the concept that a portion of the ectodomain is lost during intramolecular cleavage. Cleavage within the c-myc epitope could also lead to loss of this epitope. The two cleavage site hypothesis would also predict detection in TSHRmyc cells of the A subunit with may A 10 to the amino terminus of this subunit. or, even more definitively, by crosslinking with radiolabeled TSH.

The TSHRmyc cells do not contain an amplified transgenome and express fewer receptors (~100,000 per cell) (15) than TSHR-10,000 cells. Nevertheless, both anti-myc mAb 9E10 and mAb A10 were equally effective in detecting the single chain forms of the TSHR in these cells (Fig. 9A). It contrast, it was more difficult to detect the diffuse, glycosylated TSHR A subunit band in the TSHRmyc cells than in the TSHR-10,000 cells. However, after deglycosylation with N-glycosidase F, the 35 kDa A subunit in the TSHRmyc cells became more focused and was clearly visible on immunoprecipitation with mAb A10 to the TSHR amino terminus. In contrast, the A subunit was not detected in the same material with the anti-myc mAb 9E10. Because of the importance of this finding, a similar experiment is also shown in which the deglycosylated A subunit in TSHR-10,000 cells is included (Fig. 9B). The 35 kDa deglycosylated A subunit band detected by mAb A10 in the TSHRmyc and TSHR-10.000 cells was not an artifact because no such band was detected by immunoprecipitation with mAb 10 of precursor-labeled, untransfected HEK cells, nor did a non-relevant mAb (to TPO) detect this band in TSHRmyc cells (Fig. 9C).

Finally, we applied the most sensitive method (in our hands) to confirm the presence of the TSHR A subunit in TSHRmyc cells, namely the covalent cross-linking of radiolabeled TSH to the surface of intact cells in monolayer culture. By this means, the A subunit was clearly evident in TSHRmyc cells (Fig. 10). Further, the proportion between the A subunit and the uncleaved, single subunit receptor detected by TSH cross-linking was the same in the TSHRmyc cells as in a cell line expressing similar numbers of wild-type TSHR (TSHR-0)(16).

DISCUSSION

The present evidence for two cleavage sites in the TSHR ectodomain must be viewed in the context of previous data that (in retrospect) suppon this notion. Thus, for a number of years, we were puzzled by a phenomenon that we observed in TSH crosslinking studies with certain chimeric TSH-LHICG receptors. Neither substitution of TSHR residues 261-360 ("D domain"; chimeric receptor TSH-LHR-4) nor residues 363-418 ("E domain"; chimeric receptor TSH-LHR-5) with those of the related LHJCG receptor prevented TSHR cleavage into A and B subunits (10)(Fig. 11). Only the simultaneous substitution of domains D and E abrogated cleavage. These findings are consistent with the D and E domains each containing a cleavage site.

The smaller than expected sum of the sizes of the deglycosylated TSHR A subunit and the non-glycosylated B subunit support the concept of two cleavage sites in the TSHR, with the loss of an intervening portion of the polypeptide chain. While we recognize that size estimations cannot be absolutely precise, they are sufficiently reproducible among different laboratories, using different methodologies for TSHR detection, to suggest that a piece of the TSHR has been lost during intramolecular cleavage. We observed the deglycosylated A subunit and the B subunit to be -35 kDa and -42 kDa, respectively. Others have reported A and B subunits of similar sizes (21-23). In all studies, the sum of the two subunits is approximately 5 kDa smaller than the 84kDa holoreceptor. Our data also raise the possibility that cleavage at both sites is not an all or none phenomenon. Thus, detection of smaller amounts of A and B subunits of slightly greater size than the predominant subunit forms could represent a minor degree of incomplete cleavage at either site. Alternatively, these minor A subunit components could represent incomplete deglycosylation.

A third line of evidence suggesting two cleavage sites in the TSHR ectodomain is the present observation of the selective loss of a strategically situated c-myc epitope in the two subunit, but not in the single chain, form of the TSHR. There are three possible explanations for this phenomenon:- (i) loss of a peptide fragment containing the c-myc epitope during intramolecular cleavage at two different sites, (ii) cleavage at a single cleavage site within the c-myc epitope leading to loss of, antibody recognition, (iii) a combination of these two events (two cleavage sites, one of these within the c-myc epitope). Of these possibilities, cleavage at a single site within the c-myc epitope is unlikely, for a number of reasons. Thus:- (i) single cleavage within amino acid residues 338-349 (the c-myc epitope) would generate a B subunit of 46-47 kDa, clearly larger than the actual size (42 kDa) observed experimentally (see Figs. 7A and 7B); (ii) Chimeric receptor TSH-LHR-4 (24) and a deletion mutant (residues 317-3b6) of the wild-type TSHR (25) lack the region in which the c-myc epitope was inserted (Fig. 11), yet both receptors still cleave into two subunits (7,10); (iii) The c-myc epitope sequence differs substantially from that of the wild-type receptor. Persistent cleavage in such a highly mutated region would, therefore, indicate lack of amino acid sequence specificity for a TSHR cleavage site. For all these reasons, there are likely to be two cleavage sites in the TSHR ectodomain, the more upstream of which may, or may not, be with the region of the c-myc epitope.

The unsuspected possibility of two cleavage sites in the TSHR explains why we were previously misled into deducing that TSHR cleavage occurred closely upstream, rather than closely downstream, of amino acid residue 317 (7,10,12). We made this erroneous deduction because TSHR cleavage into A and B subunits was still evident on TSH cross-linking to a receptor with residues 317-366 deleted by mutagenesis (7,25), a finding that would exclude a single, but not two, cleavages sites in this region (Fig. 11). Moreover, the A subunit in this TSHR mutant was similar in size to that of the wild-type TSHR. In retrospect, cleavage a few residues upstream or downstream of pivotal residue 317 would be difficult to discern from a relatively large (-74 kDa) cross-linked product.

It is of interest that the c-myc epitope lies within a 50 amino acid segment (residues 317-366) that we observed to be unique to the TSHR when compared to other glycoprotein hormone receptors (3). Although the precise boundaries of this 50 amino acid "insertion" are uncertain (because of low homology among the receptors in adjacent regions), this TSHR segment has been the subject of intense study. The very hydrophilic nature of residues 317-355 led us to speculate that it was a projection on the exterior of the TSHR molecule, perhaps important in ligand specificity (25). Surprisingly, however, its deletion had no effect on TSH binding or on TSH-mediated signal transduction (25). The deduced superficial topography of TSHR residues 317-366 was also the reason for selection of this region for c-myc epitope tagging. Other investigators have used synthetic peptides corresponding to portions of this region for generating antisera to the TSHR. One peptide in particular (residues 352-367) is highly immunogenic and is reported to be recognized by TSHR autoantibodies in the majority of Graves' sera (26,27). Further, an antiserum to a closely related synthetic peptide (residues 352-366) recognize the A subunit in FRTL-5 rat thyroid cells, but very poorly in transfected COS cells (11). It will be interesting to correlate these findings with the possible absence of a part of this region in the cleaved TSHR.

In summary, multiple lines of evidence, taken together, suggest that there are two cleavage sites in the ectodomain of the TSHR. To our knowledge, theTSHR would he the first member of the family of G protein coupled receptors whose ectodomain appears to contain multiple cleavage sites. Purification and characterization of the putative TSHR C peptide would provide proof for the two cleavage site hypothesis. However, it is not presently feasible to detect the release of a small TSHR polypeptide fragment into the culture medium from the TSHRmyc cell line that is not overexpressing the TSHR. Nevertheless, the evidence for two cleavage sites in the TSHR ectodomain provides an impetus to future studies on the structure-function of the TSHR subunits.

REFERENCES CITED IN EXAMPLE 2

1 Buckland PR, Rickards CR, Howells RD, Jones ED, Rees Smith B 1982 Photo-affinity labelling of the thyrotropin receptor. FEBS Letters 145:245-249

2 Parmentier M, Libert F, Maenhaut C, Lefort A, Gerard C, Perret J, Van Sande J, Dumont JE, Vassart G 1989 Molecular cloning of the thyrotropin receptor. Science 246:1620-1622

3 Nagayama Y, Kaufman KD, Seto P, Rapoport B 1989 Molecular cloning, sequence and functional expression of the cDNA for the human thyrotropin receptor. Biochem.Biophys.Res.Comm. 165:1184-1190

4 Libert F, Lefort A. Gerard C, Parmentier M, Perret J, Ludgate M. Dumont JE, Vassart G 1989 Cloning, sequencing and expression of the human thyrouopin (TSH) receptor: Evidence for binding of autoantibodies. Biochem.Biophys.Res.Comm. 165:1250-1255

5 Misrahi M, Loosfelt H, Atger M, Sar S, Guiochon-Mantel A, Milgrom E 1990 Cloning, sequencing and expression of human TSH receptor. Biochem.Biophys.Res.Comm. 166:394-403

6 Furmaniak J, Hashirn FA, Buckland PR, Petersen VB, Beever K, Howells RD, Rees Smith B 1987 Photoaffinity labelling of the TSH receptor on FRTL5 cells. FEBS Letters 215:316-322

7 Russo D. Chazenbalk GD, Nagayama Y, Wadsworth HL. Seto P, Rapoport B 1991 A new structural model for the thyrotropin receptor as determined by covalent crosslinking of thyrotropin to the recombinant receptor in intact cells: Evidence for a single polypeptide chain. Mol.Endocrinol. 5:1607-1612

8 Van Sande J, Massart C, Costagliola S, Allgeier A, Cetani F, Vassart G, Dumont JE 1996 Specific activation of the thyrotropin receptor by trypsin. Mol.& Cellular Endo 119:161-168

9 Chazenbalk GD, McLachlan SM, Nagayama Y, Rapoport B 1996 Is receptor cleavage into two subunits necessary for thyrotropin action? Biochem.Biophys.Res.Comm. 225:479-484

10 Russo D, Nagayama Y, Chazenbalk GD, Wadsworth HL, Rapoport B 1992 Role of amino acids 261-418 in proteolytic cleavage of the extracellular region of the human thyrotropin receptor. Endocrinology 130:2135-2138

11 Ban T, Kosugi S, Kohn LD 1992 Specific antibody to the thyrotropin receptor identifies multiple receptor forms in membrane of cells transfected with wild-type receptor complementary deoxyribonucleic acid: Characterization of their relevance to receptor synthesis, processing, structure, and function. Endocrinology 131:815-829

12 Chazenbalk GD, Rapoport B 1994 Cleavage of the thyrotropin receptor does not occur at a classical subtilisin-related proprotein covertase endoproteolytic site. J Biol Chem 269:32209-32213

13 Couet J, Sakhavut S, Jolivet A, Vu Hai M-T, Milgrom E, Misrahi M 1996 Shedding of human thyrotropin receptor ectodomain: Involvement of a matrix metalloprotease. J Biol Chem 271:4545-4552

14 Chazenbalk GD, Kakinuma A, Jaume JC, McLachlan SM, Rapoport B 1996 Evidence for negative cooperativity among human thyrotropin receptors overexpressed in mammalian cells. Endocrinology 137:4586-4591

15 Tanaka K, Nagayama Y, Yamasaki H, Hayashi H, Samba H, Yamashita S, Niwa M 1996 Epitope-tagging of a functional thyrotropin receptor: detection of the native receptor on intact cells. Biochem.Biophys.Res.Comm. 228: 21-28

16 Kakinuma A, Chazenbalk G, Filetti S, McLachlan SM, Rapoport B 1996 Both the 5' and 3' non-coding regions of the thyrotropin receptor messenger RNA influence the level of receptor protein expression in transfected mammalian cells. Endocrinology 137:2664-2669

17 Nicholson LB, Vlase H, Graves P, Nilsson M, Molne J, Huang GC, Morgenthaler NG, Davies TF, McGregor AM, Banga JP 1996 Monoclonal antibodies to the human thyrotropin receptor: Epitope mapping and binding to the native receptor on the basolateral plasma membrane of thyroid follicular cells. J Mol Endocrinol 16:159-170

18 Laemmli UK 1970 Cleavage of structural proteins during the assembly of the head of bacteriophage T4. Nature 227:680-685

19 Rapoport B, McLachlan SM, Kakinuma A, Chazenbalk GD 1996 Critical relationship between autoantibody recognition and TSH receptor maturation as reflected in the acquisition of mature carbohydrate. J Clin Endocrinol Metab 81:2525-2533

20 Goldfine ID, Amir SM, Petersen AW. Ingbar SH 1974 Preparation of biologically active 125I-TSH. Endocrinology 95:1228-1233

21 Loosfelt H, Pichon C, Jolivet A, Misrahi M, Caillou B, Jamous M, Vannier B, Milgrom E 1992 Two-subunit structure of the human thyrotropin receptor. Proc Natl Acad Sci USA 895:3765-3769

22 Misrahi M. Ghinea N, Sar S, Saunier B. Jolivet A, Loosfelt H, Cerutti M, Devauchelle G, Milgrom E 1994 Processing of the precursors of the human thyroid-stimulating hormone receptor in various eukaryotic cells (human thyrocytes, transfected L cells and bacul ovirus- infected insect cells). Eur.J.Biochem. 222:711-719

23 Graves PN, Vlase H, Bobovnikova Y, Davies TF 1996 Multimeric complex formation by the thyrotropin receptor in solubilized thyroid membranes. Endocrinology 137:3915-3920

24 Nagayama Y, Wadsworth HL., Chazenbalk GD, Russo D. Seto P, Rapoport B 1991 Thyrotropin-luteinizing hormonelchorionic gonadotropin receptor extracellular domain chimeras as probes for TSH receptor function. Proc Natl Acad Sci USA 88:902-905

25 Wadsworth HL, Chazenbalk GD, Nagayama Y, Russo D, Rapoport B 1990 An insertion in the human thymtropin receptor critical for high affinity hormone binding. Science 249:1423-1425

26 Kosugi S, Akamizu T, Takai O, Prabhakar BS, Kohn LD 1991 The extracellular domain of the TSH receptor has an immunogenic epitope reactive with Graves' IgG but unrelated to receptor function as well as determinants having different roles for high affinity TSH binding and the activity of thyroid-stimulating autoantibodies. Thyroid 1:321-330

27 Kosugi S, Ban T, Akamizu T, Kohn LD 1991 Site-directed mutagenesis of a portion of the extracellular domain of the rat thyrotropin receptor important in autoimmune thyroid disease and nonhomologous with gonadotropin receptors. J Biol Chem 266:19413-19418

28 Kobe B, Deisenhofer J 1993 Crystal structure of porcine ribonuclease inhibitor, a protein with leucine-rich repeats. Nature 366:751-756

29 Kajava AV, Vassart G, Wodak SJ 1995 Modeling of the three-dimensional structure of proteins with the typical leucine-rich repeats. Structure 3:867-877

## EXAMPLE 3: EVIDENCE FOR NEGATIVE COOPERATIVITY AMONG HUMAN THYROTROPIN RECEPTORS OVEREXPRESSED IN MAMMALIAN CELLS

In this Example, high level expression of TSHR in CHO cells is described, as well as high constitutive activity of the TSHR in the absence of ligand, and the binding of TSH to the single subunit, uncleaved TSHR. Moreover, high level expression is associated with apparent negative co-operativity among the TSHR in terms of their affinity for ligand.

INTRODUCTION

The thyrotropin receptor (TSHR) is of pivotal importance in the regulation of thyroid gland physiological function and is also the direct cause of hyperthyroidism when targeted by autoantibodies in Graves' disease (reviewed in 1-3). The availability of purified TSHR would be a major advance in understanding the structure-function relationship of the TSHR and well as in exploring the pathogenesis of Graves' disease, one of the most common organ-specific autoimmune diseases affecting humans. Because of the very small number of TSHR in thyroid tissue (1), high level expression of recombinant TSHR has been a goal vigorously sought by numerous investigators over the past six years. Most effort has been applied to prokaryotic (4-9) and insect cell (8,10-14) systems in which high level expression can be achieved for many proteins. Although there are reports of TSH or TSHR autoantibody binding to such material (9,10,14,15), as well as to synthetic TSHR peptides (15-20) and cell-free translation products (21), other investigators have encountered serious difficulties in either protein production or ligand recognition (5,8,13,22).

Five years ago, because of our success in using dihydrofolate reductase (dhfr) minigene amplification to overexpress thyroid peroxidase in Chinese hamster ovary (CHO) cells (23), we had attempted the same approach with the TSH holoreceptor. However, we did not attain a high level of expression (Kaufman et al., unpublished data). Disillusionment in our subsequent sojourn with bacterial and baculovirus approaches led us to re-attempt overexpression, by dhfr-minigene amplification, of the TSHR ectodomain in CHO cells. However, most of this protein was retained within the cell in a form with immature carbohydrate which was not recognized by either TSH or functional TSHR autoantibodies (24).

More recently, we have come to realize that the 5'- and 3'-untranslated regions of the human TSHR have a major suppressive effect on TSHR expression in CHO cells (25). Because our initial attempt at TSH holoreceptor amplification by the dhfr-minigene approach had utilized a full-length (4 kb) TSHR cDNA (26) containing both untranslated regions, we repeated, once again, TSHR cDNA amplification using only the 2.3 kb translated region. We now report the high level of expression of TSHR in CHO cells and confirm the high constitutive activity of the TSHR in the absence of ligand (27,28). Moreover, we find that higher levels of expression are associated with apparent negative co-operativity among the TSHR in terms of their affinity for ligand.

METHODS

Construction of plasmid for high level expression of the human TSHR-ECD; A 2.7 kb TSHR cDNA containing the coding region and 0.4 kb of the 3' untranslated region was excised with Sal I and Xba I from pTSHR-5TR-NEO-ECE (25). This fragment was restricted with Hinc II to release the 2.3 kb TSHR coding region, which was then inserted into the Xba I (blunted) and Sal I sites in pSV2-DHFR-ECE-TPO (23) following removal of its Sal I-Xba I insert. The new plasmid, termed pSV2-DHFR-ECE-TSHR, was transfected by the calcium phosphate precipitation method (29) into dihydrofolate reductase (dhfr)-deficient CHO cells (CHO-DG44; kindly provided by Dr. Robert Schimke, Stanford University, Palo Alto, CA). Stably transfected cells were selected in thymidine-, guanine-, and hypoxanthine-free Ham's F-12 medium supplemented with 10% dialyzed fetal calf serum, penicillin (100 U/ml), gentamicin (50 $\mu$g/ml) and amphotericin B (2.5 $\mu$g/ml). After limiting dilution, 14 different clones were tested for TSHR expression by measurement of intracellular cAMP after TSH stimulation (see below). The clone with the greatest response was selected for transgenome amplification. Methotrexate (MTX) was added to the selective cell culture medium at an initial concentration of 20 nM and surviving cells were expanded. The methotrexate concentration was sequentially increased until a final concentration of 10,000 nM (10 $\mu$M) was reached. Cells were recloned by limiting dilution at 800 nM and 10,000 nM methotrexate.

Immunoprecipitation of precursor-labeled TSHR: CHO clonal cell lines stably expressing the TSHR were grown to confluence in 100 mm diameter culture dishes. After rinsing with phosphate-buffered saline (PBS), cells were pre-incubated (0.5 h, twice) in DME-H21 methionine- and cysteine-free medium containing 5% heat inactivated fetal calf serum. The cells were then pulsed (1 h at 37 C) in 5 ml fresh medium supplemented with - 0.5 mCi of $^{35}$S-methionine/cysteine (> 1000 Ci/mmole. DuPont NEN, Wilmington DE). After aspiration of the medium and rinsing the cells once with PBS, chase was performed for 8 h in standard, non-selective F12 medium with 10% fetal calf serum. Cells were washed twice with PBS and scraped into 1 ml ice-cold 20 mM Hepes, pH 7.2, 150 mM NaCl containing the protease inhibitors phenylmethylsulfonyl fluoride (PMSF) (100 $\mu$g/ml), leupeptin (1 $\mu$g/ml) aprotinin (1 $\mu$g/ml), and pepstatin A (2 $\mu$g/ml) (all from Sigma. St. Louis, MO)(buffer A). The cells were pelleted (5 min, 100 x g), washed twice with PBS and resuspended in buffer A containing 1% Triton X-100. After 90 min at 4C with occasional vortexing, the mixture was centrifuged for 45 min at 100,000 x g and the supernatant was diluted 1:4 in immunoprecipitation buffer (20 mM Hepes pH 7.2, 300 mM NaCl, 0.1% sodium dodecyl sulfate, 0.5% Nonidet-P40 and 2 mM EDTA.). The solubilized cell proteins were precleared by incubation for 1 h at 4C with 150 $\mu$g normal mouse serum IgG prebound to 25 $\mu$l packed and washed protein A-agarose (Sigma). After removal of the protein A by centrifugation (3 min at 10,000 X g) in a microcentrifuge, mouse monoclonal antibodies to the TSHR (mixture of A9 and A 10, a kind gift of Dr Paul Banga, London. U.K., final dilution of 1:1000) were added. A9 and A10 recognize TSHR regions between amino acids 147-229 and 22-35, respectively (30). After 3 h at 4C, 25 $\mu$l packed and washed protein A-agarose was added and the tubes were tumbled for 1 h at 4C. The protein A was recovered by centrifugation for 3 min at 10,000 X g (4 C), washed 5 times with 1 ml immunoprecipitation buffer, and then once with 10 mM Tris, pH 7.4, 2 mM EDTA and 0.5% sodium dodecyl sulfate (SDS). Finally the pellet was resuspended in Laemmli sample buffer (31) with 0.7 M ß-mercaptoethanol (30 min at 42 C) and electrophoresed on 7.5% SDS-polyacrylamide gels (BioRad, Hercules. CA). Prestained molecular weight markers (BioRad) were included in parallel lanes. Radiolabeled proteins were visualized by autoradiography on Kodak XAR-5 X-ray film (Eastman Kodak, Rochester, NY). Densitometric analysis was performed with a BioRad scanning densitometer.

Covalent cross-linking of radiolabeled TSH: Highly purified bovine TSH (5 pg, 30 U/mg protein) was radiolabeled with $^{125}$I to a specific activity of - 80 μCi/μg protein using the Bolton-Hunter reagent (4400 Ci/mmol; DuPont-NEN) according to the protocol of the manufacturer, followed by Sephadex G-100 chromatography (32). CHO clonal cell lines stably expressing the TSH receptor at different levels of amplification with methotrexate, were grown to confluence in 100 mm diameter culture dishes. Cells were incubated for 2h at 37 C with 5 μCi $^{125}$I-TSH in 5 ml modified Hank's buffer (280 mM sucrose substituting for NaCl to maintain isotonicity) supplemented with 0.25% BSA (binding buffer). Unbound $^{125}$I-TSH was removed by rinsing the cells three times with ice-cold binding buffer. Disuccinimidyl suberate (DSS; 1 mM; Sigma) in 10 mM Na phosphate buffer, pH 7.4. containing the protease inhibitors described above was then added for 20 min at room temperature. The cross-linking reaction was terminated by the addition of 20 mM ammonium acetate (final concentration).

After cross-linking, the cells were rinsed twice with PBS and scraped into 10 mM Tris, pH 7.5, containing the same protease inhibitors. Cells were homogenized using a Polytron homogenizer (Brinkmann Instruments, Westbury CT) and centrifuged for 5 min at 4 C (500 x g). The supernatant was centrifuged (15 min, 10,000 x g, 4C) and the pellet was resuspended in 50 μl 10 mM Tris, pH 7.5. Protein concentrations were determined by the method of Bradford (33). After the addition of Laemmli buffer (31), in the presence of 0.7 M ß-mercaptoethanol (30 min at 42 C), the samples were subjected to 7.5% SDS-PAGE gels and analyzed as described before.

TSH binding: CHO cells stably transfected with TSHR cDNA were grown to confluence in 96-well culture plates. Cells were then incubated for 2 h at 37°C in 50 μl binding buffer (see above) containing approximately 50.000 cpm of $^{125}$I-TSH in the presence or absence of increasing concentrations of unlabeled bovine TSH (Sigma). At the end of the incubation period, the cells were rapidly rinsed three times with binding buffer (4°C), solubilized with 0.1 ml NaOH and radioactivity measured in a gamma-counter. Experiments using increasing concentrations of $^{125}$I-TSH were per-formed in cells cultured under the same conditions except that no unlabeled TSH was added. Non-specific $^{125}$I-binding to untransfected CHO cells was subtracted from total counts bound to provide specific counts bound. These values were < 10% of total counts bound at the highest tracer concentration used (2.5 x 10$^7$ cpm/ml).

TSH stimulation of intracellular cAMP: Transfected CHO cells, grown to confluence in 24-well or 96-well culture plates, were incubated for 2 h at 37°C in either hypotonic medium (34,35) or in Ham's F-12 medium containing 1% bovine serum albumin, 1 mM isobutyl methylxanthine, with or without added bovine TSH (Sigma). Cyclic AMP was measured directly in the hypotonic medium. For cells exposed to TSH in isotonic Ham's F12 medium, the medium was aspirated, intracellular cAMP was extracted with 95% ethanol, evaporated to dryness and resuspended in 50 mM Na acetate, pH 6.2. Cyclic AMP was measured by radioimmunoassay using cAMP, 2'-O-succinyl $^{125}$I-iodotyrosine methyl ester (Dupont NEN) and a rabbit anti-cAMP antibody from Calbiochem, San Diego CA.

RESULTS

Quantitation by immunoprecipitation of TSHR protein expression in whole CHO cells: The process of stable transfection and progressive amplification of the TSHR holoreceptor/dhfr-minigene complex in the genome of CHO cells took approximately one year. During this period. TSHR expression in the selected clone was confirmed intermittently by $^{125}$I-TSH binding. After completion of the amplification process, we assessed the level of TSHR expression within whole cells by precursor $^{35}$S-methionine and $^{35}$S-cysteine labeling followed by immunoprecipitation under native conditions using mouse monoclonal antibodies to the TSHR (A9 + A10)(30). In this comparison (Fig. 12), we used clonal cell lines stably transfected with (i) 4 kb TSHR cDNA containing both 5'- and 3'-untranslated regions (wild-type TSHR) (26), (ii) 2.3 kb TSHR cDNA with deleted untranslated regions (5'3TR-ECE)(25), renamed TSHR-0 for this report), (iii) 2.3 kb TSHR cDNA after growth stabilization in 800 nM methotrexate (TSHR-800) and (iv) 2.3 kb TSHR cDNA after growth stabilization in 10,000 nM methotrexate (TSHR-10,000). Untransfected CHO cells were included as a negative control.

Analysis of the immunoprecipitation products under reducing conditions revealed multiple specific hands, consistent with the pattern of TSHR holoreceptors, subunits and precursors previously reported by Misrahi et al. (12) in whole cell homogenates. Of importance from the perspective of the present study was the clear increase in expression of all TSHR-specific bands in association with the dhfr amplification process. For the present study, densitometric quantitation of the 100 kDa holoreceptor band (Fig. 12, shown by the arrow), indicated that TSHR expression in TSHR-10,000 cells was 10.3 ± 2.0 (range in two separate experiments) greater than in TSHR-0 cells.

Crosslinking of TSH to TSHR on the cell surface: In stably-transfected mouse L-cells, unlike in thyroid cells, it is reported that most TSHR do not undergo normal maturation (12). Instead, the immature form of the receptor accumulates within the cell and is degraded. It was, therefore, important to determine whether the TSHR amplification observed by immunoprecipitation of cell lysates was also associated with an increase in mature TSHR expression on the cell surface. Radiolabeled TSH crosslinking to intact CHO cells in monolayer culture revealed a progressive increase in TSH binding sites with dhfr-minigene amplification (Fig. 13). By densitometric analysis, TSHR binding to TSHR-10,000 cells increased $12.8 \pm 2.8$-fold (mean $\pm$ S.E.; n = 3 experiments) over that to TSHR-0 cells. Based on the previous determination of -150,000 TSHR molecules on the surface of TSHR-0 (formerly 5'3'TR-ECE) cells (25), the present data suggest that TSHR-10,000 cells express ~$1.9 \times 10^6$ TSHR on their surface.

Two additional features regarding these cross-linking data should be pointed out. First, the autoradiogram exposure is relatively short (16 hours), at which time a signal cannot be seen with the wild-type TSHR cDNA expressed in CHO cells. Second, two ligand-TSHR complexes (~130 kDa and -75 kDa) are evident under reducing conditions. After subtraction of the molecular mass of a TSH subunit, the upper band represents a single chain holoreceptor (36,37) with an apparent mass of - 115 kDa and the lower band a dissociated A subunit (38) of - 60 kDa.

<u>Function of the overexpressed TSHR in the absence of ligand</u>: Basal cAMP levels were assessed in stably-transfected cell lines expressing different numbers of TSHR. In cells cultured in hypotonic medium (34,35) without TSH, cAMP levels were 18.5-fold higher in the TSHR-10,000 cells ($20.3 \pm 1.5$ S.E. pmoles/well, n=3) relative to the TSHR-0 cells ($1.10 \pm 0.21$ pmoles/well)(Fig. 14A). In isotonic medium (Fig. 14B), basal cAMP levels were increased to a lesser extent (6.0-fold) in TSHR-10,000 cells ($3.04 \pm 0.36$ S.E. pmoles/well, n=3) relative to the TSHR-0 cells ($0.52 \pm 0.05$ pmoles/well). These data indicate that the unliganded TSHR has significant constitutive activity. Incidentally, under hypotonic conditions and in association with the higher basal cAMP levels in the cell lines with more TSHR, the increase in cAMP levels in response to TSH stimulation was reduced from 17-fold in the TSHR-0 cells to 2.9-fold in the TSHR-800 cells and 1.4-fold in the TSHR-10,000 cells)(Fig. 14A). Similar, but less dramatic decreases in the cAMP response to the same TSH concentration were observed when cells were cultured in isotonic medium. <u>Kinetics of TSH binding to the surface of cells overexpressing the TSHR</u>: In initial experiments, we determined the kinetics of TSH binding to CHO cells overexpressing the TSHR using $^{125}$I-TSH in the presence of increasing concentrations of unlabeled TSH. Surprisingly, in contrast to TSHR-0 cells (Kd ~ $5 \times 10^{-10}$ M)(25), we observed progressively lower half-maximal inhibition values for TSH binding by TSHR-800 cells - $10^{-9}$ M) and TSHR-10,000 cells ($\sim 2 \times 10^{-9}$ M)(Fig. 154). All of these values are lower than those observed for cells transfected with the wild-type, full-length TSHR cDNA (TSHR-WT)($2 \times 10^{-10}$ M). Scatchard analysis of TSH affinity and, in particular, TSHR number could not be performed with accuracy in the TSHR-800 and TSHR-10,000 cells because their lower affinity of specific TSH binding approached that of non-specific TSH binding to untransfected CHO cells (39) and even plastic (40) (Kd ~ $6 \times 10^{-8}$ M). Indeed, it must be emphasized that in order to attain partial receptor saturation with ligand for the studies shown in Fig. 15, it was necessary to reduce the number of TSHR in each culture well. Therefore, the TSHR-800 and TSHR-10.000 cells were diluted 1:50 with untransfected cells.

In order to reduce the error introduced by subtraction of a non-specific binding component whose affinity is only approximately one order of magnitude lower than specific binding, we attempted to determine the TSH binding kinetics to TSHR-800 and TSHR-10,000 cells overexpressing the TSHR by saturation analysis using increasing amounts of $^{125}$I-TSH in the absence of unlabeled TSH. Scatchard analysis of these data was non-revealing because insufficient TSHR saturation could be attained even when adding tracer as high as $1.25 \times 10^6$ cpm/well (50 ul medium)(Fig. 16). Nevertheless, it is clear that specific TSH binding to the surface of TSHR-10,000 cells is approximately 10-fold higher than to TSHR-0 cells, consistent with the $^{125}$I-TSH cross-linking data (Fig. 13).

<u>DISCUSSION</u>

After many years of effort, we have finally achieved high level of expression of the human TSHR in mammalian cells. The extent of TSHR overexpression on the surface of TSHR-10,000 cells (~$1.9 \times 10^6$ receptors per cell, is quite remarkable considering the estimate of only $\sim 5 \times 10^3$ TSHR on thyrocytes (1). This level of expression, 10-12-fold higher than previously attained in stably-transfected mammalian cells (22,41,42), is of value because it will facilitate studies on the native TSHR. Although there are reports of TSH and TSHR autoantibody interaction with recombinant TSHR produced in bacterial (9,14), insect cell (10,14,15) and cell-free translation (21) expression systems, or as peptides (15-20), our preference is to focus on the native protein as produced in mammalian cells. TSHR generated in bacteria or insect cells is typically insoluble and requires solubilization in chaotropic agents followed by renaturation (9-11,14).

Our ultimate goal in expressing the TSHR at high levels in mammalian cells is to determine the three-dimensional structure of the TSHR. Even with overexpression, however, it remains uncertain whether or not crystals of purified TSHR will ever be attained in view of the daunting problems of the holoreceptor being a membrane-associated protein with a very large carbohydrate component. Crystallization of the purified ectodomain would be more feasible. However, in our experience, the ectodomain by itself does not mature correctly in mammalian cells and cannot be overexpressed (24). In the meanwhile, however, the holoreceptor expressed by TSHR-10.000 cells will facilitate study of subunit structure, carbohydrate composition, interaction with its physiological ligand, TSH, as well as intracellular trafficking. It should be appreciated that, until the present, and even with the availability of mouse mAb (5,30,43), the recombinant TSHR expressed in mammalian cells has required prior affinity purification for efficient immunological detection (12).

The TSHR-10,000 cells are also of potential value in providing antigen for the study of TSHR autoantibodies. Among these uses are the development of new assays for TSHR autoantibodies, flow cytometric analysis of TSHR autoantibodies in patients' sera and the isolation of human monoclonal autoantibodies from immunoglobulin gene combinatorial libraries derived from patients' B cells. However, the high basal cAMP levels in these cells reduces their sensitivity and makes them suboptimal for the bioassay of TSHR functional activity.

Besides the practical aspects of high level TSHR expression in mammalian cells, the application of which will require long-term development, the present study of the TSHR-10,000 cells provides insight into the function of the TSHR, at least as expressed in CHO cells. First, the data on stably-transfected cell lines strongly support transient transfection data (28) that the TSHR maintains a moderate level of activity in the absence of ligand. Such spontaneous activity was first observed with the $\alpha$ 1B-adrenergic receptor (44). The potential importance of this activity in the case of the thyroid gland, is that, as suggested by us previously (25), thyroid overactivity could result from an increased number of TSHR expressed on the thyrocytes of a particular individual consequent to mutations in the mRNA untranslated region in the same way as mutations in the mRNA coding region can increase the increased constitutive activity of an unchanged number of receptors (45,46).

A second interesting feature of the TSHR overexpressed in the TSHR-10,000 cells is the ability of TSH to bind to single chain TSHR expressed on the cell surface. The proportion of single chain and two-subunit TSHR on the surface of CHO cells is independent of the number of receptors expressed. However, the stronger signal with the TSHR-10,000 cells makes the phenomenon much more clear and supports our previous evidence using chimeric TSH-LH/CG receptors that cleavage into A and B subunits is not a requirement for TSH binding (37). This evidence raises the possibility that the single chain TSHR is, at least in part, a physiological receptor, a concept disputed by other investigators (5).

The most surprising feature of the CHO cells overexpressing the TSHR was the progressive reduction in affinity for TSH observed with increasing numbers of TSHR. In retrospect, we observed but did not appreciate, a similar but less dramatic decrease in TSH affinity in studies examining the role of the 5'- 3'-untranslated regions of the TSHR (25). We considered it artifactual that the affinity of the wild-type TSHR cDNA appeared to decrease from 2 x $10^{-10}$ M to 5 x $10^{-10}$ M Kd when TSHR expression increased after deletion of the untranslated regions. However, the further decreases in TSH affinity with the TSHR-800 and TSHR-10,000 cells make this conclusion inescapable. The TSH affinities in these cell lines cannot he estimated accurately because they are only about one order of magnitude higher than that for non-specific TSH binding. Prior to the molecular cloning of the TSHR, this remarkably high affinity of non-specific binding, observed also to plastic (40), was considered to be a second, lower affinity type of TSHR (17,47). Nevertheless, on the basis of the TSH concentrations causing half-maximal displacement, the affinities for TSH of the TSHR-10,000 and TSHR,-800 cells are clearly lower than for the TSHR-0 cells.

The most likely explanation for the phenomenon of decreasing affinity with receptor number is, in our opinion, negative cooperativity between TSHR. It is possible that the TSHR aggregate or "patch" in the fluid plasma membrane, especially at high density. Conformational changes in the TSHR itself or steric hindrance to TSH binding could also lead to an apparent reduction in affinity, as could adducts between holoreceptors and TSHR B subunits. Excess B subunits have been observed previously in cells expressing the TSHR (5,48). The lower affinity TSHR affinity could also, perhaps, occur if a greater proportion of TSHR were coupled to G proteins. Further studies are required to explore which, if any, of these alternatives is the underlying mechanism. We recognize that the functional changes observed in the TSHR expressed at high density will require caution in extrapolating structural information on these receptors to the TSHR present in thyrocytes. Nevertheless, it is feasible that TSHR negative cooperativity could represent another type of autoregulation, so typical of the thyroid, associated with maintaining stable thyroid function.

REFERENCE FOR EXAMPLE 3

1 Rees Smith B. McLachlan SM, Furmaniak J 1988 Autoantibodies to the thyrotropin receptor. Endocr Rev 9:106-121

2 Nagayama Y, Rapoport B 1992 The thyrotropin receptor twenty five years after its discovery: new insights following its molecular cloning. Mol.Endocrinol. 6:145-156

3 Vassart G. Dumont JE 1992 The thyrotropin receptor and the regulation of thyrocyte function and growth. Endocr Rev 13:596-611

4 Takai O, Desai RK, Seetharamaiah GS, Jones CA, Allaway GP, Akamizu T, Kohn LD, Prabhakar BS 1991 Prokaryotic expression of the thyrotropin receptor and identification of an immunogenic region of the protein using synthetic peptides. Biochem.Biophys.Res.Comm. 179:319-326

5 Loosfelt H, Pichon C, Jolivet A, Misrahi M, Caillou B, Jamous M, Vannier B, Milgrom E 1992 Two-subunit structure of the human thyrotropin receptor. Proc Natl Acad Sci USA 895:3765-3769

6 Harfst E, Johnstone AP, Nussey SS 1992 Characterization of the extracellular region of the human thyrotropin receptor expressed as a recombinant protein. J Mol Endocrinol 9:227-236

7 Huang GC, Collison KS, McGregor AM, Banga JP 1992 Expression of a human thyrotropin receptor fragment in Escherichia coli and its interaction with the hormone and autoantibodies from patients with Graves' disease. J Mol Endocrinol 8:137-144

8 Costagliola S. Alcalde L. Ruf J, Vassart G. Ludgate M 1994 Overexpression of the extracellular domain of the thyrotrophin receptor in bacteria; production of thyrotrophin-binding inhibiting immunoglobulins. J Mol Endocrinol 13:11-21

9 Graves PN, Vlase H, Davies TF 1995 Folding of the recombinant human thyrotropin (TSH) receptor extracellular domain: identification of folded monomeric and tetrameric complexes that bind TSH receptor autoantibodies. Endocrinology 136:521-527

10 Huang GC, Page MJ, Nicholson LB, Collison KS, McGregor AM, Banga JP 1993 The thyrotropin hormone receptor of Graves' disease: overexpression of the extracellular domain in insect cells using recombinant baculovirus, immunoaffinity purification and analysis of autoantibody binding. J Mol Endocrinol 10:127-142

11 Seetharamaiah GS, Desai RK, Dallas JS, Tahara K, Kohn LD, Prabhakar BS 1993 Induction of TSH binding inhibitory immunoglobulins with the extracellular domain of human thyrotropin receptor produced using baculovirus expression system. Autoimmunity 14:315-320

12 Misrahi M, Ghinea N, Sar S, Saunier B, Jolivet A, Loosfelt H, Cerutti M, Devauchelle G, Milgrom E 1994 Processing of the precursors of the human thyroid-stimulating hormone receptor in various eukaryotic cells (human thyrocytes, transfected L cells and baculovirus-infected insect cells). Eur.J.Biochem. 222:711-719

13 Chazenbalk GD, Rapoport B 1995 Expression of the extracellular region of the thyrotropin receptor in a baculovirus vector using a promoter active earlier than the polyhedrin promoter: Implications for the expression of functional, highly glycosylated proteins. J Biol Chem 270:1543-1549

14 Vlase H, Graves P, Magnusson RP, Davies TF 1995 Human autoantibodies to the thyrotropin receptor: recognition of linear, folded, and glycosylated recombinant extracellular domain. J Clin Endocrinol Metab 80:46-53

15 Fan J-L, Seetharamaiah GS, Desai RK, Dallas JS, Wagle NM, Prabhakar BS 1993 Analysis of autoantibody reactivity in patients with Graves' disease using recombinant extracellular domain of the human thyrotropin receptor and synthetic peptides. Autoimmunity 15:285-291

16 Morris JC, Jiang N-S, Hay ID, Charlesworth MC, McCormick DJ, Ryan RJ 1988 The effects of synthetic alpha-subunit peptides on thyroid-stimulating immunoglobulin activity. J Clin Endocrinol Metab 67:707-712

17 Atassi MZ. Manshouri T, Sakata S 1991 Localization and synthesis of the hormone-binding regions of the human thyrotropin receptor. Proc Natl Acad Sci USA 88:3613-3617

18 Morris JC, Bergert ER, McCormick DJ 1993 Structure-function studies of the human thyrotropin receptor. Inhibition of binding of labeled thyrotropin (TSH) by synthetic human TSH receptor peptides. J Biol Chem 268:10900-10905

19 Bryant WP. Bergert ER, Morris JC 1994 Delineation of amino acid residues within hTSHr 256-275 that participate in hormone binding. J.Biol Chem 269:30935-30938

20 Morris JC, Gibson JL, Haas EJ, Bergert ER, Dallas JS, Prabhakar BS 1994 Identification of epitopes and affinity purification of thyroid stimulating auto-antibodies using synthetic human TSH receptor peptides. Autoimmunity 17: 287-299

21 Morgenthaler NG, Tremble J. Huang G, Scherbaum WA, McGregor AM, Banga JP 1996 Binding of antithyrotropin receptor autoantibodies in Graves' disease serum to nascent, in vitro translated thyrotropin receptor: Ability to map epitopes recognized by antibodies. J Clin Endocrinol Metab 81:700-706

22 Harfst E, Johnstone AP, Gout I, Taylor AH, Waterfield MD, Nussey SS 1992 The use of the amplifiable high-expression vector pEE14 to study the interactions of autoantibodies with recombinant human thyrotropin receptor. Molec.Cell.Endocrinol. 83:117-123

23 Kaufman KD, Foti D, Seto P, Rapoport B 1991 Overexpression of an immunologically-intact, secreted form of human thyroid peroxidase in eukaryotic cells. Molec.Cell-Endocrinol. 78:107-114

24 Rapoport B. McLachlan SM, Kakinuma A, Chazenbalk GD 1996 Critical relationship between autoantibody recognition and TSH receptor maturation as reflected in the acquisition of mature carbohydrate. J Clin Endocrinol Metab (In Press)

25 Kakinuma A, Chazenbalk G, Filetti S. Mci.achlan S, Rapoport B 1995 Both the 5' and 3' non-coding regions of the thyrotropin receptor messenger RNA influence the level of receptor protein expression in transfected mammalian cells. Endocrinology (In Press)

26 Nagayama Y, Kaufman KD, Seto P. Rapoport B 1989 Molecular cloning, sequence and functional expression of the cDNA for the human thyrotropin receptor. BioChem.Biophys.Res.Comm. 165:1 i84-1190

27 Parma J, Duprez L, Van Sande J, Cochaux P, Gervy C, Mockel J, Dumont J, Vassart G 1993 Somatic mutations in the thyrotropin receptor gene cause hyperfunctioning thyroid adenomas. Nature 365:649-651

28 Van Sande J. Swillens S, Gerard C, Allgeier A, Massart C, Vassart G, Dumont J 1995 In Chinese hamster ovary KI cells dog and human thyrotropin receptors activate both the cyclic AMP and the phosphatidylinositol 4,5-bisphosphate cascades in the presence of thyrotropin and the cyclic AMP cascade in its absence. Eur J.Biochem 229:338-343

29 Chen C. Okayama H 1987 High-efficiency transformation of mammalian cells by plasmid DNA. Mol.Cell.Biol. 7: 2745-2752

30 Nicholson LB, Vlase H, Graves P. Nilsson M, Molne J, Huang GC, Morgenthaler NG, Davies TF, McGregor AM, Banga JP 1996 Monoclonal antibodies to the human thyrotropin receptor: Epitope mapping and binding to the native receptor on the basolateral plasma membrane of thyroid follicular cells. J Mol Endocrinol 16:159-170

31 Laemmli UK 1970 Cleavage of structural proteins during the assembly of the head of bacteriophage T4. Nature 227:680-685

32 Goldfine ID, Amir SM, Petersen AW, Ingbar SH 1974 Preparation of biologically active 125I-TSH. Endocrinology 95:1228-1233

33 Bradford MM 1976 A rapid and sensitive method for the quantitation of microgram quantities of protein utilizing the principle of protein-dye binding. Anal.Biochem. 72:248-254

34 Kasagi K, Konishi J, Iida Y, Ikekubo K, Mori T, Kuma K, Torizuka K 1982 A new in vitro assay for human thyroid stimulator using cultured thyroid cells: Effect of sodium chloride on adenosine 3',5'-monophosphate increase. J Clin Endocrinol Metab 54:108-114

35 Rapoport B, Filetti S, Takai N, Seto P, Halverson G 1982 Studies on the cyclic AMP-response to thyroid stimulating immunoglobulin (TSI) and thyrotropin (TSH) in human thyroid cell monolayers. Metabolism 31:1159-1167

36 Russo D, Chazenbalk GD, Nagayama Y, Wadsworth HL, Seto P, Rapoport B 1991 A new structural model for the thyrotropin receptor as determined by covalent crosslinking of thyrotropin to the recombinant receptor in intact cells: Evidence for a single polypeptide chain. Mol.Endocrinol. 5:1507-1612

37 Russo D, Nagayama Y, Chazenbalk GD, Wadsworth HL, Rapoport B 1992 Role of amino acids 261-418 in proteolytic cleavage of the extracellular region of the human thyrotropin receptor. Endocrinology 130:2135-2138

38 Buckland PR, Rickards CR, Howells RD, Jones ED, Rees Smith B 1982 Photo-affinity labelling of the thyrotropin receptor. FEBS Letters 145:245-249

39 Chazenbalk GD, Nagayama Y, Kaufman KD, Rapoport B 1990 The functional expression of recombinant human thyrotropin receptors in non-thyroidal eukaryotic cells provides evidence that homologous desensitization to thyrotropin stimulation requires a cell-specific factor. Endocrinology 127:1240-1244

40 Yamamoto M, Rapoport B 1978 Studies on the binding of radiolabeled thyrotropin to cultured human thyroid cells. Endocrinology 103:2011-2019

41 Costagliola S, Swillens S, Niccoli P, Dumont JE, Vassart G, Ludgate M 1992 Binding assay for thyrotropin receptor autoantibodies using the recombinant receptor protein. J Clin Endocrinol Metab 75:1540-1544

42 Matsuba T, Yamada M, Suzuki H, Kanai A, Isozaki O, Yoshida T, Tsushima T, Yasukawa K 1995 Expression of recombinant human thyrotropin receptor in myeloma cells. J.Biochem. 118:265-270

43 Seetharamaiah GS, Wagle NM, Morris JC, Prabhakar BS 1995 Generation and characterization of monoclonal antibodies to the human thyrotropin (TSH) receptor: antibodies can bind to discrete conformational or linear epitopes and block TSH binding. Endocrinology 136:2817-2824

44 Kjelsberg MA, Catecchia S, Ostrowski J, Caron MG, Lefkowitz RJ 1992 Constitutive activation of the alpha IB-adrenergic receptor by all amino acid substitutions at a single site. Evidence for a region which constrains receptor activation. J Biol Chem 267:1430-1433

45 Duprez L, Parma J, Van Sande J, Allgeier A, Leclere J, Schvartz C, Delisle M-J, Decoulx M, Orgiazzi J, Dumont J, et al 1994 Gcrmline mutations in the thyrotropin receptor gene cause non-autoimmune autosomal dominant hyperthyroidism. Nature Genet. 7:396-401

46 Parma J, Van Sande J, Swillens S, Tonacchera M, Dumont J, Vassart G 1995 Somatic mutations causing constitutive activity of the thyrotropin receptor are the major cause of hyperfunctioning thyrod adenomas: Identification of additional mutations activating both the cyclic adenosine 3',5'-monophosphate and inositol phosphate-Ca2+ cascades. Mol.Endocrinol. 9:725-733

47 Kohn, L.D., Aloj, S.M., Tombaccini, D., et al. The thyrotropin receptor. In: Biochemical actions of hormones, Vol. XII, Anonymous Academic Press, Inc. 1985, p. 457-512.

48 Couet J, Sokhavut S, Jolivet A, Vu Hai M-T, Milgrom E, Misrahi M 1996 Shedding of human thyrotropin receptor ectodomain: Involvement of a matrix metalloprotease, J Biol Chem 271:4545-4552

49 Rapoport B 1976 Dog thyroid cells in monolayer tissue culture: Adenosine 3',5'-cyclic monophosphate response to thyrotropic hormone. Endocrinology 98:1189-1197

50 Wadsworth HL, Chazenbalk GD, Nagayama Y, Russo D, Rapoport B 1990 An insertion in the human thyrotropin receptor critical for high affinity hormone binding. Science 249:1423-1425

**EXAMPLE 4: ENGINEERING THE HUMAN THYROTROPIN RECEPTOR ECTODOMAIN FROM A NON-SECRETED FORM TO A SECRETED, HIGHLY IMMUNOREACTIVE GLYCOPROTEIN THAT NEUTRALIZES AUTOANTIBODIES IN GRAVES' PATIENTS' SERA**

In this Example, it is demonstrated that carboxyl-terminal truncation of the human TSHR ectodomain generates a secreted protein with complex carbohydrate that neutralizes autoantibodies in Graves' patients' sera. Antigenically active TSHR is useful for the diagnosis, pathogenesis and immunotherapy of Graves' disease.

INTRODUCTION

Graves' disease is a very common (- 1% prevalence)(1), organ-specific autoimmune disease, affecting only humans. Unlike in diabetes mellitus, type I, a less common organ-specific disease, there is no spontaneous animal model for Graves' disease. Also in contrast to diabetes mellitus, type 1, one specific antigen is unequivocally and directly involved in the pathogenesis of Graves' disease, namely the thyrotropin receptor (TSHR). Thus autoantibodies to the TSHR activate the receptor, leading to thyroid overactivity and thyrotoxicosis (reviewed in 2). The interaction between autoantibodies and the TSHR is, therefore, of interest from the theoretical, diagnostic and (potentially) therapeutic points of view.

Because of the importance of the TSHR as an autoantigen, a large effort has been made in the seven years since the molecular cloning of its cDNA (3-5) to generate this protein in various expression systems, including bacteria (6-11), insect cells (12-16), stably-transfected mammalian cells (3,17-21) and cell-free translation (22), as well as by peptide synthesis (23,24). However, the generation of effective TSHR antigen has been extraordinarily difficult. Thus, despite this major effort and although some of these approaches have appeared promising, it is remarkable that a direct assay for TSHR autoantibodies using recombinant TSHR antigen has not supplanted the indirect, TSH binding-inhibition assay with porcine thyroid extracts (25) in use for nearly two decades. Moreover, lack of effective antigen has hampered mechanistic and structural studies of autoantibody-TSHR autoantigen interactions.

An important factor contributing to this difficulty is that TSHR autoantibodies, like TSH, predominantly recognize discontinuous, highly conformational epitopes (26-28). TSHR expressed on the surface of mammalian cells are conformationally intact and are unquestionably recognized by autoantibodies in patients' sera (29-31). Moreover, large numbers of TSHR-expressing mammalian cells can be produced in fermentors (20) and TSHR overexpression in Chinese hamster ovary (CHO cells) has been achieved by transgenome amplification (32). However, the seven membrane-spanning segments of the TSHR do not facilitate purification. Contrary to expectations, when the 418 amino acid residue, autoantibody-binding TSHR ectodomain is expressed in CHO cells without its serpentine region, it is not secreted but is retained within the cell (33,34) largely in a form containing high mannose carbohydrate (34). Moreover, this TSHR ectodomain with immature carbohydrate is not recognized by autoantibodies in patients' sera (34).

We now report that, in contrast to the entire TSHR ectodomain, progressive carboxyl-terminal truncations lead to the secretion by CHO cells of a modified TSHR ectodomain with mature, complex carbohydrate. Further, by epitope-tagging this autoantigen and by amplifying its transgenome in CHO cells, we report the first direct visualization and quantification of TSHR of mammalian cell origin. Most important, this material can neutralize all or most TSHR binding activity in patients' sera. Antigenically active TSHR will provide a major impetus for future studies on the pathogenesis of Graves' disease.

METHODS

Plasmid constructs: We generated three plasmids for expression in mammalian cells of limited TSHR ectodomain truncations (Fig. 17):- (i) TSHR-261: Plasmid TSHR-5'TR-NEO-ECE (35) contains an Afl II site at codon 260 and an Xba I site in the vector at the 3 end of the insert. The Afl II-Xba I fragment was excised and replaced with a cassette coding for 6 histidine residues (6H) followed by 2 stop codons. The cassette was created by annealing two oligonucleotides:- Sense: 5'-TTAACCATCACCACCACCATCACTGATAAT; Antisense: 5'-CTAGATTATCAGTGATGGTGGTGGTGATGG Ligation at the Afl II site generated an Asn residue upstream of the 6H, hence the nomenclature "261 ". (ii) TSHR-289: A cDNA fragment including the Afl II site at codon 260 continuing to codon 289 followed by an Spe I site was generated by PCR using Pfu DNA polymerase (Stratagene, San Diego, CA). This fragment was used to replace the Afl II - Spe II segment in TSHR-5'TR-NEO-ECE (the Spe I site is at codon 418)(26). Subsequently, an oligonucleotide cassette coding for 6H followed by 2 stop codons with Spe I and Xba I adhesive ends was inserted into the same sites of the intermediate construct. Sense: 5'-CTAGCCATCACCACCACCATCACTGATAAT; Antisense: as described above for TSHR-261. (iii) TSHR-309: Construction used the identical strategy to that of TSHR-289 except that the Afl II - Spe cDNA fragment generated by PCR extended up to codon 309.

After confirmation of the nucleotide sequences of the relevant areas, the TSHR-261, TSHR-289 and TSHR-309 cDNAs were excised with Sal I and Xba I and transferred to the vector pSV2-ECE-dhfr (36).

<u>Expression of TSHR ectodomain variants</u>: Cell lines, stably transfected with the above TSHR ectodomain cDNA variants were established in CHO dhfr-cells (CHO-DG44; kindly provided by Dr. Robert Schimke, Stanford University, Palo Alto, CA), using procedures described previously (34). Transgenome amplification was achieved by progressive adaptation to growth in methotrexate (final concentration 10 $\mu$M)(34).

<u>Detection of TSHR ectodomain variants in medium and in cells</u>: CHO cells to be tested for TSHR ectodomain variant expression were metabolically labeled with $^{35}$S-methionine/cysteine, exactly as described previously (1 hr pulse and overnight chase) (32). The medium was harvested for immunoprecipitation of secreted TSHR protein and the cells were processed further for analysis of intracellular TSHR protein, as described previously (32), with the following modifcations. Cells, lysed in buffer containing 1% Triton X-100, were centrifuged for 45 min at 100,000 x g prior to preclearing with mouse IgG and Protein A, followed by immunoprecipitationusing mouse monoclonal antibody (mAb) A10 (37) (kindly provided by Dr Paul Banga, London, U.K.; epitope at amino acid residues 22-35; final dilution of 1:1000). Medium was precleared in the same manner except that in later experiments mouse IgG was not prebound to the protein A. Samples were subjected to 10% polyacrylamide gel electrophoresis under reducing conditions. Prestained molecular weight markers (BioRad, Hercules, CA) were precalibrated against more accurate unstained markers to obtain the molecular weights indicated in the text. Radiolabeled proteins were visualized by autoradiography on Kodak XAR-5 X-ray film (Eastman Kodak, Rochester, NY). TSHR secreted into the medium was also detected by means of their 6H tag using Ni-NTA resin (QIAGEN, Inc, Chatsworth, CA) according to the procedure reported previously (34).

<u>Assay for neutralization of TSHR autoantibodies in serum of Graves' patients</u>: TSHR autoantibody kits were purchased from Kronus, San Clemente, CA. The principal of this assay is the ability of autoantibodies to compete for $^{125}$I-TSH binding to TSHR solubilized from porcine thyroid glands ("TSH binding inhibition", or TBI assay)(25). In brief, solubilized TSHR (50 ml) are preincubated (15 min) with patient's serum (50 ml). Buffer containing $^{125}$I-TSH is then added (2 h at room temperature). Solubilized TSHR complexed with TSH is precipitated by polyethylene glycol. Antibody activity is measured as % inhibition of $^{125}$I-TSH binding relative to a standard serum from a normal individual without autoantibodies. We modified this assay by preincubating (30 min at room temperature) serum from Graves' patients (25 ml) with conditioned medium from cells expressing TSHR ectodomain variants (25 ml). Solubilized TSHR (50 ml) was then added to the serum/medium mixture (50 ml). As controls, we used serum from normal individuals and conditioned medium from CHO cells secreting a truncated form of thyroid peroxidase (36).

<u>Lectin adsorption</u>: Binding of TSHR in conditioned medium to different lectins was determined for three Sepharose-linked lectins: - Wheat germ agglutinin (WGA), Bandeiraea simplificifolia and Concanavalin A (Con A) (Pharmacia, Piscataway, NJ). Medium (40 ml) was slowly stirred for 2 hr at room temperature with 0.4 ml of Sepharose-lectin. The beads were then extensively washed in batch with 10 mM Tris, pH 7.5, 150 mM NaCl and adsorbed material released (tumbling for 45 min at room temperature) with 3 ml of the same buffer supplemented with 0.25 M N acetyl-glucosamine (WGA), 20 mM a-methyl-galactopyranoside (B. Simplificifolia) and 0.5 M a-methyl-mannoside (Con A). Material (3 ml) was diluted as indicated in the text and spotted on nitrocellulose filters (Schleicher and Schuell, Keene, NH). After air drying, the filters were incubated (45 min) in 50 mM Tris buffer, pH 7.5, and 150 mM NaCl (TBS) containing 5.0% skim milk powder, rinsed and incubated (2 hr at 37°C) in TBS containing mAb A10 (1:1000) and 0.5% bovine serum albumin (BSA). The filters were rinsed, incubated (1 hr at room temperature) with alkaline phosphatase-conjugated goat anti-mouse immunoglobulin G and the signal developed as described previously (34).

<u>Immunoblots of TSHR ectodomain variants</u>: Lectin-bound TSHR-261, TSHR-289 and TSHR-309 was eluted (see above) and Laemmli sample buffer (38) with 0.7 M (final concentration) ß-mercaptoethanol was added (30 min at 45°C). Enzymatic deglycosylation with N-glycosidase F and endoglycosidase H was as described previously (34). After electrophoresis on SDS, 10% polyacrylamide gels, proteins were electophoretically transferred to PVDF membranes, which were then processed as described above with the exception that incubation in mAb A10 was overnight and the second antibody was added for 1-2 hr. In some experiments (e.g. Fig. 22), the immunoblots were developed using the BioMax-CDS-PRO kit (Eastman Kodak, Rochester, NY) according to the protocol of the manufacturer. Autoradiography was with Hyperfilm ECL (Amersham, Arlington Heights, IL).

<u>TSHR-261 partial purification</u>: Conditioned medium was harvested from CHO cells expressing TSHR-261 cultured in non-selective F12 medium containing 10% fetal calf serum, antibiotics and 5 mM Na butyrate (39). Medium (2 liters) was applied to a 70 ml Concanavalin A Sepharose column. After washing with 10 mM Tris, pH 7.5, 150 mM NaCl, bound material was eluted with - 80 ml of 0.15 M a-methyl-mannoside in the same buffer. The eluted material was made up to 50 mM imidazole, pH 7.2, and applied to two 5 ml His-Trap columns in series (Pharmacia). Elution was with buffer containing 10 mM Tris, pH 7.4, 50 mM NaCl, and 100 mM EDTA. The sample was concentrated and the buffer exchanged to 10 mM Tris, pH 7.5, 50 mM NaCl using a Centriprep 30 (Amicon, Beverly, MA). At all stages, TSHR-261 recovery was monitored by bioassay (TBI neutralization; see above). The N-terminal amino acid sequence of the deglycosylated TSHR-261, cut out from a PVDF membrane, was determined by the Protein Structure Laboratory, University of California at Davis.

RESULTS

Secretion of truncated TSHR ectodomain variants: CHO-DG44 cells were stably transfected with plasmids coding for TSHR ectodomain variants truncated at amino acid residues 261, 289 and 309 (Fig. 17). Individual clones were obtained by limiting dilution and transgenome amplification was performed by progressive adaptation to growth in methotrexate (final concentration 10 mM). One clone of each TSHR ectodomain variant, selected for high level of TSHR expression, was expanded and used for further studies.

The ectodomain variant with the greatest degree of C-terminal truncation (TSHR-261) was entirely secreted into the medium, as detected by immunoprecipitation after an overnight chase, with no receptor remaining in the cells (Fig. 18). TSHR-289, truncated to a lesser extent was secreted to an intermediate degree. The receptor remaining within the cells was present in multiple forms, the dominant band having a molecular weight lower than the secreted form. Finally, for TSHR-309, the least truncated ectodomain, secretion into the medium was relatively inefficient. Thus, proportionately less receptor was secreted than remained within the cells, the latter primarily in lower molecular weight form. Expression of the 6 His residues at the carboxyl-termini of the ectodomain variants was confirmed by nickel-NTA resin purification of precursor-labeled material secreted into the culture medium (Fig. 18). In contrast, the TSHR variants could not be clearly identified within the cell because the nickel-NTA resin bound to a large number of labeled intracellular proteins (data not shown).

Interaction of TSHR with autoantibodies in the serum of Graves' patients: Because our main purpose in generating a secreted form of the TSHR ectodomain was to obtain material suitable for study with TSHR autoantibodies in the serum of patients with Graves' disease, it was important to test the secreted TSHR ectodomain variants for this property. We used a TSH binding inhibition (TBI) assay to test whether conditioned medium from cultured cells expressing TSHR-261, TSHR-289 and TSHR-309 could neutralize autoantibody activity in a Graves' patient's serum. Of these, TSHR-261 and TSHR-289 were clearly active in terms of reversing the inhibition by TSHR autoantibodies of $^{125}$I-TSH binding (Fig. 19).

Adsorption of TSHR ectodomain variants to lectins: Although the Ni-NTA resin was effective in purifying radiolabeled TSHR secreted by CHO cells into tissue culture medium (Fig. 18), we were unable to purify unlabeled TSHR protein from medium using this approach. The Ni-NTA bound to many unlabeled proteins despite attempts to minimize non-specific interactions with imidazole and adsorption at lower pH (data not shown). We, therefore, attempted partial purification of TSHR ectodomain variants from conditioned medium using lectins. TSHR-261 in conditioned medium bound poorly to wheat germ agglutinin and Bandeiraea simplicifolia (Fig. 20). Almost all of this material remained in the "flow-through" and minimal amounts could be recovered by elution with specific sugar. In contrast, concanavalin A (Con A) was effective in extracting TSHR-261 from the medium.

Because the non-secreted, full-length TSHR ectodomain that did not interact with TSHR autoantibodies contained immature, high mannose carbohydrate and bound strongly to Con A (34), we were concerned that the Con A was extracting an inactive, high mannose component of TSHR-261, perhaps released from disintegrating cells. Fortunately, this was not the case. Thus, immunoblotting of Con A-enriched TSHR-261 showed that, like the material immunoprecipitated from conditioned medium with mAb A10 (Fig. 18), the secreted receptor was endoglycosidase H resistant and endoglycosidase F sensitive (complex carbohydrate)(Fig. 21). Indeed, this pattern was similar to the smaller amounts of TSHR-261 that could be recovered from conditioned medium using wheat germ agglutinin (Fig. 21). Most important, the Con-A enriched material was highly active in neutralizing autoantibody TBI activity in patients' sera (data not shown for these experiments; see data below on TBI activity in more extensive studies using multiple Graves' sera; Fig. 24).

In addition to TSHR-261, TSHR-289 and TSHR-309 were also extracted from culture medium using Con A. Immunoblotting indicated that TSHR-289 and TSHR-309, like TSHR-261, contained only mature, complex carbohydrate (Fig. 22). Remarkably, TSHR-261 contains ~ 20 kDa of N-linked glycosylation, 40% of its mass. The apparent molecular weights of the deglycosylated proteins (- 30, 32 and 34 kDa for TSHR-261, TSHR-289 and TSHR-309, respectively) were slightly (- 2 kDa) greater than predicted from their known amino acid sequences (including 6 H tags). A similar phenomenon was previously observed with the deglycosylated TSHR ectodomain (residues 1-418)(34). Autoantibody neutralization by partially purified TSHR-261: In the preceding studies, the secreted TSHR variants could be detected qualitatively by immunoprecipitation, immunoblotting or by autoantibody neutralization. However, it was important to determine quantitatively the amount of receptor that was interacting with TSHR autoantibodies in patients' sera. No TSHR standards are available for this purpose. Indeed, the TSHR of mammalian cell origin has never been purified sufficiently for direct visualization on a polyacrylamide gel. We selected TSHR-261 for further study because, of the three ectodomain variants, it was secreted to the greatest extent (Fig. 18) and because its "bioactivity" in terms of autoantibody recognition appeared equal to that of TSHR-289 (Fig. 19).

As monitored by autoantibody neutralization, Con A chromatography provided an initial purification of - 100-fold. Subsequently, and in contrast to its use as an initial capture stem, Ni-chelate chromatography was quite effective in generating sufficient TSHR-261 for direct visualization and quantitation by Coomassie blue staining (Fig. 23, left panel). Enzymatic deglycosylation confirmed the immunoblot evidence for a ~30 kDa polypeptide backbone with -20 kDa of complex carbohydrate (Fig. 23, right panel) and also provided the best means to quantitate the amount of receptor recovered. In 3 separate preparations from 2 liters of conditioned medium, recovery of TSHR-261 (corrected for a 40% glycan component) was 0.3 - 0.4 mg/ liter. Amino acid sequencing of the 30 kDa deglycosylated band confirmed the amino terminus of the TSHR (Met, Gly, X, Ser, Ser, Pro, Pro; X represents a Cys residue). Further purification of TSHR-261 (Sephacryl S-200) was associated with a major loss in activity (data not shown). However, semi-purified TSHR-261 (20-40% estimated purity)(Fig. 23) was quite stable at -80°C.

Partially-purified TSHR-261 was highly potent in neutralizing TSHR autoantibody TBI activity in patients' sera. In a preliminary study on a serum from a Graves' patient with moderate TSH binding inhibitory (TBI) activity, 30 ng of TSHR-261 per tube (0.15 mg/ml) completely neutralized autoantibody activity (Fig. 24). Therefore, in studies on further sera, we used 50 ng of TSHR-261 per tube (0.25 mg/ml). This concentration of TSHR-261 neutralized all or most TSH binding inhibitory activity in the 18 Graves' sera examined (Fig. 25). In a study of our most potent Graves' serum, based on IgG (150 kDa) being bivalent and assuming that 100% of the TSHR-261 molecules added to the assay were capable of autoantibody neutralization, we estimate that the <u>maximum</u> TSHR autoantibody concentration in this particular serum is - 7 mg IgG/ml. From TBI and flow cytometry data (40), most Graves' sera have autoantibody levels 10-50 fold lower than this potent serum. We, therefore, estimate that TSHR autoantibody concentrations are typically between 0.1 and I mg IgG/ml. These values are likely to be overestimates because 100% antigen binding is unlikely to be attained in the assay and because of moderate TSHR-261 lability at room temperature, the temperature of the TBI assay. Finally, although TSHR-261 was clearly recognized by TSHR autoantibodies, it did not bind $^{125}$I-TSH when substituted for the porcine holoreceptor in the TBI kit (data not shown).

DISCUSSION

Advances in the diagnosis and potential immunotherapy of Graves' disease are dependent on the availability of relatively large amounts of immunoreactive, recombinant antigen. Many studies have explored the interaction of Graves' sera with TSHR material generated in bacteria, insect cells, cell-free translates or as synthetic peptides (see Introduction). Most of these studies involved detection of recombinant material by immunoblotting, immunoprecipitation or ELISA, procedures that do not evaluate the ability of this material to interact with functional autoantibodies. Trace amounts of "functional" TSHR ectodomain material are present in insect cells infected with recombinant baculovirus containing the TSHR cDNA (15), as well as in stably transfected CHO cells (33,34). Very recently, there has been confirmation that the TSHR ectodomain generated in a baculovirus system can neutralize TSH binding inhibition (TBI) activity in Graves' patients' sera (16). However, this material was largely insoluble, the active component was not identified or quantitated and the nature of the carbohydrate (complex vs. high mannose) was not determined.

In our experience, the recombinant TSHR of mammalian cell origin is the most effective in interacting with autoantibodies. The strongest testimony to this conclusion is that crude porcine thyroid extracts are still used in the standard clinical TBI assay. TSH holoreceptor overexpression in CHO cells (32) can generate a crude detergent extract that rivals, or surpasses, the efficacy of porcine thyroid extracts (41). However, this membrane-associated material cannot be used for large scale purification for structural studies and will be difficult to use in a future direct (as opposed to an indirect TBI) assay for TSHR autoantibodies.

We have now overcome the previous inability (33,34) to generate in mammalian cells a secreted, soluble, complex carbohydrate-containing form of the TSHR ectodomain. Unlike the non-secreted, high mannose-containing ectodomain (34), secreted, C-terminal truncated TSHR ectodomain variants are recognized by autoantibodies. Whether the complex carbohydrate comprises part of the epitope(s) for TSHR autoantibodies, or whether lack of autoantibody recognition of the "high mannose" ectodomain is secondary to incorrect polypeptide folding (and, hence, retention in the endoplasmic reticulum)(42,43) is presently unknown. It is of interest (and paradoxical) that C-terminal truncation of the LH/CG receptor ectodomain at a position (amino acid residue 294) similar to the TSHR variants generates a non-secreted protein (44). On the other hand, the LH/CG receptor truncated at residue 329 or further downstream is secreted to a limited extent (45,46). Alternately-spliced truncated forms of TSHR mRNA have been detected in thyroid tissue (47,48), however whether these transcripts are actually expressed and, if so, secreted by thyrocytes is unknown.

The lectin specificity of TSHR-261 is consistent, in part, with previous data on the extraction of TSH holoreceptor activity from detergent-solubilized thyroid membranes. In this earlier study, B. simplificifolia was effective for bovine, but not for <u>human,</u> TSHR (49). Unlike the human TSHR ectodomain variants, the bovine TSH holoreceptor was also bound well by Wheat germ agglutinin and was irreversibly bound by Concanavalin A (49). Lectin chromatography by itself was insufficient for TSHR-261 purification. In the future, single-step affinity purification with a mAb will be a preferable approach. At present, murine IgG class mAb have been generated by immunization with TSHR of prokaryotic or insect cell origin (7,37,50,51). Unfortunately, most mAb (including the 6 that we have tried from two different laboratories) (7,37) do not recognize the <u>native</u>, mature mammalian TSHR that is necessary for immunological studies in Graves' disease. Future immunization with complex carbohydrate-containing TSHR-261, or with mammalian cells expressing the TSHR in association with MRC class II molecules (52), may overcome this difficulty.

It has now been determined that TSHR-289 shares many of the advantages of TSHR-261, and may be more stable than TSHR-261, which loses activity over several hours at room temperature. Thus, TSHR-289 may be preferred according to the present invention although TSHR-261 is somewhat more effectively secreted.

Autoantibody-reactivity of TSHR-261 is reminiscent of the observation nearly three decades ago that freezing and thawing thyroid tissue releases a water soluble factor (LATS absorbing activity; LAA) that neutralizes TSHR autoantibodies (53,54). LAA (like TSHR-261) was estimated to be - 50 kDa (54). More recently, a fragment of the TSHR released by trypsin has been observed to neutralize TSHR autoantibodies (55). However, the segment of the TSHR with LAA activity, produced either by freeze-thawing or with trypsin, is unknown.

There are no previous studies on the quantitative neutralization of TSHR autoantibodies in Graves' patients' sera using defined amounts of mammalian antigen. The very small (ng) amounts TSHR-261 required for autoantibody neutralization is orders of magnitude lower than those used in studies with synthetic peptides (24). This quantitative information will be useful as a future benchmark by which to judge the potency of purified TSHR of bacterial and insect cell origin. A corollary of the minute amount of antigen necessary for TSHR autoantibody neutralization is that the autoantibody concentration in patients' sera is very low, consistent with previous flow cytometry data (40). The large difference between TSHR and thyroid peroxidase autoantibody concentrations is of potential significance in understanding the pathogenesis of Graves' disease (56).

The TSHR ectodomain variant TSHR-261, despite its potent autoantibody neutralizing activity, does not bind TSH. The TSH binding site on the TSHR is discontinuous and involves multiple segments throughout the entire ectodomain, including segments downstream of residue 261 (26,57). The TSHR autoantibody epitope(s) may, therefore, be more limited than the TSH binding site. Support for this notion is provided by data from most (9,15,33,34), but not all (58,59), laboratories that TSH binding to the full TSHR ectodomain is negligible or absent. Remarkably, in contrast to TSH, hCG binds with high affinity to the isolated ectodomain of its cognate receptor, even when the latter lacks carbohydrate (reviewed in 60). The reason for this major difference between such closely related receptors is an enigma. The present data with TSHR-261 must also be reconciled with previous evidence obtained using chimeric TSH-LH/CG receptors that the epitope(s) for autoantibodies are also discontinuous and extend downstream of residue 261 (27). One possible explanation for this paradox is that TSHR-261 contains the dominant portion of a discontinuous epitope, sufficient to neutralize most TSHR reactivity in Graves' patients sera.

In summary, progressive carboxyl-terminal truncations lead to the secretion by CHO cells of modified TSHR ectodomains with mature, complex carbohydrate. Most important, TSHR-261 is highly potent in interacting with TSHR autoantibodies. Antigenically active TSHR will provide a major impetus for future studies on the diagnosis, pathogenesis and immunotherapy of Graves' disease.

REFERENCE S CITED IN EXAMPLE 4

42. Vanderpump, M. P. J., Tunbridge, W. M. G., French, J. M., Appleton, D., Bates, D., Clark, F., Grimley Evans, J., Hasan, D. M., Rodgers, H., Tunbridge, F., and Young, E. T. (1995) Clin.Endocrinol. 43, 55-68

2. Rees Smith, B., McLachlan, S. M., and Furmaniak, J. (1988) Endocr. Rev. 9, 106-121

3. Nagayama, Y., Kaufman, K. D., Seto, P., and Rapoport, B. (1989) Biochem.Biophys.Res.Comm. 165, 1184-1190

4. Libert, F., Lefort, A., Gerard, C., Parmentier, M., Perret, J., Ludgate, M., Dumont, J. E., and Vassart, G. (1989) Biochem.Biophys.Res.Comm. 165, 1250-1255

5. Misrahi, M., Loosfelt, H., Atger, M., Sar, S., Guiochon-Mantel, A., and Milgrom, E. (1990) Biochem.Biophys.Res.Comm. 166, 394-403

6. Takai, O., Desai, R. K., Seetharamaiah, G. S., Jones, C. A., Allaway, G. P., Akamizu, T., Kohn, L. D., and Prabhaltar, B. S. (1991) Biochem.Biophys.Res.Comm. 179, 319-326

7. Loosfelt, H., Pichon, C., Jolivet, A., Misrahi, M., Caillou, B., Jamous, M., Vannier, B., and Milgrom, E. (1992) Proc. Natl. Acad. Sci. USA 895, 3765-3769

8. Harfst, E., Johnstone, A. P., and Nussey, S. S. (1992) J. Mol. Endocrinol. 9, 227-236

9. Huang, G. C., Collison, K. S., McGregor, A. M., and Banga, J. P. (1992) J. Mol. Endocrinol. 8, 137-144

10. Costagliola, S., Alcalde, L., Ruf, J., Vassart, G., and Ludgate, M. (1994) J. Mol. Endocrinol. 13, 11-21

11. Graves, P. N., Vlase, H., and Davies, T. F. (1995) Endocrinology 136, 521-527

12. Huang, G. C., Page, M. J., Nicholson, L. B., Collison, K. S., McGregor, A. M., and Banga, J. P. (1993) J. Mol. Endocrinol. 10, 127-142

13. Seetharamaiah, G. S., Desai, R. K., Dallas, J. S., Tahara, K., Kohn, L. D., and Prabhakar, B. S. (1993) Autoimmunity 14, 315-320

14. Vlase, H., Graves. P., Magnusson, R. P., and Davies, T. F. (1995) J. Clin. Endocrinol. Metab. 80, 46-53

15. Chazenbalk, G. D. and Rapoport, B. (1995) J. Biol. Chem. 270, 1543-1549

16. Seetharamaiah, G. S., Dallas, J. S., Patibandla, S. A., Thotakura, N. R., and Prabhakar, B. S. (1997) J.Immunol. 158, 2798-2804

17. Perret, J., Ludgate, M., Libert, F., Gerard, C., Dumont, J. E., Vassart, G., and Parmentier, M. (1990) Biochem.Biophys.Res.Comm. 171, 1044-1050

18. Harfst, E., Johnstone, A. P., Gout, I., Taylor, A. H., Waterfield, M. D., and Nussey, S. S. (1992) Molec.Cell.Endocrinol. 83, 117-123

19. Endo, T., Ohmori, M., Ikeda, .M., Anzai, E., and Onaya, T. (1992) BBRC 186, 1391-1396

20. Matsuba, T., Yamada, M., Suzuki, H., Kanai, A., Isozaki, O., Yoshida, T., Tsushima, T., and Yasukawa, K. (1995) J.Biochem. 118, 265-270

21. Murakami, M., Miyashita, K., Kakizaki, S., Saito, S., Yamada, M., Iriuchijima, T., Takeuchi, T., and Mori, M. (1995) Eur. J. Endocrinol. 133, 80-86

22. Morgenthaler, N. G., Tremble, J., Huang, G., Scherbaum, W. A., McGregor, A. M., and Banga, J. P. (1996) J. Clin. Endocrinol. Metab. 81, 700-706

23. Nagy, E. V., Burch, H. B., Mahoney, K., Lukes, Y. G., Morris III, J. C., and Burman, K. D. (1992) BBRC 188, 28-33

24. Morris, J. C., Gibson, J. L., Haas, E. J., Bergert, E. R., Dallas, J. S., and Prabhakar, B. S. (1994) Autoimmunity 17, 287-299

25. Shewring, G. A. and Rees Smith, B. (1982) Clin.Endocrinol. 17, 409-417

26. Nagayama, Y., Wadsworth, H. L., Chazenbalk, G. D., Russo, D., Seto, P., and Rapoport, B. (1991) Proc. Natl. Acad. Sci. USA 88, 902-905

27. Nagayama, Y., Wadsworth, H. L., Russo, D., Chazenbalk, G. D., and Rapoport, B. (1991) J. Clin. Invest. 88, 336-340

28. Tahara, K., Ban, T., Minegishi, T., and Kohn, L. D. (1991) Biochem.Biophys.Res.Comm. 179, 70-77

29. Wadsworth, H. L., Chazenbalk, G. D., Nagayama, Y., Russo, D., and Rapoport, B. (1990) Science 249, 1423-1425

30. Ludgate, M., Perret, J., Parmentier, M., Gerard, C., Libert, F., Dumont, J. E., and Vassart, G. (1990) Molec.Cell.Endocrinol. 73, R13-R18

31. Filetti, S., Foti, D., Costante, G., and Rapoport, B. (1991) J. Clin. Endocrinol. Metab. 72, 1096-1101

32. Chazenbalk, G. D., Kakinuma, A., Jaume, J. C., McLachlan. S. M., and Rapoport, B. (1996) Endocrinology 137, 4586-4591

33. Harfst, E., Johnstone, A. P., and Nussey, S. S. (1992) Lancet 339, 193-194

34. Rapoport, B., McLachlan, S. M., Kakinuma, A., and Chazenbalk, G. D. (1996) J. Clin. Endocrinol. Metab. 81, 2525-2533

35. Kakinuma, A., Chazenbalk, G., Filetti, S., McLachlan, S. M., and Rapoport, B. (1996) Endocrinology 137, 2664-2669

36. Kaufman, K. D., Foti, D., Seto, P., and Rapoport, B. (1991) Molec.Cell.Endocrinol. 78, 107-114

37. Nicholson, L. B., Vlase, H., Graves, P., Nilsson, M., Molne, J., Huang, G. C., Morgenthaler, N. G., Davies, T. F., McGregor, A. M., and Banga, J. P. (1996) J. Mol. Endocrinol. 16, 159-170

38. Laemmli, U. K. (1970) Nature 227, 680-685

39. Domer, A. J., Wasley, L. C., and Kaufman, R. J. (1989) J. Biol. Chem. 264, 20602-20607

40. Jaume, J. C., Kakinuma, A., Chazenbalk, G. D., Rapoport, B., and McLachlan, S. M. (1997) J. Clin. Endocrinol. Metab. 82, 500-507

41. Kakinuma, A., Chazenbalk, G. D., Jaume, J. C., Rapoport, B., and McLachlan, S. M. (1997) J. Clin. Endocrinol. Metab. (in press)

42. Kornfeld, R. and Kornfeld, S. (1985) Annu. Rev. Biochem. 54, 631-646

43. Robbins, P. W., Hubbard, S. C., Turco, S. J., and Wirth, D. F. (1977) Cell 12, 893-900

44. Koo, Y. B., Ji, I., and Ji, T. H. (1994) Endocrinology 134, 19-26

45. Tsai-Morris, C. H., Buczko, E., Wang, W., and Dufau, M. L. (1990) J. Biol. Chem. 265, 19385-19388

46. VuHai-LuuThi, M. T., Misrahi, M., Houllier, A., Jolivet, A., and Milgrom. E. (1992) Biochem. 31, 8377-8383

47. Takeshita, A., Nagayama, Y., Fujiyama, K., Yokoyama, N., Namba, H., Yamashita, S., Izumi, M., and Nagataki, S. (1992) Biochem.Biophys.Res.Comm. 188, 1214-1219

48. Graves, P. N., Tomer, Y., and Davies. T. F. (1992) Biochem.Biophys.Res.Comm. 187, 1135-1143

49. Kress, B. C. and Spiro, R. G. (1986) Endocrinology 118, 974-979

50. Johnstone, A. P., Cridland, J. C., DaCosta, C. R., Harfst, E., and Shepherd, P. S. (1994) Molec.Cell.Endocrinol. 105, R1-R9

51. Seetharamaiah, G. S., Wagle, N. M., Morris, J. C., and Prabhakar, B. S. (1995) Endocrinology 136, 2817-2824

52. Shimojo, N., Kohno, Y., Yamaguchi, K., Kikuoka, S., Hoshioka, A., Niimi, H., Hirai, A., Tamura, Y., Saito, Y., Kohn, L. D., and Tahara, K. (1996) Proc. Natl. Acad Sci. USA 93, 11074-11079

53. Smith, B. R. (1970) J. Endocrinol. 46, 45-54

54. Dirmikis, S. and Munro, D. S. (1973) J. Endocrinol. 58, 577-590

55. Foti, D., Russo, D., Costante, G., and Filetti, S. (1991) J. Clin. Endocrinol. Metab. 73, 710-716

56. McLachlan, S. M. and Rapoport, B. (1993) J.Int.Med. 234, 347-359

57. Nagayama, Y., Russo, D., Chazenbalk, G. D., Wadsworth, H. L., and Rapoport, B. (1990) Biochem.Biophys.Res.Comm. 173, 1150-1156

58. Shi, Y., Zou, M., Parhar, R. S., and Farid, N. R. (1993) Thyroid 3, 129-133

59. Seetharamaiah, G. S., Kurosky, A., Desai, R. K., Dallas, J. S., and Prabhakar, B. S. (1994) Endocrinology 134, 549-554

60. Segaloff, D. L. and Ascoli, M. (1993) Endocr. Rev. 14, 324-347

## EXAMPLE 5: THYROTROPIN RECEPTOR AUTOANTIBODIES IN SERUM ARE PRESENT AT MUCH LOWER LEVELS THAN THYROID PEROXIDASE AUTOANTIBODIES: ANALYSIS BY FLOW CYTOMETRY

This Example presents data providing the strongest support for the notion that TSHR autoantibodies in the sera of patients with autoimmune thyroid disease arc present at much lower levels than arc TPO autoantibodies. This finding has important implications for the diagnostic detection of TSHR autoantibodies and for understanding the pathogenesis of Graves' disease.

INTRODUCTION

At present, autoantibodies to the thyrotropin receptor (TSHR) in the sera of patients with Graves' disease are detected most effectively by an indirect approach, namely their ability to inhibit radiolabeled TSH binding (1). Over many years, studies to directly determine TSHR autoantibody binding to its antigen have been fraught with difficulty. For example, immunoblotting or immunoprecipitation of thyroid tissue or cell extracts with patients' sera (2,3) have not revealed antigen consistent with the presently known structure of the TSHR. Indirect immunofluorescence with thyroid cells in monolayer culture has only been observed with two highly selected sera (4). Even with recombinant TSHR preparations, it has been difficult to demonstrate a direct interaction between serum autoantibodies and the TSHR. Thus, prior purification of either antigen (5-7) or antibody (8), or the use of cell-free translates (9), has been necessary. This difficulty with autoantibodies contrasts with excellent TSHR recognition by antibodies from immunized animals, in particular monoclonal antibodies (10-13).

A number of factors may contribute to the difficulty in detecting TSHR autoantibody binding by direct means, including:- i) low TSHR concentration, ii) requirement for conformational integrity of the antigen, iii) the high background observed with polyclonal antibodies in human sera and, iv) low autoantibody titer. Autoantibodies to thyroid peroxidase may be present at very high concentrations (14). An early study suggested that TSHR autoantibodies are present at very high concentrations in serum (2-3% of total IgG). More recently, consistent with the small number of TSHR autoantibody-specific B cells in Graves' patients (16), immunofluorescence data suggest that TSHR autoantibody concentrations may be low (4,17). The use of different assays to detect TPO and TSHR autoantibodies has precluded a direct comparison of their concentrations in the same serum.

In the present study, we have used a new TSHR-expressing mammalian cell line that overcomes two of these handicaps, namely TSHR concentration and structural integrity. Thus, TSHR-10,000 Chinese hamster ovary (CHO) cells express high numbers of mature TSHR on their surface in vivo (18). Using these cells, we now report the ability to detect directly, by flow cytometry, binding of autoantibodies in a few patients' sera to the native TSHR. However, flow cytometric analysis of thyroid peroxidase (TPO) autoantibodies in the same sera indicate that TSHR autoantibodies are present at much lower levels than are TPO autoantibodies. This finding has important implications for the diagnostic detection of TSHR autonantibodies and for understanding the pathogenesis of Graves' disease.

METHODS

Sera used for flow cytometry: Serum BB1, kindly provided by Dr. Stephanie Lee, New England Medical Center Hospitals, Boston, was from a patient with Graves' disease who subsequently became hypothyroid with the development of blocking antibodies. Thirty sera were generously provided to us by Mr. Juan Tercero, Corning-Nichols Institute. San Juan Capistrano, California. These sera were submitted without clinical information for the assay of autoantibodies to the TSHR (see below), presumably for suspicion of Graves' disease. Ten of the 30 sera were selected for the absence of TSHR autoantibodies, 10 for the presence of moderate TSH binding inhibitory (TBI) activity and a further 10 for their high (>50%) TBI activities. Four laboratory personnel were the source of sera from individuals clearly without autoimmune thyroid disease and four sera were from patients with systemic lupus erythematosus with anti-DNA and/or anti-cardiolipin antibodies.

All sera were tested in our laboratory for TBI activity using a commercially available diagnostic kit (Kronus, San Clemente, CA). The 30 sera obtained from Coming-Nichols had been tested previously using the same assay with very similar results. TSHR autoantibody titers of selected sera were determined by dilution of these sera in a serum from a normal individual without a history of autoimmune thyroid disease and with undetectable TSHR autoantibody activity. Sera were also tested for TPO autoantibodies using $^{125}$I-TPO (recombinant) as described previously (19).

Cells expressing TPO and TSHR: Construction of plasmids and Chinese hamster ovary (CHO) stable transfections for overexpression of the human TSHR and TPO have been described elsewhere (18,20). These cells, as well as untransfected CHO cells (DG44; kindly provided by Dr. Robert Schimke, Stanford University, Palo Alto, CA) were propagated in Ham's F-12 medium supplemented with 10% fetal calf serum, penicillin (100 U/ml), gentamicin (50 $\mu$g/ml) and amphotericin B (2.5 $\mu$g/ml).

Flow cytometric analysis (FACS): For analysis of patients' sera, cells were processed as described previously for TPO expression on CHO cells using patients' sera and human monoclonal autoantibodies (21,22), with modifications. In brief, after detachment by mild trypsinization, cells were pelleted and rinsed (5 min at 100 x g) in Ham's F-12 medium supplemented with 10% dialyzed fetal calf serum and antibiotics as above. For preadsorption, untransfected CHO cells were resuspended in 0.18 ml of buffer A (phosphate-buffered saline, 10 mM Hepes, pH 7.4, 0.05% Na azide and 2% fetal calf serum heat-inactivated at 56°C for 30 min). Sera (20 $\mu$l) were added (final concentration of 1:10, or as specified in the text) and gently mixed for 60 min at 4°C. After removal of the cells by centrifugation, the sera were divided in two 0.1 ml aliquots. One aliquot was added to CHO cells expressing either the TSHR or TPO, the other aliquot to untransfected CHO cells. After incubation for 60 min at 4°C, the cells were rinsed three times in buffer A and resuspended in 0.1 ml of same buffer. Affinity-purified goat anti-human IgG (1.5$\mu$l)(Fc specific, fluorescein isothiocyanate-conjugated; Caltag, South San Francisco, CA) was added to the cells for 45 min at 4°C. After three washes in buffer A, the cells were analyzed using the Becton Dickinson FACScan-CELLQuest system. Three parameters (forward scatter, FSC, 90° side scatter, SSC, and FL1 detector) were use for the analysis. All assays included cells treated with second antibody alone and serum from normal individuals. All sera were analyzed by flow cytometry at least twice.

Detection of TSHR on TSHR-10,000 cells using mouse monoclonal antibodies (A9 and A10)(1:100 final concentration)(13) and rabbit antiserum (1:60 final concentration)(R8)(23) to the TSHR (all kindly provided by Dr. Paul Banga, London, England) was according to the protocol described for the human sera, except for the use of the use of the following second antibodies:- (i) affinity-purified goat anti-mouse IgG (0.8 $\mu$l)(fluorescein isothiocyanate-conjugated) and (ii) affinity-purified goat anti-rabbit IgG (0.5 $\mu$l)(fluorescein isothiocyanate-conjugated), both from Caltag. South San Francisco, CA.

RESULTS

Flow cytometric analysis of CHO cells expressing the TSHR: We have previously been unable to detect the TSHR stably expressed on the surface of CHO cells using Graves' sera. Recently, however, two reagents became available to us; CHO cells expressing very high numbers of TSHR (nearly 2 x $10^6$) on their surface (18), and a Graves' serum (BBI) particularly potent in the indirect TSH binding inhibition (TBI) assay. Using this serum, a very small specific signal (relative to control untransfected cells) was detected with our original line (24) expressing ~ 16,000 TSHR per cell (Fig. 26A). With the same serum, a stronger signal was observed with a line expressing - 150,000 TSHR per cell (TSHR-0; previously termed 5'3'TR-ECE)(25)(Fig. 26B). Progressively greater specific fluorescence was evident with TSHR-800 and TSHR-10,000 cells expressing - $10^6$ and 1.9 x $10^6$ receptors per cell, respectively (18)(Fig. 26C and 26D). Expression of TSHR antigen on the surface of the transfected CHO cells, previously determined by radiolabeled TSH binding and TSH-mediated signal transduction (18,24,25), was confirmed using a rabbit polyclonal antiserum (R8) to the TSHR (Fig. 27C). Murine monoclonal antibodies (A9 and A10)(13) were less effective in recognizing the native TSHR on the cell surface (Fig. 27A, 27B). The TSHR-10,000 cells were, therefore, used in subsequent attempts to detect TSHR autoantibodies in the sera of other patients by direct binding to the native antigen.

Use of TSHR-10,000 cells to detect TSHR autoantibodies in different sera: In preliminary studies, we observed that some sera, regardless of whether or not they contained TSHR autoantibodies, elicited high fluorescence on flow cytometry with TSHR-10,000 cells. We observed similar high fluorescence when these sera were incubated with untransfected CHO cells not expressing the TSHR (data not shown). Therefore, some sera contained antibodies against unknown antigens on the surface of CHO cells. For this reason, we instituted a preadsorption step, in which sera were preincubated with untransfected CHO cells prior to addition to the TSHR-10,000 cells. Preadsorption was effective in eliminating, or greatly reducing, this non-specific background. For example, without preadsorption, a serum (7H) with a high TBI value (81.7%) induced a strong signal on TSHR-10,000 cells relative to that of a serum without TSHR autoantibodies (Fig. 28A). However, after preadsorption, the activity in this apparently positive serum was clearly non-specific (Fig. 28B). In contrast, with sera like BBI (see above), preadsorption actually enhanced the specificity of the signal (Fig. 28C and 28D).

We have studied the interaction with the TSHR-10,000 cells and untransfected CHO cells of 39 sera after preadsorption on untransfected CHO cells (Table 1). In addition to serum BB1, this panel included 10 sera (IL - 10L) without TBI activity (1 - 4.2% inhibition); 10 sera (1M - 10M) with moderately high TBI values (17.3 - 39.4% inhibition);

10 sera (1H - 10H) with high TBI levels (52 - 95.1% inhibition); four normal individuals without autoimmune thyroid disease and four patients with systemic lupus erythematosus with anti-DNA and/or anti-cardiolipin antibodies. None of the sera from normal individuals, individuals with negative TBI values or patients with systemic autoimmunity generated a positive signal on flow cytometry. Remarkably, including BB1 (selected for its very high TBI activity) only four of the 21 sera positive for TBI (10M, 3H and 10H) unequivocally recognized the TSHR on flow cytometry using cells expressing large numbers of receptors (Table 1).

Specificity of the FACS signals on TSHR-10,000 cells observed with sera BB1, 10M, 3H and 10H was evident by the return to background of the signals following pre-adsorption on TSHR-10,000 cells, rather than on untransfected cells (Fig. 29).

The TSHR autoantibody titers in the four sera positive for the TSHR on flow cytometry were determined in the TB1 assay (Table 2). Dilution of these sera indicated that BB1 and 10H had similar, high TSHR autoantibody titers, consistent with their strong fluorescence signals on flow cytometry. The lower TBI titer of serum 3H was also consistent with its relatively low fluorescence signal. Surprisingly, serum 10M, with the lowest TSHR autoantibody titer, generated a strong signal on flow cytometry, raising the possibility of the presence in this serum of "neutral" autoantibodies to the TSHR that do not inhibit TSH binding.

TPO autoantibodies detected by flow cytometry: In view of the small number of sera that could unequivocally recognize the TSHR by flow cytometry, we studied TPO autoantibodies in the same sera by the same approach using CHO cells overexpressing TPO on their surface (20). TPO autoantibodies commonly coexist with TSHR autoantibodies. Indeed, of the 20 TBI positive sera (1-10M and 1-10H), 19 bound > 13% $^{125}$I-TPO, well above the upper limit detected in the sera of normal individuals (2.6% binding)(Table 1). In addition, two of the TBI-negative sera (7L and 10L) were also TPO autoantibody positive by this method. Strikingly, all 20 sera with detectable TPO autoantibodies were clearly positive on flow cytometry with CHO-TPO cells. Not only were more sera positive for TPO than for the TSHR by flow cytometry, but the net fluorescence (after subtraction of fluorescence with non-transfected cells) was far higher with TPO-expressing cells than with TSHR-expressing cells (Table 1). Like the TSHR-10,000 cells, the cells overexpressing TPO (TPO-10,000) have ~ $2 \times 10^6$ TPO molecules on their surface (unpublished data).

We studied the serum (BB1) with the highest TSHR autoantibody level by flow cytometry to determine the extent to which the serum could be diluted before loss of a fluorescence signal (Fig. 29). Serum from a normal individual was diluted in a similar manner (open histograms). Both sera, at all dilutions tested, were preadsorbed on untransfected CHO cells prior to incubation with TSHR-10,000 cells. The fluorescence signal with BB1 (Fig. 30: left panels, shaded histograms) progressively diminished upon dilution. At 1:10 the net signal (BB1 - the normal serum) was 276.3 fluorescence units. At 1:1000, BBI elicited only a very small signal (net fluorescence of 7.3 units). When the same adsorbed serum was incubated with TPO-10,000 cells, the net fluorescence diminished from 1240.9 to 243.6 fluorescence units (Fig. 30; right panels, shaded histograms). Similar fluorescence (243.6 and 276.3 units) was evident for TPO autoantibodies and TSHR autoantibodies at dilutions of 1:1000 and 1:10, respectively. Thus, by flow cytometry, the titer of TPO autoantibodies in the BB1 serum is - 100-fold higher than that for TSHR autoantibodies in the same serum.

It should be noted that with a number of sera, fluorescence was lower with TSHR-10,000 or (less commonly) TPO-10,000 cells than with untransfected control cells, resulting in a negative "net" fluorescence value (Table 1). This greater fluorescence elicited by some sera with control cells could not be eliminated by more stringent adsorption (data not shown).

DISCUSSION

The present study, facilitated by the recent availability of CHO cells overexpressing the human TSHR (18), demonstrates that IgG class autoantibodies to the TSHR can be detected directly by flow cytometry. However, an unequivocal signal by flow cytometry was obtained with only 4 of 21 TBI positive sera, including some selected on the basis of their high TSHR autoantibody activity. Indeed, 11 of these sera had high TBI levels (>50% inhibition). In contrast to TSHR autoantibodies, all 20 sera with TPO autoantibodies detected by $^{125}$I-TPO binding gave strongly positive signals on flow cytometry using TPO-expressing CHO cells.

Several lines of evidence suggest that the factor responsible for the low frequency and amplitude of TSHR autoantibody detection by flow cytometry relative to TPO autoantibodies is the very low levels in serum of the former. First, the availability of cells expressing very large numbers of TSHR in native conformation eliminates the limiting factor of insufficient antigen for adequate detection. Thus, both the TSHR-10,000 cells and the TPO-overexpressing CHO cell line have similar numbers (- $2 \times 10^6$) of specific antigen molecules on their surface. Second, the weak signal observed with TSHR autoantibodies cannot be attributed to a low affinity for antigen. Thus, even though their affinities have not been directly determined, they are capable of competing for high affinity (~ $10^{-10}$ M) TSH binding to the TSHR. TPO autoantibodies are known to be of similar affinity for their antigen (reviewed in 26). Finally, a 1:1000 dilution of a potent serum for TSHR autoantibodies nearly eliminated its signal on flow cytometry, whereas at the same dilution, TPO autoantibodies in this serum continued to produce a very strong signal.

The reason for the "net negative" fluorescence for TSHR-10,000 cells observed with some sera is unknown. One possible explanation for this phenomenon is that the high level of TSHR or TPO expression diminishes expression of another, unknown antigen in CHO cells recognized by these sera (21). Expression of this antigen could, for example, be diminished by disruption of its gene by one of the many transgenome copies produced by the amplification process. Presumably, the very high fluorescence observed with TPO autoantibody-positive sera masks the phenomenon of a negative net fluorescence.

The present findings confirm our previous hypothesis (17) of low TSHR autoantibody levels in serum, based on the inability to detect TSHR autoantibodies by indirect immunofluorescence on CHO cells stably expressing the TSHR cDNA. Our data are in accordance with the detection of an immunofluorescence signal on cultured thyroid cells by only two exceptionally potent Graves' sera (4). Of interest, detection of TSHR autoantibodies by flow cytometry in a serum (10M) with only moderate TBI activity, provides support for the concept that, in addition to autoantibodies to the TSH binding site, some TSHR autoantibodies recognize epitopes outside of this region.

The very low concentration of TSHR autoantibodies in serum carries a number of implications. First, our findings explain the previous difficulties in using Graves' sera to detect the TSHR by immunoblotting or by immunoprecipitation. Second, the data also explain the lesser ability, relative to TPO and thyroglobulin autoantibodies, to detect TSHR autoantibody synthesis and secretion by patients' lymphocytes in vitro (27,28). This low frequency of TSHR-specific B lymphocytes and plasma cells is also likely to contribute to the great difficulty in obtaining IgG-class human monoclonal autoantibodies to the TSHR (reviewed in 29). Third, flow cytometry cannot be used as an alternative to currently used clinical assays to detect TSHR autoantibodies. Fourth, and of greater importance, it is likely to be difficult to establish direct binding assays for TSHR autoantibodies of clinical relevance, even with purified antigen.

In conclusion, the present data provide the strongest support for the notion that TSHR autoantibodies in the sera of patients with autoimmune thyroid disease are present at much lower levels than are TPO autoantibodies. This finding carries important implications for future studies to understand the pathogenesis of Graves' disease. Thus, as hypothesized previously (17), the low concentration in serum of TSHR autoantibodies suggests that, although often present for many years, they arise at an early stage of the autoimmune process. Support for this concept is provided by restricted kappa or lambda light chain usage (30-32) and relative restriction to the IgG1 subclass (33) of TSHR autoantibodies in some patients.

REFERENCE CITED IN EXAMPLE 5

61. Shewring, GA, Rees Smith, B. An improved radioreceptor assay for TSH receptor antibodies. Clin.Endocrinol. 1982; 17:409-417.

2. Heyma, P, Harrison, LC. Precipitation of the thyrotropin receptor and identification of thyroid autoantigens using Graves' disease immunoglobulins. J Clin Invest. 1984; 74:1090-1097.

3. Rapoport. B, Seto, P, Magnusson, RP. Immunoprecipitation of radiolabeled human thyroid cell proteins by serum from patients with autoimmune thyroid disease. Endocrinology. 1984; 115:2137-2144.

4. De Forteza, R. Smith, CU, Amin, J. McKenzie, JM, Zakarija, M. Visualization of the thyrotropin receptor on the cell surface by potent autoantibodies. J Clin Endocrinol & Metab. 1994; 78:1271-1273.

5. Huang, GC, Page, MJ. Roberts, AJ, Malik, AN, Spence, H, McGregor, AM, Banga, JP. Molecular cloning of a human thyrotropin receptor cDNA fragment. FEBS Letters. 1990; 264:193-197.

6. Graves, PN, Vlase, H, Davies, TF. Folding of the recombinant human thyrotropin (TSH) receptor extracellular domain: identification of folded monomeric and tetrameric complexes that bind TSH receptor autoantibodies. Endocrinology. 1995; 136:521-527.

7. Vlase, H, Graves, P, Magnusson, RP, Davies, TF. Human autoantibodies to the thyrotropin receptor: recognition of linear, folded, and glycosylated recombinant extracellular domain. J Clin Endocrinol Metab. 1995: 80:46-53.

8. Tonacchera, M, Costagliola. S, Cetani, F, Ducobu, J, Stordeur. P, Vassart, G, Ludgate, M. Patient with monoclonal gammopathy, thyrotoxicosis, pretibial myxedema and thyroid-associated ophthalmopathy; demonstration of direct binding of autoantibodies to the thyrotropin receptor. Eur J Endocrinol. 1996; 134:97-103.

9. Morgenthaler, NG, Tremble, J, Huang, G, Scherbaum, WA, McGregor, AM, Banga, JP. Binding of antithyrotropin receptor autoantibodies in Graves' disease serum to nascent, in vitro translated thyrotropin receptor: Ability to map epitopes recognized by antibodies. J Clin Endocrinol Metab. 1996; 81:700-706.

10. Loosfelt, H, Pichon, C, Jolivet, A, Misrahi, M, Caillou, B. Jamous, M, Vannier, B, Milgrom. E. Two-subunit structure of the human thyrotropin receptor. Proc Natl Acad Sci USA. 1992; 895:3765-3769.

11. Johnstone, AP, Cridland, JC, DaCosta. CR, Harfst, E, Shepherd, PS. Monoclonal antibodies that recognize the native human thyrotropin receptor. Molec.Cell.Endocrinol. 1994; 105:R1-R9.

12. Seetharamaiah. GS. Wagle, NM, Morris, JC, Prabhakar, BS. Generation and characterization of monoclonal antibodies to the human thyrotropin (TSH) receptor: antibodies can bind to discrete conformational or linear epitopes and block TSH binding. Endocrinology. 1995; 136:2817-2824.

13. Nicholson, LB, Vlase, H, Graves, P, Nilsson, M, Molne, J, Huang, GC, Morgenthaler, NG, Davies, TF, McGregor, AM, Banga, JP. Monoclonal antibodies to the human thyrotropin receptor: Epitope mapping and binding to the native receptor on the basolateral plasma membrane of thyroid follicular cells. J Mol Endocrinol. 1996; 16:159-170.

14. Beever, K, Bradbury, J, Phillips, D, McLachlan, SM, Pegg, C, Goral, A. Overbeck, W, Feifel, G, Rees Smith, B. Highly sensitive assays of autoantibodies to thyroglobulin and to thyroid peroxidase. Clin Chem. 1989; 35: 949-1954.

15. Smith, BR. Characterization of long-acting thyroid stimulator gG binding protein. Biochim. Biophys. Acta. 1971; 229:649-662.

16. Rees Smith, B. McLachlan, SM. Furmaniak. J. Autoantibodies to the thyrotropin receptor. Endocr Rev. 1988; 9: 106-121.

17. McLachlan, SM, Rapoport. B. Recombinant thyroid autoantigens: the keys to the pathogenesis of autoimmune thyroid disease. J.Int.Med. 1993; 234:347-359.

18. Chazenbalk. GD, Kakinuma, A, Jaume, JC. McLachlan, SM. Rapoport, B. Evidence for negative cooperativity among human thyrotropin receptors overexpressed in mammalian cells. Endocrinology. 1996;(In Press)

19. Portolano, S, Chazenbalk, GD, Seto. P. Hutchison, JS, Rapoport, B, McLachlan, SM. Recognition by recombinant autoimmune thyroid disease-derived Fab fragments of a dominant conformational epitope on human thyroid peroxidase. J Clin Invest. 1992; 90:720-726.

20. Kaufman, KD, Foti. D, Seto. P, Rapoport, B. Overexpression of an immunologically-intact, secreted form of human thyroid peroxidase in eukaryotic cells. Molec.Cell.Endocrinol. 1991; 78:107-114.

21. Kaufman, KD, Rapoport, B, Seto, P. Chazenbalk, GD, Magnusson, RP. Generation of recombinant, enzymatically-active, human thyroid peroxidase and its recognition by antibodies in the sera of patients with Hashimoto's thyroiditis. J Clin Invest. 1989; 84:394-403.

22. Nishikawa, T, Rapoport, B, McLachlan, SM. Exclusion of two major areas on thyroid peroxidase from the immunodominant region containing the conformational epitopes recognized by human autoantibodies. J Clin Endocrinol Metab. 1994; 79:1648-1654.

23. Vlase, H, Nakashima, M, Graves, PN, Tomer, Y. Morris, JC. Davies, TF. Defining the major antibody epitopes on the human thyrotropin receptor in immunized mice: evidence for intramolecular epitope spreading. Endocrinology. 1995: 136:4415-4423.

24. Nagayama, Y, Kaufman, KD, Seto, P, Rapoport, B. Molecular cloning, sequence and functional expression of the cDNA for the human thyrotropin receptor. Biochem.Biophys.Res.Comm. 1989; 165:1184-1190.

25. Kakinuma, A, Chazenbalk, G, Filetti, S, McLachlan, S. Rapoport, B. Both the 5' and 3' non-coding regions of the thyrotropin receptor messenger RNA influence the level of receptor protein expression in transfected mammalian cells. Endocrinology. 1996; 137:2664-2669.

26. McLachlan, SM, Rapoport. B. Genetic and epitopic analysis of thyroid peroxidase (TPO) autoantibodies: markers of the human thyroid autoimmune response. Clin.Exp.Immunol. 1995; 101:200-206.

27. McLachlan, SM, Rees Smith, B, Petersen, VB, Davies, TF, Hall, R. Thyroid stimulating autoantibody production in vitro. Nature. 1977; 270:447-449.

28. McLachlan, SM, Pegg, CAS, Atherton, MC, Middleton, SM, Clark, F, Rees Smith. B. TSH receptor antibody synthesis by thyroid lymphocytes. Clin.Endocrinol. 1986; 24:223-230.

29. McLachlan, SM, Rapoport, B. Editorial: Monoclonal human autoantibodies to the TSH receptor: the holy grail, and why are we looking for it? J Clin Endocrinol Metab. 1996; 81:3152-3154.

30. Zakarija, MJ. Immunochemical characterization of the thyroid-stimulating antibody (TSab) of Graves' disease: evidence for restricted heterogeneity. J.Clin.Lab.Immunol. 1983; 10:77-85.

31. Knight, J, Laing, P, Knight, A, Adams, D, Ling, N. Thyroid stimulating autoantibodies usually contain only lambda-light chains: evidence for the "forbidden clone" theory. J Clin Endocrinol Metab. 1986; 62:342-347.

32. Williams, RCJ, Marshall, NJ, Kilpatrick, K, Montano, J. Brickell, PM, Goodall, M, Ealey, PA, Shine, B, Weetman. AP, Craig, RK. Kappa/lambda immunoglobulin distribution in Graves' thyroid-stimulating antibodies. J Clin Invest. 1988; 82:1306-1312.

33. Weetman, AP, Yateman, ME, Ealey, PA, Black, CM, Reimer, CB, Williams, RCJ, Shine, B, Marshall, NJ. Thyroid-stimulating antibody activity between different immunoglobulin G subclasses. J Clin Invest. 1990; 86:723-727.

## EXAMPLE 6: IMPLICATION OF N-LINKED GLYCOSYLATION IN THE EVOLUTIONARY DIVERGENCE OF RECEPTOR SUBUNIT STRUCTURE

The thyrotropin receptor (TSHR) and lutropin/chorionic gonadotropin receptor (LH/CGR) are closely related members of the G protein-coupled receptor family with glycoprotein hormone ligands. Both the TSHR and LH/CGR have large, heavily glycosylated ectodomains with leucine-rich repeats and are encoded by numerous exons (1-3). Despite their common evolutionary background, these receptors have several remarkable differences aside from the specificity of their ligands. For example, disease-causing autoantibodies to the TSHR are common, whereas autoantibodies to the LH/CGR are extremely rare. Thus, engagement of the TSHR by autoantibodies is the direct cause of thyrotoxicosis in Graves' disease, an organ-specific autoimmune disorder affecting only humans. Another striking difference exists at the structural level. As detected by TSH crosslinking to the surface of intact cells, the functional TSHR exists in two forms; a single chain receptor and a receptor with two subunits (4.5). In contrast, the LH/CGR exists only in a single chain form (reviewed in 6). Variable proportions of single chain and two-subunit forms of the TSHR are also observed on immunoprecipitation or immunoblotting of mammalian cell extracts (7-11). The two subunit TSHR involves a ligand binding, glycosylated A subunit and a membrane-associated B subunit linked by disulfide bonds (12). Because the TSHR is encoded by a single mRNA species (13-16), the A and B subunits must be formed by intramolecular cleavage, a process believed to involve a matrix mellatoprotease (17).

Recent evidence raised the surprising possibility that cleavage of the TSHR into A and B subunits does not occur at a single site as previously suspected, but at two sites (18), a feature unique to currently known members of the family of G protein-coupled receptors with seven membrane-spanning segments. In the present study, we sought to identify the amino acid residues related to each putative cleavage site, using a strategy involving mutagenesis of selected chimeric TSH-LH/CG receptors. The basis for this approach was that cleavage into A and B subunits does not occur in a chimeric receptor (TSH-LHR-6) in which the carboxyl terminal portion of the TSHR ectodomain (amino acid residues 261-418; arbitrary domains "D and E") is replaced with the corresponding region of the LH/CG receptor (19.20)(Fig. 31). On the other hand, cleavage still occurs when either domain D or domain E (residues 261-360 and 363-418; chimeric receptors TSH-LHR-4 and TSH-LHR-5, respectively) are substituted on an individual basis. These data, together with other information (18) suggested that cleavage sites 1 and 2 were in TSHR domains D and E, respectively.

Because elimination by mutagenesis of only one cleavage site would not be sufficient to abrogate receptor cleavage, we mutated regions in the vicinity of putative site 1 on a background of TSH-LHR-5 (21). Conversely, we mutated regions in the vicinity of putative site 2 on a background of TSH-LHR-4 (22). By this means, we were able to determine the structural significance of each mutation on receptor cleavage into subunits, as determined by $^{125}$I-TSH crosslinking to monolayers of intact, stably-transfected cells (23). As a general principle for mutagenesis, we replaced TSHR residues with the homologous residues of the non-cleaving LH/CG receptor. Residues common to both receptors were unaltered. In the case of a 50 amino acid region that is unique to the TSHR and lacking in the LH/CG receptor (14) (Fig. 31), homologous substitutions could not be performed so we introduced alanine residues in most instances. It is noteworthy that this 50 amino acid segment (estimated to be between residues 317-366) is very hydrophilic and can be deleted from the wild-type TSHR without loss of ligand binding and function (24) and without preventing cleavage into A and B subunits (5).

_The deglycosylated TSHR A subunit is - 35 kDa in size (18,25), which would place cleavage site 1 at approximately amino acid residue 335 (numbering includes a 21 residue signal peptide). On this basis, we initially targeted residues 330-338 for alanine scanning mutagenesis (Fig. 32A). None of these individual substitutions prevented cleavage of chimeric receptor TSH-LHR-5, as determined by release of the TSH-linked A subunit on disulfide bond reduction (Fig. 32B). Note that, consistent with previous TSH cross-linking studies to intact cells (4,5,18,26), only a portion of the TSHR on the cell surface cleave into two subunits, with TSH also binding to single polypeptide chain TSHR. Because of the possibility that a single amino acid residue may be insufficient to disrupt a cleavage site, we next performed multiple substitutions over a wider area between residues 324 and 352, also without abolishing cleavage. Finally, we mutagenized TSH-LHR-5 even further afield (residues 317-323), well upstream of the calculated cleavage site and up to the carboxyl-terminus of the D domain (residues 353-362), also without effect (Fig. 32B). _Mutagenesis of TSH-LHR-4 in the vicinity predicted to harbor putative cleavage site 2 (Fig. 33A) identified residues 367-375 (E1 subdomain) as being related to TSHR subunit formation (Fig. 33B). Subdivision of the E 1 region into two segments next identified residues 367-371 (Ela segment) to be associated with ectodomain cleavage. The subsequent substitution of individual alanines at residues 367. 368, 369 and 371 (residue 370 being conserved) did not abrogate cleavage, suggesting that multiple amino acids contributed to this event. Therefore, to refine further the cleavage-related residues, we created two overlapping mutants within the E1a segment. E1a1(GQE$_{367-369}$NET) and E1a2 (ELK$_{369-371}$T-Y)(Fig. 33A). Abrogation of cleavage in the former identified residues 367-369 to be involved in cleavage at putative site 2 (Fig. 33B).

The inability of single amino acid substitutions to prevent cleavage at putative site 2, as well the involvement of multiple amino acids in this cleavage site, was instructive in view of our inability to localize putative cleavage site 1 despite the generation of twenty one new receptors mutagenized in the anticipated region. Given the vast number of additional permutations that might be required to characterize site 1, further mutagenesis was daunting. Moreover, a number of possibilities existed:- (i) Cleavage site 1 could be upstream of amino acid residue 317. However, in this case the deglycosylated A subunit would be 33 kDa or less, smaller than observed by multiple investigators (11,18,25); (ii) The specificity of cleavage at site 1 was "relaxed" and, (iii) our hypothesis for two cleavage sites was wrong, despite the strong supporting evidence that we had obtained previously (18).

In order to exclude the possibility of only a single cleavage site, we introduced the E1a1 mutation

(GQE$_{367-369}$NET), known to prevent cleavage of TSH-LHR-4, into the wild-type TSHR. Thus, if this segment was the sole site related to TSHR cleavage, its introduction into the wild-type TSHR should prevent cleavage. Conversely, if cleavage still occurred in the wild-type TSHR harboring the GQE$_{367-369}$ NET mutation, there must be two cleavage sites in the TSHR ectodomain. Cross-linking of $^{125}$I-TSH to this new construct clearly indicated cleavage (Fig. 34), proving the existence of site 1. Taken together, the data suggest relaxed specificity in the amino acid motif for the upstream cleavage site (site 1).

Remarkably, the mutation (GQE$_{367-369}$NET) that abrogates cleavage at site 2 introduces a consensus sequence for an N-linked glycosylation site. A carbohydrate side-chain in this vicinity could, therefore, prevent ectodomain cleavage, for example by steric hindrance of a proteolytic enzyme. As mentioned above, the mutagenesis strategy used to investigate cleavage site 2 in the TSHR involved substitution into the TSHR of the corresponding segments of the non-cleaving LH/CG receptor. The GQE$_{367-369}$NET substitution in the TSHR transposes NET$_{291-293}$ from the LH/CG receptor, a motif that is glycosylated in the latter receptor (27). In order to determine the functional significance of the GQE$_{367-369}$NET substitution in TSH-LHR-4, we introduced into this chimeric receptor a motif (AAA) that would not be a potential glycosylation site (Fig. 33A). In contrast to the non-cleaving receptor, TSH-LHR-4-GQE$_{367-369}$AAA did cleave (Fig. 33B).

The evolutionary divergence of the TSHR into a receptor that cleaves into two subunits is unique and enigmatic. Unlike thrombin (28), TSH does not cleave its receptor; two subunit TSHR are present in transfected cells cultured in the absence of TSH (5,25). TSH binds to both cleaved and uncleaved forms of the TSHR. Moreover, TSH action does not require a cleaved receptor (24,29). One possibility is that TSHR receptor cleavage, including the release of a small polypeptide between cleavage sites 1 and 2, may be related to the very common occurrence of disease-causing autoantibodies, a phenomenon rarely, if ever, encountered with other members of the G protein-coupled receptor family.

In summary, data obtained from thirty three new TSHR variants stably expressed in CHO cells (i) provide proof for the existence of two cleavage sites in the TSHR, (ii) identify three amino acid residues in the TSHR that, when substituted with the homologous residues of the LH/CG receptor, prevent cleavage at the second, downstream cleavage site (site 2) and, (iii) reveal that cleavage or non-cleavage at this second site is related to N-linked glycosylation. To our knowledge, the presence or absence of glycosylation represents a novel mechanism by which two closely-related receptors have evolved into having a difference in subunit structure. Greater understanding of its structural features will also contribute to a better understanding of why the TSHR is a pivotal autoantigen in human autoimmune disease.

REFERENCES CITED IN EXAMPLE 6 AND NOTES

33. B. Gross, M. Misrahi, S. Sar, E. Milgrom, Biochem.Biophys.Res.Comm. 177, 679 (1991).

2. C. H. Tsai-Morris. E. Buczko, W. Wang, X.-Z. Xie, M. L. Dufau, J. Biol. Chem. 266, 11355 (1991).

3. Y. B. Koo, I, Ji, R. G. Slaughter, T. H. Ji, Endocrinology 128, 2297 (1991).

4. J. Furmaniak, et al, FEBS Letters 215, 316 (1987).

5. D. Russo, et al, Mol.Endocrinol. 5. 1607 (1991).

6. D. L. Segaloff, M. Ascoli, Endocr. Rev. 14. 324 (1993).

7. H. Loosfelt, et al, Proc. Natl. Acad. Sci. USA 895, 3765 (1992).

8. J. Hata, et al, Biochem.Biophys.Res.Comm. 164, 1268 (1989).

9. E. Potter, et al. BBRC 205, 361 (1994).

10. G. D. Chazenbalk, A. Kakinuma, J. C. Jaume, S. M. McLachlan, B. Rapoport, Endocrinology 137, 4586 (1996).

11. P. N. Graves, H. Vlase, Y. Bobovnikova, T. F. Davies, Endocrinology 137, 3915 (1996).

12. P. R. Buckland, C. R. Rickards, R. D. Howells, E. D. Jones, B. Rees Smith. FEBS Letters 145, 245 (1982).

13. M. Parmentier, et al, Science 246, 1620 (1989).

14. Y. Nagayama, K. D. Kaufman, P. Seto, B. Rapoport, Biochem.Biophys.Res.Comm. 165, 1184 (1989).

15. F. Libert, et al, Biochem.Biophys.Res.Comm. 165, 1250 (1989).

16. M. Misrahi. et al, Biochem.Biophys.Res.Comm. 166, 394 (1990).

17. J. Couet, et al, J. Biol. Chem. 271, 4545 (1996).

18. G. D. Chazenbalk, et al, Endocrinology (1997). in press

19. Y. Nagayama, et al, Proc. Natl. Acad. Sci. USA 88, 902 (1991).

20. D. Russo, Y. Nagayama, G. D. Chazenbalk, H. L. Wadsworth, B. Rapoport, Endocrinology 130, 2135 (1992).

21. In order to increase the level of receptor expression, we deleted the 5' and 3' untranslated regions of chimeric receptor TSH-LHR-5 (19) by transposing its Afl II - Spe I fragment (domains D and E)(Fig. 31) with the corresponding fragment in TSHR-5'3TR-NEO-ECE (30). Mutations in domain D of chimeric receptor TSH-LHR-5 were generated by PCR using overlapping primers and Pfu DNA polymerase (Stratagene, San Diego, CA). The nucleotide sequences of the PCR fragments were confirmed by the dideoxy nucleotide termination method (31). Plasmids were stably transfected with Lipofectin (Gibco-BRL, Gaithersburg, MD) into Chinese hamster ovary (CHO) cells cultured in

**EP 0 959 896 B1**

Ham's F-12 medium supplemented with 10% fetal calf serum (FCS) and standard antibiotics. Selection was with with 400 μg/ml G418 (GIBCO). Surviving clones (> 100 per 100 mm diameter culture dish) were pooled and propagated for further study.

22. For our initial series of mutations in domain E of chimeric receptor TSH-LHR-4, we used the E1, E2 and E3 mutations (Fig. 33A) previously constructed in the wild-type TSHR (32). These mutations were transposed into the cDNA for TSH-LHR-4 (Eco RV - Xba I fragment). Subsequent finer mutations in the E domain of TSH-LHR-4 were made by PCR using overlapping primers, with replacement of the Afl II - Spe I fragment. The nucleotide sequences of all PCR products were confirmed and stably-transfected CHO cell lines were generated as described for the domain D mutations.

23. Covalent crosslinking of bovine $^{125}$I-TSH to monolayers of intact cells was performed with disuccinimidyl suberate (DSS; 1 mM; Sigma), as described previously (10). Membrane preparations from the cells were subjected SDS-PAGE and autoradiography.

24. H. L. Wadsworth, G. D. Chazenbalk, Y. Nagayama, D. Russo, B. Rapoport, Science 249, 1423 (1990).
25. M. Misrahi, et al, Eur.J.Biochem. 222, 711 (1994).
26. G. D. Chazenbalk, B. Rapoport, J. Biol. Chem. 269, 32209 (1994).
27. D. P. Davis, T. G. Rozell, X. Liu, D. L. Segaloff, Mol.Endocrinol. 11.550 (1997).
28. T.-K. H. Vu, D. T. Hung, V. I. Wheaton, S. R. Coughlin, Cell 64, 1057 (1991).
29. G. D. Chazenbalk, S. M. McLachlan, Y. Nagayama, B. Rapoport, Biochem.Biophys.Res.Comm, 225, 479 (1996).
30. A. Kakinuma, G. Chazenbalk, S. Filetti, S. M. McLachlan, B. Rapoport. Endocrinology 137, 2664 (1996).
31. F. Sanger. S. Nicklen. A. R. Coulson, Proc. Natl. Acad Sci. USA 74, 5463 (1977).
32. Y. Nagayama, B. Rapoport, Endocrinology 131, 548 (1992).

**Claims**

1. A method of producing a form of the human thyrotropin receptor (TSHR) ectodomain, comprising

   a) culturing a host cell comprising a vector comprising a recombinant nucleic acid sequence encoding a secreted, soluble, complex carbohydrate-containing, endoglycosidase H resistant and endoglycosidase F sensitive form of the TSHR ectodomain which is C-terminal truncated at an amino acid residue selected from the group consisting of amino acid residues 261, 289 and 309 of the TSHR, wherein said culturing is under conditions allowing expression of said form of the TSHR ectodomain, and
   b) recovering said form of the TSHR ectodomain.

2. A method according to claim 1, wherein the vector is a plasmid.

3. A method according to claim 1 or 2, wherein the host cell is a mammalian cell.

4. A method according to claim 3, wherein the host cell is a Chinese Hamster Ovary cell.

5. A method according to claim 3 or 4, wherein the host cell is stably transfected.

**Patentansprüche**

1. Verfahren zur Herstellung einer Form der humanen Thyrotropinrezeptor (TSHR)-Ektodomäne, umfassend

   a) das Kultivieren einer Wirtszelle, die einen Vektor umfasst, der eine rekombinante Nukleinsäuresequenz umfasst, die für eine sezernierte, lösliche, komplexe Kohlenhydrate enthaltende, gegen Endoglycosidase H resistente und für Endoglycosidase F sensitive Form der TSHR-Ektodomäne kodiert, die C-terminal an einem Aminosäurerest verkürzt ist, der ausgewählt ist aus der Gruppe bestehend aus den Aminosäureresten 261, 289 und 309 des TSHR, wobei das Kultivieren unter Bedingungen erfolgt, die die Expression der genannten Form der TSHR-Ektodomäne gestatten, und
   b) die Gewinnung der genannten Form der TSHR-Ektodomäne.

2. Verfahren nach Anspruch 1, wobei der Vektor ein Plasmid ist.

3. Verfahren nach Anspruch 1 oder 2, wobei die Wirtszelle eine Säugerzelle ist.

**4.** Verfahren nach Anspruch 3, wobei die Wirtszelle eine Ovarialzelle des chinesischen Hamsters ist.

**5.** Verfahren nach Anspruch 3 oder 4, wobei die Wirtszelle stabil transfiziert ist.

**Revendications**

**1.** Procédé de production d'une forme de l'ectodomaine du récepteur de la thyrotropine humaine (TSHR), comprenant :

a) la mise en culture d'une cellule hôte comprenant un vecteur contenant une séquence d'acide nucléique recombinant codant pour une forme de l'ectodomaine TSHR sécrétée, soluble, contenant des glucides complexes, résistante à l'endoglycosidase H et sensible à l'endoglycosidase F, tronquée en terminaison C au niveau d'un résidu d'acide aminé choisi dans le groupe constitué des résidus d'acides aminés 261, 289 et 309 du TSHR, ladite mise en culture se faisant dans des conditions qui permettent l'expression de ladite forme de l'ectodomaine TSHR, et
b) la récupération de ladite forme de l'ectodomaine TSHR.

**2.** Procédé selon la revendication 1, dans lequel le vecteur est un plasmide.

**3.** Procédé selon la revendication 1 ou 2, dans lequel la cellule hôte est une cellule de mammifère.

**4.** Procédé selon la revendication 3, dans lequel la cellule hôte est une cellule d'ovaire de hamster chinois.

**5.** Procédé selon la revendication 3 ou 4, dans lequel la cellule hôte est transfectée de manière stable.

FIG.1A

FIG.1B

FIG.2

FIG.3

FIG.4

FIG.5A

FIG.5B

TSHR-10,000

CHASE

3 HOURS    16 HOURS

Da

15 —

83 —

63 —

48 —

38 —

34 —

ENDO F   CON   ENDO H   ENDO F   CON   ENDO H

← SINGLE SUBUNIT (Complex)
← SINGLE SUBUNIT (High mannose)

← A SUBUNIT (Complex)

← A SUBUNIT (Deglycosylated)

FIG.6A

TSHR    CHO    TSHR    CHO
10,000          10,000

kDa

→ 115 —

83 —

→ 63 —

48 —

A10    A10    T3-365    TPO    TPO

mAb

FIG.6B

EP 0 959 896 B1

B SUBUNIT

mAb    T3-495    T3-365

TSHR 10,000 | TSHR 10,000

kDa
115-
83-
63-
48-
SUBUNIT  38-
34-

ENDO F    CON    ENDO H    ENDO F    CON    ENDO H

**FIG.7A**

B SUBUNIT

mAb    T3-495    T3-365

CHO  TSHR    CHO  TSHR
10,000        10,000

kDa
115-
83-
63-
48-
B SUBUNIT  38-
34-

**FIG.7B**

EP 0 959 896 B1

FIG.7C

FIG.7D

EP 0 959 896 B1

FIG.8

EP 0 959 896 B1

FIG.9A

**FIG.9B**

kDa    TSHRmyc    TSHR 10,000

115 —
83 —

A SUBUNIT → 63 —
(Glycosylated)

48 —

A SUBUNIT → 38 —
(Deglycosylated) 34 —

CON | ENDO F | CON | ENDO F | CON | ENDO F

mAb   A10    9E10    A10

**FIG.9C**

kDa   TSHRmyc   HEK

115 —
83 —

63 —

48 —

38 —
34 —

mAb   TPO    A10

EP 0 959 896 B1

FIG.10

FIG.11

FIG.12

FIG.13

FIG.14A

FIG.14B

FIG.15

FIG.16

FIG.17

TSHR ECTODOMAIN VARIANTS

FIG.18

FIG.19

# TSH RECEPTOR ECTODOMAIN 261

Conditioned Medium

Wheat Germ Agglutinin     Eluate

Flow-through

Bandeiraea Simplicifolia     Eluate

Flow-through

Concanavalin A     Eluate

Flow-through

1    1/5    1/25
Dilution

# FIG.20

FIG.21

ENDO    H  -  F    H  -  F    H  -  F

Bandeiraea Simplificifolia    Concanavalin A    Wheat Germ Agglutinin

← 50 kDa

← 30 kDa

TSH RECEPTOR ECTODOMAIN VARIANT

FIG.22

EP 0 959 896 B1

Concanavalin A

Ni-Chelate

kDa

115 —

83 —

63 —

48 —

38 —

34 —

**FIG.23A**

Concanavalin A
+
Ni·Chelate

kDa

115 —

83 —

63 —

48 —                    ←  TSHR-261
                           Glycosylated

38 —   ←  Endoglycosidase F

34 —   ←  TSHR-261
           Deglycosylated

+    +    -    Endoglycosidase F

-    +    +    TSHR-261

**FIG.23B**

FIG.24

FIG.25

125I-TSH BINDING (% OF MAXIMUM)

GRAVES' PATIENTS

NORMAL INDIVIDUALS

− TSHR-261

+ TSHR-261 (50 ng/TUBE)

EP 0 959 896 B1

FIG.26A

FIG.26B

FIG.26C

FIG.26D

FIG.27A

FIG.27B

FIG.27C

FIG.28A

FIG.28B

FIG.28C

FIG.28D

FIG.29A

FIG.29B

FIG.29C

FIG.29D

FIG.30A

FIG.30B

FIG.30C

FIG.30D

FIG.30E

FIG.30F

FIG.30G

FIG.30H

FIG.31

EP 0 959 896 B1

------ LH/CG RECEPTOR

317   321 324 327 330   334     339 342 345   349   353   358   363

ALNSPLHQEYEENLGDSIVGYKEKSKFQDTHNNAHYYVFFEEQEDEIIGF   TSH RECEPTOR

A
A
A
A
A
A
A
A
A
A

GAAA
AAA
AAA
AAA
AAAA
AAA
AAA
AAA
AAAA
AAAA
AAAAQ
QAQNQ

FIG.32A

FIG.32B

FIG.32C

EP 0 959 896 B1

NETLYSAIFEENELSGWDYDYGFC–SPKTLQCAPEPDAFNPCEDIMGYAFLP     LH/CG RECEPTOR

367          377          388                                    418

GQELKNPQEETLQAFDSHYDYTICGDSEDMVCTPKSDEFNPCEDIMGYKFLR     TSH RECEPTOR

NET–YSAIF                                                        E1
                                            MEMBRANE              E2
–ENELSGWD– –|                                                    E3
            GF–SPKTL

NET–Y                                                            E1a
    SAIF                                                         E1b

A                                                               $G_{367}A$

 A                                                              $G_{368}A$

  A                                                             $G_{369}A$

   A                                                            $G_{371}A$

NET                                                             E1a1–NET  (GQE $_{267-268}$ NET)

 T–Y                                                            E1a2      (ELK $_{269-271}$ T–Y)

AAA                                                             E1a1–AAA  (GQE $_{267-268}$ AAA)

FIG.33A

EP 0 959 896 B1

FIG.33B

$^{125}$I-TSH cross-linked to:-

← Single chain TSH-LHR-4 holoreceptor

← A subunit of cleaved receptor

E1 E2 E3

| E1 a | E1 b | E1 a2 | 367 | 368 | 369 | 371 | Amino acids substituted |

EP 0 959 896 B1

FIG.33C

Wild-type TSHR

Single chain
← holoreceptor

← A subunit of
cleaved receptor

GQE₃₆₇₋₃₆₉NET
No mutation

FIG.34

## REFERENCES CITED IN THE DESCRIPTION

### Patent documents cited in the description

- US 4683202 A
- US 4800159 A
- US 4683195 A

### Non-patent literature cited in the description

- **Hunt et al.** *Endocrine,* 1995, vol. 3, 233-240
- **Hunt et al.** *Experimental and Clinical Endocrinology,* 1992, vol. 100, 22-27
- **Sambrook, J. et al.** Molecular Cloning: A Laboratory Manual. Cold Spring Harbor Laboratory Press, 1989
- Handbook of Molecular and Cellular Methods in Biology and Medicine. CRC Press, 1995
- Directed Mutagenesis: A Practical Approach. IRL Press, 1991
- **Jones, J.** Amino Acid and Peptide Synthesis. Oxford Science Publications, 1992
- **Austen, B. M. ; Westwood, O. M. R.** Protein Targeting and Secretion. IRL Press, 1991
- Remington's Pharmaceutical Sciences
- **Goodman ; Gilman's.** The Pharmacological Basis of Therapeutics. MacMillan Publishing Co, 1985
- Remington's Pharmaceutical Sciences. Mack Publishing Co, 1990
- **Ansel, H. C. ; Popovich, N. G.** Pharmaceutical Dosage Forms and Drug Delivery Systems. Lea & Febiger, Pub, 1990
- Protein purification: Principles and Practice. Springer Verlag, 1982
- New Methods in Peptide Mapping for the Characterization of Proteins. CRC Press, 1996
- **Fauchere J.** *Adv. Drug Res.,* 1986, vol. 15, 29
- **Evans et al.** *J. Med. Chem.,* 1987, vol. 30, 1229
- Nucleic Acids in Chemistry and Biology. IRL Press, 1990
- PCR Technology: Principles and Applications for DNA Amplification. Stockton Press, 1989
- **Lehninger, A.** Biochemistry. Worth Publishers, 1975
- **Sell, S.** Immunology, Immunopathology & Immunity. Appleton & Lange, Publ, 1996
- **Male, D. et al.** Advanced Immunology. Times Mirror Int'1 Publishers Ltd., Publ, 1996
- **Stites, D. P. ; Terr, A. I.** Basic and Clinical Immunology. Appleton & Lange, Publ, 1991
- **Abbas, A. K. et al.** Cellular and Molecular Immunology. W. B. Saunders Co., Publ, 1991
- **Reels Smith, B ; McLachlan, SM ; Furmaniak, J.** Autoantibodies to the thyrotropin receptor. *Endocr Rev.,* 1988, vol. 9, 106-121
- **Orgiazzi, J ; Williams. DE ; Chopra, IJ ; Solomon, DH.** Human thyroid adenyl cyclase-stimulating activity in immunoglobulin G of patients with Graves' disease. *J Clin Endocrinol Metab.,* 1976, vol. 42, 341-354
- **Endo, K ; Kasagi, K ; Konishi, J ; Ikekubo. K. ; Tatsuyb, O ; Takeda. Y ; Mori, T ; Torizuka, K.** Detection and properties of TSH-binding inhibitor immunoglobulins in patients with Graves' disease and Hashimoto's thyroiditis. *J Clin Endocrinol Metab.,* 1978, vol. 46, 734-739
- **Matsuura, N ; Yamada, Y ; Nohara, Y ; Konishi, J ; Kasagi, K ; Endo, K ; Kojima, K ; Wataya. K.** Familial neonatal transient hypothyroidism due to maternal TSH-binding inhibitor immunoglobulins. *N Engl J Med.,* 1980, vol. 303, 738-741
- **McKenzie, JM ; Zakarija, M.** Clinical Review 3: The clinical use of thyrotropin receptor antibody measurements. *J Clin Endocrinol Metab.,* 1989, vol. 69, 1093-1096
- **Shewring, GA ; Rees Smith, B.** An improved radioreceptor assay for TSH receptor antibodies. *Clin.Endocrinol.,* 1982, vol. 17, 409-417
- **Nagayama, Y ; Kaufman, KD ; Seto, P ; Rapoport. B.** Molecular cloning, sequence and functional expression of the cDNA for the human thyrotropin receptor. *Biochem.Biophys.Res.Comm.,* 1989, vol. 165, 1184-1190
- **Perret, J ; Ludgate, M ; Libert, F ; Gerard, C ; Dumont. JE ; Vassan, G ; Parmentier, M.** Stable expression of the human TSH receptors in CHO cells and characterization of differentially expressing clones. *Biochem.Biophys.Res.Comm.,* 1990, vol. 171, 1044

• **Harfst. E ; Johnstone, AP ; Gout, I ; Taylor, AH ; Walerlield, MD ; Nussey. SS.** The use of the amplifiable high-expression vector pEE14 to study the interactions of autoantibodies with recombinant human thyrotropin receptor. *Molec.Cell.I.Endocrinol.,* 1992, vol. 83, 117.123

• **Matsuba, T ; Yamada. M ; Suzuki, H ; Kanai, A ; Isozaki, O ; Yoshida, T ; Tsushima, T ; Yasukawa, K.** Expression of recombinant human thyrotropin receptor in myeloma cells. *J.Biochem.,* 1995, vol. 118, 265-270

• **Murakami, M ; Miyashita, K ; Kakizaki, S ; Saito. S. ; Yarnada, M ; Iriuchijima, T ; Takeuchi. T ; Mori, M.** Clinical usefulness of thyroid-stimulating antiobody measurement using Chinese hamster ovary cells expressing human thyrotropin receptors. *Eur J Endocrinol.,* 1995, vol. 133, 80-86

• **Kim, WB ; Cho, BY ; Park, HY ; Lee, HK ; Kohn, LD ; Tahara, K ; Koh, C-S.** Epitopes for thyroid-stimulating antibodies in Graves' sera: A possible link of heterogeneity to differences in response to antithyroid drug treatment. *J Clin Endocrinol Metab.,* 1996, vol. 81, 1758-1767

• **Takai, O ; Desai. RK ; Seetharatnaiah, GS ; Jones, CA ; Allaway, GP ; Akamizu, T ; Kohn, LD ; Prabhakar, BS.** Prokaryotic expression of the thyrotropin receptor and identification of an immunogenic region of the protein using synthetic peptides. *Biochem.Biophys.Ites.Comm.,* 1991, vol. 179, 319-326

• **Loosfelt, H ; Pichon, C ; Jolivet A ; Misrahi, M ; Caillou, B ; Jamous. M ; Vannier. B ; Milgrom, E.** Two-subunit structure of the human thyrotropin receptor. *Proc Natl Acad Sci USA.,* 1992, vol. 895, 3765-3769

• **Harfst. E ; Johnstone. AP ; Nussey, SS.** Characterization of the extracellular region of the human thyrotropin receptor expressed as a recombinant protein. *J Mol Endocrinol.,* 1992, vol. 9, 227-236

• **Huang, GC ; Collison, KS ; McGregor. AM ; Banga, JP.** Expression of a human thyrotropin receptor fragment in Escherichia coli and its interaction with the hormone and autoantibodies from patients with Graves' disease. *J Mol Endocrinol.,* 1992, vol. 8, 137-144

• **Costagliola, S ; Alcalde, L ; Ruf, J ; Vassart, G ; Ludgate, M.** Overexpression of the extracellular domain of the thyrotrophin receptor in bacteria, production of thyrotrophin-binding inhibiting immunoglobulins. *J Mol Endocrinol.,* 1994, vol. 13, 11-21

• **Graves, PN ; Vlase, H ; Davies, TF.** Folding of the recombinant human thyrotropin (TSH) receptor extracellular domain: identification of folded monomeric and tetrameric complexes that bind TSH receptor autoantibodies. *Endocrinology,* 1995, vol. 136, 521-527

• **Huang, GC. ; Page, M ; Nicholson, LB ; Collison, KS ; McGregor, AM. ; Banga. JP.** The thyrotropin hormone receptor of Graves' disease: overexpression of the extracellular domain in insect cells using recombinant baculovirus, immunoaffinity purification and analysis of autoantibody binding. *J Mol Endocrinol.,* 1993, vol. 10, 127-142

• **Seetharamaiah, GS. ; Desai, RK ; Dallas. JS ; Tahara, K ; Kohn, LD ; Prabhakar, BS.** Induction of TSH binding inhibitory immunoglobulins with the extracellular domain of human thyrotropin receptor produced using baculovims expression system. *Autoimmunity,* 1993, vol. 14, 315-320

• **Vlase, H ; Graves, P ; Magnusson, RP ; Davies. TF.** Human autoantibodies to the thyrotropin receptor: recognition of linear, folded, and glycosylated recombinant extracellular domain. *J Clin Endocrinol Metab.,* 1995, vol. 80, 46-53

• **Morgenthaler, NG ; Tremble, J ; Huang, G ; Scherbaum. WA ; McGregor, AM ; Banga, JP.** Binding of antithyrotropin receptor autoantibodies in Graves' disease serum to nascent, in vitro translated thyrotropin receptor: Ability to map epitopes recognized by antibodies. *J Clin Endocrinol Metab.,* 1996, vol. 81, 700-706

• **Filetti, S ; Foti, D ; Costante. G ; Rapoport, B.** Recombinant human TSH receptor in a radioreceptor assay for the measurement of TSH receptor autontibodies. *J Clin Endocrinol Metab.,* 1991, vol. 72, 1096-1101

• **Libert, F. ; Parmentier, M. ; Maenhaut, C ; Lefort. A ; Gerard, C ; Ferret, J ; Van Sande. J ; Dumont, JE ; Vassart, G.** Molecular cloning of a dog thyrotropin (TSH) receptor variant. *Molec.Cell.Endocrinol.,* 1990, vol. 68, R15-RI7

• **Costagliola, S ; Swillens, S ; Niccoli, P ; Dumont, JE ; Vassart, G ; Ludgate, M.** Binding assay for thyrotropin receptor autoantibodies using the recombinant receptor protein. *J Clin Endocrinol Metab.,* 1992, vol. 75, 1540-1544

• **Kakinuma, A ; Chazenbalk. G ; Filetti, S ; McLachlan, SM ; Rapoport, B.** Both the 5' and 3' non-coding regions of the thyrotropin receptor messenger RNA influence the level of receptor protein expression in transfected mammalian cells. *Endocrinology,* 1996, vol. 137, 2664-2669

• **Chazenbalk. GD ; Kakinuma, A ; Jaume, JC ; McLachlan. SM ; Rapoport, B.** Evidence for negative cooperativity among human thyrotropin receptors overexpressed in mammalian cells. *Endocrinology,* 1996, vol. 137, 4586-4591

• **Goldfine. ID ; Amir, SM ; Petersen, AW ; Ingbar, SH.** Preparation of biologically active 125I-TSH. *Endocrinology,* 1974, vol. 95, 1228-1233

• **Tramontano, D ; Ingbar, SH.** Properties and regulation of the thyrotropin receptor in the FRTL5 rat thyroid cell line. *Endocrinology,* 1986, vol. 118, 1945-1951

- **Rees Smith, B ; Hall, R.** Measurement of thyrotropin receptor antibodies. *Methods in Enzymology,* 1981, vol. 74, 405-420
- **Hinds. WE ; Takai, N ; Rapoport, B ; Filetti, S ; Clark, OH.** Thyroid-stimulating immunoglobulin bioassay using cultured human thyroid cells. *J Clin Endocrinol Metab.,* 1981, vol. 52, 1204-1210
- **Rapoport. B. ; Greenspan. FS ; Filetti, S ; Pepitone, M.** Clinical experience with a human thyroid cell bioassay for thyroid-stimulating immunoglobulin. *J Clin Endocrinol Metab.,* 1984, vol. 58, 332-338
- **Kasagi, K ; Konishi, J ; Iida, Y ; Ikekubo, K ; Mori, T ; Kuma, K ; Torizuka, K.** A new in vitro assay for human thyroid stimulator using cultured thyroid cells: Effect of sodium chloride on adenosine 3'.5'-monophosphate increase. *J Clin Endocrinol Metab.,* 1982, vol. 54, 108-114
- **Pierce, JG ; Parsons, If.** Glycoprotein hormones: structure and function. *Ann Rev Biochem.,* 1981, vol. 50, 465-495
- **Vitti, P ; Elisei, R ; Tonacchera, M ; Chiovato, L ; Mancusi, F ; Rago, T ; Mammoli, C ; Ludgate, M ; Vassart, G ; Pinchera, A.** Detection of thyroid-stimulating antibody using Chinese hamster ovary cells transected with cloned human thyrotropin receptor. *J.Clin.Endo.Metab.,* 1993, vol. 76, 499-503
- **Jaume, JC ; Kakinuma, A ; Chazenbalk, GD ; Rapoport, B ; McLachlan. SM.** TSH receptor autoantibodies in serum are present at much lower concentrations than thyroid peroxidase autoantibodies: Analysis by flow cytometry. *J Clin Endocrinol Metab.,* 1997
- **Libert, F ; Lefort, A ; Gerard, C ; Parmentier, M ; Ferret. J ; Ludgate, M ; Dumont JE ; Vassart, G.** Cloning, sequencing and expression of the human thyrotropin (TSH) receptor: Evidence for binding of autoantibodies. *Biochem.Biophys.Res.Comm.,* 1989, vol. 165, 1250-1255
- **Misrahi, M ; Loosfelt. H ; Atger, M ; Sar, S ; Guiochon-Mantel, A ; Milgrom, E.** Cloning, sequencing and expression of human TSH receptor. *Biochem.Biophys.Res.Comm.,* 1990, vol. 166, 394-403
- **Endo, T ; Ohmori, M ; Ikeda, M ; Anzai. E ; Onaya, T.** Heterogeneous responses of recombinant human thyrotropin receptor to immunoglobulins from patients with Graves' disease. *BBRC,* 1992, vol. 186, 1391-1396
- **Beall, GN ; Kruger, SR.** Binding of 1251-human TSH by gamma globulins of sera containing thyroid-stimulating immunoglobulin (TSI). *Life Sciences,* 1983, vol. 32, 77-83
- **Szkudlinski, MW ; Teh, NG ; Grossmann, M ; Tropea, JE ; Weintraub, BD.** Engineering human glycoprotein hormone superactive analogues. *Nature Biotech.,* 1996, vol. 14, 1257-1263
- **Buckland PR ; Rickards CR ; Howells RD ; Jones ED ; Rees Smith B.** Photo-affinity labelling of the thyrotropin receptor. *FEBS Letters,* 1982, vol. 145, 245-249
- **Parmentier M ; Libert F ; Maenhaut C ; Lefort A ; Gerard C ; Perret J ; Van Sande J ; Dumont JE ; Vassart G.** Molecular cloning of the thyrotropin receptor. *Science,* 1989, vol. 246, 1620-1622
- **Nagayama Y ; Kaufman KD ; Seto P ; Rapoport B.** Molecular cloning, sequence and functional expression of the cDNA for the human thyrotropin receptor. *Biochem.Biophys.Res.Comm.,* 1989, vol. 165, 1184-1190
- **Libert F ; Lefort A ; Gerard C ; Parmentier M ; Perret J ; Ludgate M ; Dumont JE ; Vassart G.** Cloning, sequencing and expression of the human thyrouopin (TSH) receptor: Evidence for binding of autoantibodies. *Biochem.Biophys.Res.Comm.,* 1989, vol. 165, 1250-1255
- **Misrahi M ; Loosfelt H ; Atger M ; Sar S ; Guiochon-Mantel A ; Milgrom E.** Cloning, sequencing and expression of human TSH receptor. *Biochem.Biophys.Res.Comm.,* 1990, vol. 166, 394-403
- **Furmaniak J ; Hashirn FA ; Buckland PR ; Petersen VB ; Beever K ; Howells RD ; Rees Smith B.** Photoaffinity labelling of the TSH receptor on FRTL5 cells. *FEBS Letters,* 1987, vol. 215, 316-322
- **Russo D. ; Chazenbalk GD ; Nagayama Y ; Wadsworth HL ; Seto P ; Rapoport B.** A new structural model for the thyrotropin receptor as determined by covalent crosslinking of thyrotropin to the recombinant receptor in intact cells: Evidence for a single polypeptide chain. *Mol.Endocrinol.,* 1991, vol. 5, 1607-1612
- **Van Sande J ; Massart C ; Costagliola S ; Allgeier A ; Cetani F ; Vassart G ; Dumont JE.** Specific activation of the thyrotropin receptor by trypsin. *Mol.& Cellular Endo,* 1996, vol. 119, 161-168
- **Chazenbalk GD ; McLachlan SM ; Nagayama Y ; Rapoport B.** Is receptor cleavage into two subunits necessary for thyrotropin action?. *Biochem.Biophys.Res.Comm.,* 1996, vol. 225, 479-484
- **Russo D ; Nagayama Y ; Chazenbalk GD ; Wadsworth HL ; Rapoport B.** Role of amino acids 261-418 in proteolytic cleavage of the extracellular region of the human thyrotropin receptor. *Endocrinology,* 1992, vol. 130, 2135-2138
- **Ban T ; Kosugi S ; Kohn LD.** Specific antibody to the thyrotropin receptor identifies multiple receptor forms in membrane of cells transfected with wild-type receptor complementary deoxyribonucleic acid: Characterization of their relevance to receptor synthesis, processing, structure, and function. *Endocrinology,* 1992, vol. 131, 815-829

- **Chazenbalk GD ; Rapoport B.** Cleavage of the thyrotropin receptor does not occur at a classical subtilisin-related proprotein covertase endoproteolytic site. *J Biol Chem,* 1994, vol. 269, 32209-32213
- **Couet J ; Sakhavut S ; Jolivet A ; Vu Hai M-T ; Milgrom E ; Misrahi M.** Shedding of human thyrotropin receptor ectodomain: Involvement of a matrix metalloprotease. *J Biol Chem,* 1996, vol. 271, 4545-4552
- **Chazenbalk GD ; Kakinuma A ; Jaume JC ; McLachlan SM ; Rapoport B.** Evidence for negative cooperativity among human thyrotropin receptors overexpressed in mammalian cells. *Endocrinology,* 1996, vol. 137, 4586-4591
- **Tanaka K ; Nagayama Y ; Yamasaki H ; Hayashi H ; Samba H ; Yamashita S ; Niwa M.** Epitope-tagging of a functional thyrotropin receptor: detection of the native receptor on intact cells. *Biochem.Biophys.Res.Comm.,* 1996, vol. 228, 21-28
- **Kakinuma A ; Chazenbalk G ; Filetti S ; McLachlan SM ; Rapoport B.** Both the 5' and 3' non-coding regions of the thyrotropin receptor messenger RNA influence the level of receptor protein expression in transfected mammalian cells. *Endocrinology,* 1996, vol. 137, 2664-2669
- **Nicholson LB ; Vlase H ; Graves P ; Nilsson M ; Molne J ; Huang GC ; Morgenthaler NG ; Davies TF ; McGregor AM ; Banga JP.** Monoclonal antibodies to the human thyrotropin receptor: Epitope mapping and binding to the native receptor on the basolateral plasma membrane of thyroid follicular cells. *J Mol Endocrinol,* 1996, vol. 16, 159-170
- **Laemmli UK.** Cleavage of structural proteins during the assembly of the head of bacteriophage T4. *Nature,* 1970, vol. 227, 680-685
- **Rapoport B ; McLachlan SM ; Kakinuma A ; Chazenbalk GD.** Critical relationship between autoantibody recognition and TSH receptor maturation as reflected in the acquisition of mature carbohydrate. *J Clin Endocrinol Metab,* 1996, vol. 81, 2525-2533
- **Goldfine ID ; Amir SM ; Petersen AW ; Ingbar SH.** Preparation of biologically active 125I-TSH. *Endocrinology,* 1974, vol. 95, 1228-1233
- **Loosfelt H ; Pichon C ; Jolivet A ; Misrahi M ; Caillou B ; Jamous M ; Vannier B ; Milgrom E.** Two-subunit structure of the human thyrotropin receptor. *Proc Natl Acad Sci USA,* 1992, vol. 895, 3765-3769
- **Misrahi M. ; Ghinea N ; Sar S ; Saunier B ; Jolivet A ; Loosfelt H ; Cerutti M ; Devauchelle G ; Milgrom E.** Processing of the precursors of the human thyroid-stimulating hormone receptor in various eukaryotic cells (human thyrocytes, transfected L cells and bacul ovirus- infected insect cells). *Eur.J.Biochem.,* 1994, vol. 222, 711-719

- **Graves PN ; Vlase H ; Bobovnikova Y ; Davies TF.** Multimeric complex formation by the thyrotropin receptor in solubilized thyroid membranes. *Endocrinology,* 1996, vol. 137, 3915-3920
- **Nagayama Y ; Wadsworth HL. ; Chazenbalk GD ; Russo D ; Seto P ; Rapoport B.** Thyrotropin-luteinizing hormonelchorionic gonadotropin receptor extracellular domain chimeras as probes for TSH receptor function. *Proc Natl Acad Sci USA,* 1991, vol. 88, 902-905
- **Wadsworth HL ; Chazenbalk GD ; Nagayama Y ; Russo D ; Rapoport B.** An insertion in the human thymtropin receptor critical for high affinity hormone binding. *Science,* 1990, vol. 249, 1423-1425
- **Kosugi S ; Akamizu T ; Takai O ; Prabhakar BS ; Kohn LD.** The extracellular domain of the TSH receptor has an immunogenic epitope reactive with Graves' IgG but unrelated to receptor function as well as determinants having different roles for high affinity TSH binding and the activity of thyroid-stimulating autoantibodies. *Thyroid,* 1991, vol. 1, 321-330
- **Kosugi S ; Ban T ; Akamizu T ; Kohn LD.** Site-directed mutagenesis of a portion of the extracellular domain of the rat thyrotropin receptor important in autoimmune thyroid disease and nonhomologous with gonadotropin receptors. *J Biol Chem,* 1991, vol. 266, 19413-19418
- **Kobe B ; Deisenhofer J.** Crystal structure of porcine ribonuclease inhibitor, a protein with leucine-rich repeats. *Nature,* 1993, vol. 366, 751-756
- **Kajava AV ; Vassart G ; Wodak SJ.** Modeling of the three-dimensional structure of proteins with the typical leucine-rich repeats. *Structure,* 1995, vol. 3, 867-877
- **Rees Smith B. ; McLachlan SM ; Furmaniak J.** Autoantibodies to the thyrotropin receptor. *Endocr Rev,* 1988, vol. 9, 106-121
- **Nagayama Y ; Rapoport B.** The thyrotropin receptor twenty five years after its discovery: new insights following its molecular cloning. *Mol.Endocrinol.,* 1992, vol. 6, 145-156
- **Vassart G. ; Dumont JE.** The thyrotropin receptor and the regulation of thyrocyte function and growth. *Endocr Rev,* 1992, vol. 13, 596-611
- **Takai O ; Desai RK ; Seetharamaiah GS ; Jones CA ; Allaway GP ; Akamizu T ; Kohn LD ; Prabhakar BS.** Prokaryotic expression of the thyrotropin receptor and identification of an immunogenic region of the protein using synthetic peptides. *Biochem.Biophys.Res.Comm.,* 1991, vol. 179, 319-326
- **Harfst E ; Johnstone AP ; Nussey SS.** Characterization of the extracellular region of the human thyrotropin receptor expressed as a recombinant protein. *J Mol Endocrinol,* 1992, vol. 9, 227-236

- **Huang GC ; Collison KS ; McGregor AM ; Banga JP.** Expression of a human thyrotropin receptor fragment in Escherichia coli and its interaction with the hormone and autoantibodies from patients with Graves' disease. *J Mol Endocrinol,* 1992, vol. 8, 137-144
- **Costagliola S. ; Alcalde L. ; Ruf J ; Vassart G ; Ludgate M.** Overexpression of the extracellular domain of the thyrotrophin receptor in bacteria; production of thyrotrophin-binding inhibiting immunoglobulins. *J Mol Endocrinol,* 1994, vol. 13, 11-21
- **Graves PN ; Vlase H ; Davies TF.** Folding of the recombinant human thyrotropin (TSH) receptor extracellular domain: identification of folded monomeric and tetrameric complexes that bind TSH receptor autoantibodies. *Endocrinology,* 1995, vol. 136, 521-527
- **Huang GC ; Page MJ ; Nicholson LB ; Collison KS ; McGregor AM ; Banga JP.** The thyrotropin hormone receptor of Graves' disease: overexpression of the extracellular domain in insect cells using recombinant baculovirus, immunoaffinity purification and analysis of autoantibody binding. *J Mol Endocrinol,* 1993, vol. 10, 127-142
- **Seetharamaiah GS ; Desai RK ; Dallas JS ; Tahara K ; Kohn LD ; Prabhakar BS.** Induction of TSH binding inhibitory immunoglobulins with the extracellular domain of human thyrotropin receptor produced using baculovirus expression system. *Autoimmunity,* 1993, vol. 14, 315-320
- **Misrahi M ; Ghinea N ; Sar S ; Saunier B ; Jolivet A ; Loosfelt H ; Cerutti M ; Devauchelle G ; Milgrom E.** Processing of the precursors of the human thyroid-stimulating hormone receptor in various eukaryotic cells (human thyrocytes, transfected L cells and baculovirus-infected insect cells). *Eur.J.Biochem.,* 1994, vol. 222, 711-719
- **Chazenbalk GD ; Rapoport B.** Expression of the extracellular region of the thyrotropin receptor in a baculovirus vector using a promoter active earlier than the polyhedrin promoter: Implications for the expression of functional, highly glycosylated proteins. *J Biol Chem,* 1995, vol. 270, 1543-1549
- **Vlase H ; Graves P ; Magnusson RP ; Davies TF.** Human autoantibodies to the thyrotropin receptor: recognition of linear, folded, and glycosylated recombinant extracellular domain. *J Clin Endocrinol Metab,* 1995, vol. 80, 46-53
- **Fan J-L ; Seetharamaiah GS ; Desai RK ; Dallas JS ; Wagle NM ; Prabhakar BS.** Analysis of autoantibody reactivity in patients with Graves' disease using recombinant extracellular domain of the human thyrotropin receptor and synthetic peptides. *Autoimmunity,* 1993, vol. 15, 285-291
- **Morris JC ; Jiang N-S ; Hay ID ; Charlesworth MC ; McCormick DJ ; Ryan RJ.** The effects of synthetic alpha-subunit peptides on thyroid-stimulating immunoglobulin activity. *J Clin Endocrinol Metab,* 1988, vol. 67, 707-712
- **Atassi MZ. ; Manshouri T ; Sakata S.** Localization and synthesis of the hormone-binding regions of the human thyrotropin receptor. *Proc Natl Acad Sci USA,* 1991, vol. 88, 3613-3617
- **Morris JC ; Bergert ER ; McCormick DJ.** Structure-function studies of the human thyrotropin receptor. Inhibition of binding of labeled thyrotropin (TSH) by synthetic human TSH receptor peptides. *J Biol Chem,* 1993, vol. 268, 10900-10905
- **Bryant WP. ; Bergert ER ; Morris JC.** Delineation of amino acid residues within hTSHr 256-275 that participate in hormone binding. *J.Biol Chem,* 1994, vol. 269, 30935-30938
- **Morris JC ; Gibson JL ; Haas EJ ; Bergert ER ; Dallas JS ; Prabhakar BS.** Identification of epitopes and affinity purification of thyroid stimulating auto-antibodies using synthetic human TSH receptor peptides. *Autoimmunity,* 1994, vol. 17, 287-299
- **Morgenthaler NG ; Tremble J. ; Huang G ; Scherbaum WA ; McGregor AM ; Banga JP.** Binding of antithyrotropin receptor autoantibodies in Graves' disease serum to nascent, in vitro translated thyrotropin receptor: Ability to map epitopes recognized by antibodies. *J Clin Endocrinol Metab,* 1996, vol. 81, 700-706
- **Harfst E ; Johnstone AP ; Gout I ; Taylor AH ; Waterfield MD ; Nussey SS.** The use of the amplifiable high-expression vector pEE14 to study the interactions of autoantibodies with recombinant human thyrotropin receptor. *Molec.Cell.Endocrinol.,* 1992, vol. 83, 117-123
- **Kaufman KD ; Foti D ; Seto P ; Rapoport B.** Overexpression of an immunologically-intact, secreted form of human thyroid peroxidase in eukaryotic cells. *Molec.Cell-Endocrinol.,* 1991, vol. 78, 107-114
- **Rapoport B. ; McLachlan SM ; Kakinuma A ; Chazenbalk GD.** Critical relationship between autoantibody recognition and TSH receptor maturation as reflected in the acquisition of mature carbohydrate. *J Clin Endocrinol Metab,* 1996
- **Kakinuma A ; Chazenbalk G ; Filetti S. ; Mci.achlan S ; Rapoport B.** Both the 5' and 3' non-coding regions of the thyrotropin receptor messenger RNA influence the level of receptor protein expression in transfected mammalian cells. *Endocrinology,* 1995
- **Nagayama Y ; Kaufman KD ; Seto P. ; Rapoport B.** Molecular cloning, sequence and functional expression of the cDNA for the human thyrotropin receptor. *BioChem.Biophys.Res.Comm.,* 1989, vol. 165, 1 i84-1190

- **Parma J ; Duprez L ; Van Sande J ; Cochaux P ; Gervy C ; Mockel J ; Dumont J ; Vassart G.** Somatic mutations in the thyrotropin receptor gene cause hyperfunctioning thyroid adenomas. *Nature,* 1993, vol. 365, 649-651
- **Van Sande J. ; Swillens S ; Gerard C ; Allgeier A ; Massart C ; Vassart G ; Dumont J.** In Chinese hamster ovary KI cells dog and human thyrotropin receptors activate both the cyclic AMP and the phosphatidylinositol 4,5-bisphosphate cascades in the presence of thyrotropin and the cyclic AMP cascade in its absence. *Eur J.Biochem,* 1995, vol. 229, 338-343
- **Chen C. ; Okayama H.** High-efficiency transformation of mammalian cells by plasmid DNA. *Mol.Cell.Biol.,* 1987, vol. 7, 2745-2752
- **Nicholson LB ; Vlase H ; Graves P. ; Nilsson M ; Molne J ; Huang GC ; Morgenthaler NG ; Davies TF ; McGregor AM ; Banga JP.** Monoclonal antibodies to the human thyrotropin receptor: Epitope mapping and binding to the native receptor on the basolateral plasma membrane of thyroid follicular cells. *J Mol Endocrinol,* 1996, vol. 16, 159-170
- **Bradford MM.** A rapid and sensitive method for the quantitation of microgram quantities of protein utilizing the principle of protein-dye binding. *Anal.Biochem.,* 1976, vol. 72, 248-254
- **Kasagi K ; Konishi J ; Iida Y ; Ikekubo K ; Mori T ; Kuma K ; Torizuka K.** A new in vitro assay for human thyroid stimulator using cultured thyroid cells: Effect of sodium chloride on adenosine 3',5'-monophosphate increase. *J Clin Endocrinol Metab,* 1982, vol. 54, 108-114
- **Rapoport B ; Filetti S ; Takai N ; Seto P ; Halverson G.** Studies on the cyclic AMP-response to thyroid stimulating immunoglobulin (TSI) and thyrotropin (TSH) in human thyroid cell monolayers. *Metabolism,* 1982, vol. 31, 1159-1167
- **Russo D ; Chazenbalk GD ; Nagayama Y ; Wadsworth HL ; Seto P ; Rapoport B.** A new structural model for the thyrotropin receptor as determined by covalent crosslinking of thyrotropin to the recombinant receptor in intact cells: Evidence for a single polypeptide chain. *Mol.Endocrinol.,* 1991, vol. 5, 1507-1612
- **Chazenbalk GD ; Nagayama Y ; Kaufman KD ; Rapoport B.** The functional expression of recombinant human thyrotropin receptors in non-thyroidal eukaryotic cells provides evidence that homologous desensitization to thyrotropin stimulation requires a cell-specific factor. *Endocrinology,* 1990, vol. 127, 1240-1244
- **Yamamoto M ; Rapoport B.** Studies on the binding of radiolabeled thyrotropin to cultured human thyroid cells. *Endocrinology,* 1978, vol. 103, 2011-2019
- **Costagliola S ; Swillens S ; Niccoli P ; Dumont JE ; Vassart G ; Ludgate M.** Binding assay for thyrotropin receptor autoantibodies using the recombinant receptor protein. *J Clin Endocrinol Metab,* 1992, vol. 75, 1540-1544
- **Matsuba T ; Yamada M ; Suzuki H ; Kanai A ; Isozaki O ; Yoshida T ; Tsushima T ; Yasukawa K.** Expression of recombinant human thyrotropin receptor in myeloma cells. *J.Biochem.,* 1995, vol. 118, 265-270
- **Seetharamaiah GS ; Wagle NM ; Morris JC ; Prabhakar BS.** Generation and characterization of monoclonal antibodies to the human thyrotropin (TSH) receptor: antibodies can bind to discrete conformational or linear epitopes and block TSH binding. *Endocrinology,* 1995, vol. 136, 2817-2824
- **Kjelsberg MA ; Catecchia S ; Ostrowski J ; Caron MG ; Lefkowitz RJ.** Constitutive activation of the alpha IB-adrenergic receptor by all amino acid substitutions at a single site. Evidence for a region which constrains receptor activation. *J Biol Chem,* 1992, vol. 267, 1430-1433
- **Duprez L ; Parma J ; Van Sande J ; Allgeier A ; Leclere J ; Schvartz C ; Delisle M-J ; Decoulx M ; Orgiazzi J ; Dumont J et al.** Gcrmline mutations in the thyrotropin receptor gene cause non-autoimmune autosomal dominant hyperthyroidism. *Nature Genet.,* 1994, vol. 7, 396-401
- **Parma J ; Van Sande J ; Swillens S ; Tonacchera M ; Dumont J ; Vassart G.** Somatic mutations causing constitutive activity of the thyrotropin receptor are the major cause of hyperfunctioning thyrod adenomas: Identification of additional mutations activating both the cyclic adenosine 3',5'-monophosphate and inositol phosphate-Ca2+ cascades. *Mol.Endocrinol.,* 1995, vol. 9, 725-733
- The thyrotropin receptor. In: Biochemical actions of hormones. **Kohn, L.D. ; Aloj, S.M. ; Tombaccini, D. et al.** Anonymous. Academic Press, Inc, 1985, vol. XII, 457-512
- **Couet J ; Sokhavut S ; Jolivet A ; Vu Hai M-T ; Milgrom E ; Misrahi M.** Shedding of human thyrotropin receptor ectodomain: Involvement of a matrix metalloprotease. *J Biol Chem,* 1996, vol. 271, 4545-4552
- **Rapoport B.** Dog thyroid cells in monolayer tissue culture: Adenosine 3',5'-cyclic monophosphate response to thyrotropic hormone. *Endocrinology,* 1976, vol. 98, 1189-1197
- **Wadsworth HL ; Chazenbalk GD ; Nagayama Y ; Russo D ; Rapoport B.** An insertion in the human thyrotropin receptor critical for high affinity hormone binding. *Science,* 1990, vol. 249, 1423-1425
- **Vanderpump, M. P. J. ; Tunbridge, W. M. G. ; French, J. M. ; Appleton, D. ; Bates, D. ; Clark, F. ; Grimley Evans, J. ; Hasan, D. M. ; Rodgers, H. ; Tunbridge, F.** *Clin.Endocrinol.,* 1995, vol. 43, 55-68

- **Rees Smith, B. ; McLachlan, S. M. ; Furmaniak, J.** *Endocr. Rev.,* 1988, vol. 9, 106-121
- **Nagayama, Y. ; Kaufman, K. D. ; Seto, P. ; Rapoport, B.** *Biochem.Biophys.Res.Comm.,* 1989, vol. 165, 1184-1190
- **Libert, F. ; Lefort, A. ; Gerard, C. ; Parmentier, M. ; Perret, J. ; Ludgate, M. ; Dumont, J. E. ; Vassart, G.** *Biochem.Biophys.Res.Comm.,* 1989, vol. 165, 1250-1255
- **Misrahi, M. ; Loosfelt, H. ; Atger, M. ; Sar, S. ; Guiochon-Mantel, A. ; Milgrom, E.** *Biochem.Biophys.Res.Comm.,* 1990, vol. 166, 394-403
- **Takai, O. ; Desai, R. K. ; Seetharamaiah, G. S. ; Jones, C. A. ; Allaway, G. P. ; Akamizu, T. ; Kohn, L. D. ; Prabhaltar, B. S.** *Biochem.Biophys.Res.Comm.,* 1991, vol. 179, 319-326
- **Loosfelt, H. ; Pichon, C. ; Jolivet, A. ; Misrahi, M. ; Caillou, B. ; Jamous, M. ; Vannier, B. ; Milgrom, E.** *Proc. Natl. Acad. Sci. USA,* 1992, vol. 895, 3765-3769
- **Harfst, E. ; Johnstone, A. P. ; Nussey, S. S.** *J. Mol. Endocrinol.,* 1992, vol. 9, 227-236
- **Huang, G. C. ; Collison, K. S. ; McGregor, A. M. ; Banga, J. P.** *J. Mol. Endocrinol.,* 1992, vol. 8, 137-144
- **Costagliola, S. ; Alcalde, L. ; Ruf, J. ; Vassart, G. ; Ludgate, M.** *J. Mol. Endocrinol.,* 1994, vol. 13, 11-21
- **Graves, P. N. ; Vlase, H. ; Davies, T. F.** *Endocrinology,* 1995, vol. 136, 521-527
- **Huang, G. C. ; Page, M. J. ; Nicholson, L. B. ; Collison, K. S. ; McGregor, A. M. ; Banga, J. P.** *J. Mol. Endocrinol.,* 1993, vol. 10, 127-142
- **Seetharamaiah, G. S. ; Desai, R. K. ; Dallas, J. S. ; Tahara, K. ; Kohn, L. D. ; Prabhakar, B. S.** *Autoimmunity,* 1993, vol. 14, 315-320
- **Vlase, H. ; Graves, P. ; Magnusson, R. P. ; Davies, T. F.** *J. Clin. Endocrinol. Metab.,* 1995, vol. 80, 46-53
- **Chazenbalk, G. D. ; Rapoport, B.** *J. Biol. Chem.,* 1995, vol. 270, 1543-1549
- **Seetharamaiah, G. S. ; Dallas, J. S. ; Patibandla, S. A. ; Thotakura, N. R. ; Prabhakar, B. S.** *J.Immunol.,* 1997, vol. 158, 2798-2804
- **Perret, J. ; Ludgate, M. ; Libert, F. ; Gerard, C. ; Dumont, J. E. ; Vassart, G. ; Parmentier, M.** *Biochem.Biophys.Res.Comm.,* 1990, vol. 171, 1044-1050
- **Harfst, E. ; Johnstone, A. P. ; Gout, I. ; Taylor, A. H. ; Waterfield, M. D. ; Nussey, S. S.** *Molec.Cell.Endocrinol.,* 1992, vol. 83, 117-123
- **Endo, T. ; Ohmori, M. ; Ikeda, .M. ; Anzai, E. ; Onaya, T.** *BBRC,* 1992, vol. 186, 1391-1396
- **Matsuba, T. ; Yamada, M. ; Suzuki, H. ; Kanai, A. ; Isozaki, O. ; Yoshida, T. ; Tsushima, T. ; Yasukawa, K.** *J.Biochem.,* 1995, vol. 118, 265-270
- **Murakami, M. ; Miyashita, K. ; Kakizaki, S. ; Saito, S. ; Yamada, M. ; Iriuchijima, T. ; Takeuchi, T. ; Mori, M.** *Eur. J. Endocrinol.,* 1995, vol. 133, 80-86
- **Morgenthaler, N. G. ; Tremble, J. ; Huang, G. ; Scherbaum, W. A. ; McGregor, A. M. ; Banga, J. P.** *J. Clin. Endocrinol. Metab.,* 1996, vol. 81, 700-706
- **Nagy, E. V. ; Burch, H. B. ; Mahoney, K. ; Lukes, Y. G. ; Morris III, J. C. ; Burman, K. D.** *BBRC,* 1992, vol. 188, 28-33
- **Morris, J. C. ; Gibson, J. L. ; Haas, E. J. ; Bergert, E. R. ; Dallas, J. S. ; Prabhakar, B. S.** *Autoimmunity,* 1994, vol. 17, 287-299
- **Shewring, G. A. ; Rees Smith, B.** *Clin.Endocrinol.,* 1982, vol. 17, 409-417
- **Nagayama, Y. ; Wadsworth, H. L. ; Chazenbalk, G. D. ; Russo, D. ; Seto, P. ; Rapoport, B.** *Proc. Natl. Acad. Sci. USA,* 1991, vol. 88, 902-905
- **Nagayama, Y. ; Wadsworth, H. L. ; Russo, D. ; Chazenbalk, G. D. ; Rapoport, B.** *J. Clin. Invest.,* 1991, vol. 88, 336-340
- **Tahara, K. ; Ban, T. ; Minegishi, T. ; Kohn, L. D.** *Biochem.Biophys.Res.Comm.,* 1991, vol. 179, 70-77
- **Wadsworth, H. L. ; Chazenbalk, G. D. ; Nagayama, Y. ; Russo, D. ; Rapoport, B.** *Science,* 1990, vol. 249, 1423-1425
- **Ludgate, M. ; Perret, J. ; Parmentier, M. ; Gerard, C. ; Libert, F. ; Dumont, J. E. ; Vassart, G.** *Molec.Cell.Endocrinol.,* 1990, vol. 73, R13-R18
- **Filetti, S. ; Foti, D. ; Costante, G. ; Rapoport, B.** *J. Clin. Endocrinol. Metab.,* 1991, vol. 72, 1096-1101
- **Chazenbalk, G. D. ; Kakinuma, A. ; Jaume, J. C. ; McLachlan. S. M. ; Rapoport, B.** *Endocrinology,* 1996, vol. 137, 4586-4591
- **Harfst, E. ; Johnstone, A. P. ; Nussey, S. S.** *Lancet,* 1992, vol. 339, 193-194
- **Rapoport, B. ; McLachlan, S. M. ; Kakinuma, A. ; Chazenbalk, G. D.** *J. Clin. Endocrinol. Metab.,* 1996, vol. 81, 2525-2533
- **Kakinuma, A. ; Chazenbalk, G. ; Filetti, S. ; McLachlan, S. M. ; Rapoport, B.** *Endocrinology,* 1996, vol. 137, 2664-2669
- **Kaufman, K. D. ; Foti, D. ; Seto, P. ; Rapoport, B.** *Molec.Cell.Endocrinol.,* 1991, vol. 78, 107-114
- **Nicholson, L. B. ; Vlase, H. ; Graves, P. ; Nilsson, M. ; Molne, J. ; Huang, G. C. ; Morgenthaler, N. G. ; Davies, T. F. ; McGregor, A. M. ; Banga, J. P.** *J. Mol. Endocrinol.,* 1996, vol. 16, 159-170
- **Laemmli, U. K.** *Nature,* 1970, vol. 227, 680-685
- **Domer, A. J. ; Wasley, L. C. ; Kaufman, R. J.** *J. Biol. Chem.,* 1989, vol. 264, 20602-20607

- **Jaume, J. C. ; Kakinuma, A. ; Chazenbalk, G. D. ; Rapoport, B. ; McLachlan, S. M.** *J. Clin. Endocrinol. Metab.,* 1997, vol. 82, 500-507
- **Kakinuma, A. ; Chazenbalk, G. D. ; Jaume, J. C. ; Rapoport, B. ; McLachlan, S. M.** *J. Clin. Endocrinol. Metab.,* 1997
- **Kornfeld, R. ; Kornfeld, S.** *Annu. Rev. Biochem.,* 1985, vol. 54, 631-646
- **Robbins, P. W. ; Hubbard, S. C. ; Turco, S. J. ; Wirth, D. F.** *Cell,* 1977, vol. 12, 893-900
- **Koo, Y. B. ; Ji, I. ; Ji, T. H.** *Endocrinology,* 1994, vol. 134, 19-26
- **Tsai-Morris, C. H. ; Buczko, E. ; Wang, W. ; Dufau, M. L.** *J. Biol. Chem.,* 1990, vol. 265, 19385-19388
- **VuHai-LuuThi, M. T. ; Misrahi, M. ; Houllier, A. ; Jolivet, A. ; Milgrom. E.** *Biochem.,* 1992, vol. 31, 8377-8383
- **Takeshita, A. ; Nagayama, Y. ; Fujiyama, K. ; Yokoyama, N. ; Namba, H. ; Yamashita, S. ; Izumi, M. ; Nagataki, S.** *Biochem.Biophys.Res.Comm.,* 1992, vol. 188, 1214-1219
- **Graves, P. N. ; Tomer, Y. ; Davies. T. F.** *Biochem.Biophys.Res.Comm.,* 1992, vol. 187, 1135-1143
- **Kress, B. C. ; Spiro, R. G.** *Endocrinology,* 1986, vol. 118, 974-979
- **Johnstone, A. P. ; Cridland, J. C. ; DaCosta, C. R. ; Harfst, E. ; Shepherd, P. S.** *Molec.Cell.Endocrinol.,* 1994, vol. 105, R1-R9
- **Seetharamaiah, G. S. ; Wagle, N. M. ; Morris, J. C. ; Prabhakar, B. S.** *Endocrinology,* 1995, vol. 136, 2817-2824
- **Shimojo, N. ; Kohno, Y. ; Yamaguchi, K. ; Kikuoka, S. ; Hoshioka, A. ; Niimi, H. ; Hirai, A. ; Tamura, Y. ; Saito, Y. ; Kohn, L. D.** *Proc. Natl. Acad Sci. USA,* 1996, vol. 93, 11074-11079
- **Smith, B. R.** *J. Endocrinol.,* 1970, vol. 46, 45-54
- **Dirmikis, S. ; Munro, D. S.** *J. Endocrinol.,* 1973, vol. 58, 577-590
- **Foti, D. ; Russo, D. ; Costante, G. ; Filetti, S.** *J. Clin. Endocrinol. Metab.,* 1991, vol. 73, 710-716
- **McLachlan, S. M. ; Rapoport, B.** *J.Int.Med.,* 1993, vol. 234, 347-359
- **Nagayama, Y. ; Russo, D. ; Chazenbalk, G. D. ; Wadsworth, H. L. ; Rapoport, B.** *Biochem.Biophys.Res.Comm.,* 1990, vol. 173, 1150-1156
- **Shi, Y. ; Zou, M. ; Parhar, R. S. ; Farid, N. R.** *Thyroid,* 1993, vol. 3, 129-133
- **Seetharamaiah, G. S. ; Kurosky, A. ; Desai, R. K. ; Dallas, J. S. ; Prabhakar, B. S.** *Endocrinology,* 1994, vol. 134, 549-554

- **Segaloff, D. L. ; Ascoli, M.** *Endocr. Rev.,* 1993, vol. 14, 324-347
- **Heyma, P ; Harrison, LC.** Precipitation of the thyrotropin receptor and identification of thyroid autoantigens using Graves' disease immunoglobulins. *J Clin Invest.,* 1984, vol. 74, 1090-1097
- **Rapoport. B ; Seto, P ; Magnusson, RP.** Immunoprecipitation of radiolabeled human thyroid cell proteins by serum from patients with autoimmune thyroid disease. *Endocrinology,* 1984, vol. 115, 2137-2144
- **De Forteza, R. ; Smith, CU ; Amin, J. ; McKenzie, JM ; Zakarija, M.** Visualization of the thyrotropin receptor on the cell surface by potent autoantibodies. *J Clin Endocrinol & Metab.,* 1994, vol. 78, 1271-1273
- **Huang, GC ; Page, MJ. ; Roberts, AJ ; Malik, AN ; Spence, H ; McGregor, AM ; Banga, JP.** Molecular cloning of a human thyrotropin receptor cDNA fragment. *FEBS Letters,* 1990, vol. 264, 193-197
- **Vlase, H ; Graves, P ; Magnusson, RP ; Davies, TF.** Human autoantibodies to the thyrotropin receptor: recognition of linear, folded, and glycosylated recombinant extracellular domain. *J Clin Endocrinol Metab.,* 1995, vol. 80, 46-53
- **Tonacchera, M ; Costagliola. S ; Cetani, F ; Ducobu, J ; Stordeur. P ; Vassart, G ; Ludgate, M.** Patient with monoclonal gammopathy, thyrotoxicosis, pretibial myxedema and thyroid-associated ophthalmopathy; demonstration of direct binding of autoantibodies to the thyrotropin receptor. *Eur J Endocrinol.,* 1996, vol. 134, 97-103
- **Morgenthaler, NG ; Tremble, J ; Huang, G ; Scherbaum, WA ; McGregor, AM ; Banga, JP.** Binding of antithyrotropin receptor autoantibodies in Graves' disease serum to nascent, in vitro translated thyrotropin receptor: Ability to map epitopes recognized by antibodies. *J Clin Endocrinol Metab.,* 1996, vol. 81, 700-706
- **Loosfelt, H ; Pichon, C ; Jolivet, A ; Misrahi, M ; Caillou, B ; Jamous, M ; Vannier, B ; Milgrom. E.** Two-subunit structure of the human thyrotropin receptor. *Proc Natl Acad Sci USA.,* 1992, vol. 895, 3765-3769
- **Johnstone, AP ; Cridland, JC ; DaCosta. CR ; Harfst, E ; Shepherd, PS.** Monoclonal antibodies that recognize the native human thyrotropin receptor. *Molec.Cell.Endocrinol.,* 1994, vol. 105, R1-R9
- **Seetharamaiah. GS. ; Wagle, NM ; Morris, JC ; Prabhakar, BS.** Generation and characterization of monoclonal antibodies to the human thyrotropin (TSH) receptor: antibodies can bind to discrete conformational or linear epitopes and block TSH binding. *Endocrinology,* 1995, vol. 136, 2817-2824

- **Nicholson, LB ; Vlase, H ; Graves, P ; Nilsson, M ; Molne, J ; Huang, GC ; Morgenthaler, NG ; Davies, TF ; McGregor, AM ; Banga, JP.** Monoclonal antibodies to the human thyrotropin receptor: Epitope mapping and binding to the native receptor on the basolateral plasma membrane of thyroid follicular cells. *J Mol Endocrinol.,* 1996, vol. 16, 159-170
- **Beever, K ; Bradbury, J ; Phillips, D ; McLachlan, SM ; Pegg, C ; Goral, A. ; Overbeck, W ; Feifel, G ; Rees Smith, B.** Highly sensitive assays of autoantibodies to thyroglobulin and to thyroid peroxidase. *Clin Chem.,* 1989, vol. 35, 949-1954
- **Smith, BR.** Characterization of long-acting thyroid stimulator gG binding protein. *Biochim. Biophys. Acta,* 1971, vol. 229, 649-662
- **Rees Smith, B. ; McLachlan, SM. ; Furmaniak. J.** Autoantibodies to the thyrotropin receptor. *Endocr Rev.,* 1988, vol. 9, 106-121
- **McLachlan, SM ; Rapoport. B.** Recombinant thyroid autoantigens: the keys to the pathogenesis of autoimmune thyroid disease. *J.Int.Med.,* 1993, vol. 234, 347-359
- **Chazenbalk. GD ; Kakinuma, A ; Jaume, JC. ; McLachlan, SM. ; Rapoport, B.** Evidence for negative cooperativity among human thyrotropin receptors overexpressed in mammalian cells. *Endocrinology,* 1996
- **Portolano, S ; Chazenbalk, GD ; Seto. P. ; Hutchison, JS ; Rapoport, B ; McLachlan, SM.** Recognition by recombinant autoimmune thyroid disease-derived Fab fragments of a dominant conformational epitope on human thyroid peroxidase. *J Clin Invest.,* 1992, vol. 90, 720-726
- **Kaufman, KD ; Foti. D ; Seto. P ; Rapoport, B.** Overexpression of an immunologically-intact, secreted form of human thyroid peroxidase in eukaryotic cells. *Molec.Cell.Endocrinol.,* 1991, vol. 78, 107-114
- **Kaufman, KD ; Rapoport, B ; Seto, P. ; Chazenbalk, GD ; Magnusson, RP.** Generation of recombinant, enzymatically-active, human thyroid peroxidase and its recognition by antibodies in the sera of patients with Hashimoto's thyroiditis. *J Clin Invest.,* 1989, vol. 84, 394-403
- **Nishikawa, T ; Rapoport, B ; McLachlan, SM.** Exclusion of two major areas on thyroid peroxidase from the immunodominant region containing the conformational epitopes recognized by human autoantibodies. *J Clin Endocrinol Metab.,* 1994, vol. 79, 1648-1654
- **Vlase, H ; Nakashima, M ; Graves, PN ; Tomer, Y. ; Morris, JC ; Davies, TF.** Defining the major antibody epitopes on the human thyrotropin receptor in immunized mice: evidence for intramolecular epitope spreading. *Endocrinology,* 1995, vol. 136, 4415-4423
- **Nagayama, Y ; Kaufman, KD ; Seto, P ; Rapoport, B.** Molecular cloning, sequence and functional expression of the cDNA for the human thyrotropin receptor. *Biochem.Biophys.Res.Comm.,* 1989, vol. 165, 1184-1190
- **Kakinuma, A ; Chazenbalk, G ; Filetti, S ; McLachlan, S. ; Rapoport, B.** Both the 5' and 3' non-coding regions of the thyrotropin receptor messenger RNA influence the level of receptor protein expression in transfected mammalian cells. *Endocrinology,* 1996, vol. 137, 2664-2669
- **McLachlan, SM ; Rapoport. B.** Genetic and epitopic analysis of thyroid peroxidase (TPO) autoantibodies: markers of the human thyroid autoimmune response. *Clin.Exp.Immunol.,* 1995, vol. 101, 200-206
- **McLachlan, SM ; Rees Smith, B ; Petersen, VB ; Davies, TF ; Hall, R.** Thyroid stimulating autoantibody production in vitro. *Nature,* 1977, vol. 270, 447-449
- **McLachlan, SM ; Pegg, CAS ; Atherton, MC ; Middleton, SM ; Clark, F ; Rees Smith. B.** TSH receptor antibody synthesis by thyroid lymphocytes. *Clin.Endocrinol.,* 1986, vol. 24, 223-230
- **McLachlan, SM ; Rapoport, B.** Editorial: Monoclonal human autoantibodies to the TSH receptor: the holy grail, and why are we looking for it?. *J Clin Endocrinol Metab.,* 1996, vol. 81, 3152-3154
- **Zakarija, MJ.** Immunochemical characterization of the thyroid-stimulating antibody (TSab) of Graves' disease: evidence for restricted heterogeneity. *J.Clin.Lab.Immunol.,* 1983, vol. 10, 77-85
- **Knight, J ; Laing, P ; Knight, A ; Adams, D ; Ling, N.** Thyroid stimulating autoantibodies usually contain only lambda-light chains: evidence for the "forbidden clone" theory. *J Clin Endocrinol Metab.,* 1986, vol. 62, 342-347
- **Williams, RCJ ; Marshall, NJ ; Kilpatrick, K ; Montano, J ; Brickell, PM ; Goodall, M ; Ealey, PA ; Shine, B ; Weetman. AP ; Craig, RK.** Kappa/lambda immunoglobulin distribution in Graves' thyroid-stimulating antibodies. *J Clin Invest.,* 1988, vol. 82, 1306-1312
- **Weetman, AP ; Yateman, ME ; Ealey, PA ; Black, CM ; Reimer, CB ; Williams, RCJ ; Shine, B ; Marshall, NJ.** Thyroid-stimulating antibody activity between different immunoglobulin G subclasses. *J Clin Invest.,* 1990, vol. 86, 723-727
- **B. Gross ; M. Misrahi ; S. Sar ; E. Milgrom.** *Biochem.Biophys.Res.Comm.,* 1991, vol. 177, 679
- **C. H. Tsai-Morris ; E. Buczko ; W. Wang ; X.-Z. Xie ; M. L. Dufau.** *J. Biol. Chem.,* 1991, vol. 266, 11355
- **Y. B. Koo ; I, Ji ; R. G. Slaughter ; T. H. Ji.** *Endocrinology,* 1991, vol. 128, 2297
- **J. Furmaniak et al.** *FEBS Letters,* 1987, vol. 215, 316

- **D. Russo et al.** *Mol.Endocrinol.,* 1991, vol. 5, 1607
- **D. L. Segaloff ; M. Ascoli.** *Endocr. Rev.,* 1993, vol. 14, 324
- **H. Loosfelt et al.** *Proc. Natl. Acad. Sci. USA,* 1992, vol. 895, 3765
- **J. Hata et al.** *Biochem.Biophys.Res.Comm.,* 1989, vol. 164, 1268
- **E. Potter et al.** *BBRC,* 1994, vol. 205, 361
- **G. D. Chazenbalk ; A. Kakinuma ; J. C. Jaume ; S. M. McLachlan ; B. Rapoport.** *Endocrinology,* 1996, vol. 137, 4586
- **P. N. Graves ; H. Vlase ; Y. Bobovnikova ; T. F. Davies.** *Endocrinology,* 1996, vol. 137, 3915
- **P. R. Buckland ; C. R. Rickards ; R. D. Howells ; E. D. Jones ; B. Rees Smith.** *FEBS Letters,* 1982, vol. 145, 245
- **M. Parmentier et al.** *Science,* 1989, vol. 246, 1620
- **Y. Nagayama ; K. D. Kaufman ; P. Seto ; B. Rapoport.** *Biochem.Biophys.Res.Comm.,* 1989, vol. 165, 1184
- **F. Libert et al.** *Biochem.Biophys.Res.Comm.,* 1989, vol. 165, 1250
- **M. Misrahi et al.** *Biochem.Biophys.Res.Comm.,* 1990, vol. 166, 394
- **J. Couet et al.** *J. Biol. Chem.,* 1996, vol. 271, 4545
- **G. D. Chazenbalk et al.** *Endocrinology,* 1997
- **Y. Nagayama et al.** *Proc. Natl. Acad. Sci. USA,* 1991, vol. 88, 902
- **D. Russo ; Y. Nagayama ; G. D. Chazenbalk ; H. L. Wadsworth ; B. Rapoport.** *Endocrinology,* 1992, vol. 130, 2135
- **H. L. Wadsworth ; G. D. Chazenbalk ; Y. Nagayama ; D. Russo ; B. Rapoport.** *Science,* 1990, vol. 249, 1423
- **M. Misrahi et al.** *Eur.J.Biochem.,* 1994, vol. 222, 711
- **G. D. Chazenbalk ; B. Rapoport.** *J. Biol. Chem.,* 1994, vol. 269, 32209
- **D. P. Davis ; T. G. Rozell ; X. Liu ; D. L. Segaloff.** *Mol.Endocrinol.,* 1997, vol. 11, 550
- **T.-K. H. Vu ; D. T. Hung ; V. I. Wheaton ; S. R. Coughlin.** *Cell,* 1991, vol. 64, 1057
- **G. D. Chazenbalk ; S. M. McLachlan ; Y. Nagayama ; B. Rapoport.** *Biochem.Biophys.Res.Comm,* 1996, vol. 225, 479
- **A. Kakinuma ; G. Chazenbalk ; S. Filetti ; S. M. McLachlan ; B. Rapoport.** *Endocrinology,* 1996, vol. 137, 2664
- **F. Sanger ; S. Nicklen ; A. R. Coulson.** *Proc. Natl. Acad Sci. USA,* 1977, vol. 74, 5463
- **Y. Nagayama ; B. Rapoport.** *Endocrinology,* 1992, vol. 131, 548